# FASCICULE DE BREVET EUROPEEN

(11) **EP 2 829 545 B1**
(45) Date de publication et mention de la délivrance du brevet: **22.06.2016**
(21) Numéro de dépôt: 14178030.4
(22) Date de dépôt: 22.07.2014
(51) Int. Cl.: A61N 5/10, A61K 45/06, C07F 9/6558, C07F 9/09, A61K 31/675, C07F 9/6561

(54) **Nouveaux derives phosphates, leur procede de preparation et les compositions pharmaceutiques qui les contiennent**
Neue Phosphatderivate, ihr Herstellungsverfahren und pharmazeutische Zusammensetzungen, die sie enthalten
New phosphate derivatives, method of preparing same and pharmaceutical compositions containing them

(30) Priorité: 23.07.2013 FR 1357259
(43) Date de publication de la demande: 28.01.2015
(73) Titulaire: Les Laboratoires Servier, 92284 Suresnes Cedex (FR); Vernalis (R&D) Limited, Winnersh Berkshire RG41 5RD (GB)
(72) Inventeur: Le Tiran, Arnaud, 78290 Croissy sur Seine (FR); Le Diguarher, Thierry, 45550 Saint Denis de l'Hôtel (FR); Starck, Jérôme-Benoît, 92500 Rueil-Malmaison (FR); Henlin, Jean-Michel, 92150 Suresnes (FR); Guillouzic, Anne-Françoise, 92000 Nanterre (FR); De Nanteuil, Guillaume, 92150 Suresnes (FR); Geneste, Olivier, 92500 Rueil-Malmaison (FR); Davidson, James Edward Paul, Cambridge Cambridgeshire CB22 5DJ (GB); Murray, James Brooke, Linton Cambridgeshire CB21 4YL (GB); Chen, I-Jen, Cambridge, Cambridgeshire CB1 3NY (GB)

(56) Documents cités:
- PORTER J ET AL: "Atropisomeric small molecule Bcl-2 ligands: Determination of bioactive conformation", BIOORGANIC & MEDICINAL CHEMISTRY LETTERS, PERGAMON, AMSTERDAM, NL, vol. 19, no. 6, 15 mars 2009 (2009-03-15), pages 1767-1772, XP026005876, ISSN: 0960-894X, DOI: 10.1016/J.BMCL.2009.01.071 [extrait le 2009-01-27]
- PORTER J ET AL: "Tetrahydroisoquinoline amide substituted phenyl pyrazoles as selective Bcl-2 inhibitors", BIOORGANIC & MEDICINAL CHEMISTRY LETTERS, PERGAMON, AMSTERDAM, NL, vol. 19, no. 1, 31 octobre 2008 (2008-10-31), pages 230-233, XP025816913, ISSN: 0960-894X, DOI: 10.1016/J.BMCL.2008.10.113 [extrait le 2008-10-31]
- HEIDI L. PEREZ ET AL: "Identification of a phenylacylsulfonamide series of dual Bcl-2/Bcl-xL antagonists", BIOORGANIC & MEDICINAL CHEMISTRY LETTERS, vol. 22, no. 12, 2 mai 2012 (2012-05-02), pages 3946-3950, XP055046515, ISSN: 0960-894X, DOI: 10.1016/j.bmcl.2012.04.103
- BUNDGAARD H: "DESIGN AND APPLICATION OF PRODRUGS", TEXTBOOK OF DRUG DESIGN AND DEVELOPMENT, XX, XX, 1991, pages 113-191, XP001188113,
- KOMAROVA, N. L., BOLAND, C. R.: NATURE, vol. 499, 18 July 2013 (2013-07-18),

## Description

La présente invention concerne de nouveaux dérivés phosphates, leur procédé de préparation et les compositions pharmaceutiques qui les contiennent.

Les composés de la présente invention sont nouveaux et présentent des caractéristiques pharmacologiques et pharmacocinétiques très intéressantes pour leur utilisation dans le domaine de l'apoptose et de la cancérologie.

L'apoptose, ou mort cellulaire programmée, est un processus physiologique crucial pour le développement embryonnaire et le maintien de l'homéostasie tissulaire.

La mort cellulaire de type apoptotique fait intervenir des changements morphologiques, tels que la condensation du noyau, la fragmentation de l'ADN, ainsi que des phénomènes biochimiques, tels que l'activation des caspases qui vont dégrader des composants structuraux clés de la cellule pour induire son désassemblage et sa mort. La régulation du processus d'apoptose est complexe et implique l'activation ou la répression de plusieurs voies de signalisation intracellulaire (Cory S. et al., Nature Review Cancer, 2002, 2, 647-656).

Des dérégulations de l'apoptose sont impliquées dans certaines pathologies. Une apoptose accrue est liée aux maladies neurodégénératives telles que la maladie de Parkinson, la maladie d'Alzheimer et l'ischémie. Inversement, des déficiences dans l'exécution de l'apoptose jouent un rôle important dans le développement des cancers et leur chimiorésistance, des maladies auto-immunes, des maladies inflammatoires et des infections virales. Ainsi, l'échappement à l'apoptose fait partie des signatures phénotypiques du cancer (Hanahan D. et al., Cell 2000, 100, 57-70).

Les protéines anti-apoptotiques de la famille Bcl-2 sont associées à de nombreuses pathologies. L'implication des protéines de la famille Bcl-2 est décrite dans de nombreux types de cancer, tel que le cancer colorectal, le cancer du sein, le cancer du poumon à petites cellules, le cancer du poumon non à petites cellules, le cancer de la vessie, le cancer de l'ovaire, le cancer de la prostate, la leucémie lymphoïde chronique, le lymphome folliculaire, le myélome... La surexpression des protéines anti-apoptotiques de la famille Bcl-2 est impliquée dans la tumorogenèse, dans la résistance à la chimiothérapie et dans le pronostique clinique des patients atteints de cancer. Il existe donc un besoin thérapeutique de composés inhibant l'activité anti-apoptotique des protéines de la famille Bcl-2. Parmi les inhibiteurs de Bcl-2 déjà connus dans la littérature, on distingue les composés 1-[2-[(3,4-dihydro-2(1H)-isoquinolin-2-yl)carbonyl]phenyl]-pyrazole-3-carboxamide décrits dans Bioorganic & Medicinal Chemistry Letters, Vol. 19 (6), 2009, 1767-1772 et Bioorganic & Medicinal Chemistry Letters, Vol. 19 (1), 2008, 230-233. D'autres dérivés carboxamides de pyrazole sont divulgués dans Biorganic & Medicinal Chemistry Letters, Vol. 22 (12), 2012, 3946-3950 Tous présentent un intérêt potentiel dans le traitement du cancer. Par ailleurs, la préparation de prodrogues par estérification d'un groupe hydroxyle avec l'acide phosphorique est décrite dans Bundgaard H, "Design and application of prodrugs", 1991**.**

Les composés de la présente invention outre leur nouveauté, présentent des propriétés pharmacologiques et pharmacocinétiques permettant de les utiliser dans les pathologies impliquant un défaut d'apoptose, comme par exemple dans le traitement du cancer, des maladies auto-immunes et du système immunitaire.

La présente invention concerne plus particulièrement un composé phosphate de formule (I) : 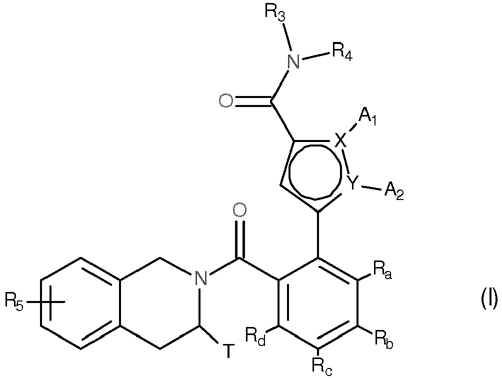 dans laquelle :
◆ X et Y représentent un atome de carbone ou un atome d'azote, étant entendu qu'ils ne peuvent représenter simultanément deux atomes de carbone ou deux atomes d'azote,
◆ A₁ et A₂ forment ensemble avec les atomes qui les portent un hétérocycle Het éventuellement substitué, aromatique ou non, constitué de 5, 6 ou 7 chaînons, et pouvant contenir, en plus de l'azote représenté par X ou par Y, un à 3 hétéroatomes choisis indépendamment parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié, ou bien, A₁ et A₂ représentent indépendamment l'un de l'autre un atome d'hydrogène, un polyhalogénoalkyle (C₁-C₆) linéaire ou ramifié, un groupement alkyle (C₁-C₆) linéaire ou ramifié ou un cycloalkyle,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
   ou bien R₁ et R₂ forment avec l'atome d'azote qui les porte un hétérocycloalkyle,
◆ R₃ représente un groupement alkyle (C₁-C₆) linéaire ou ramifié, alkényle (C₂-C₆) linéaire ou ramifié, alkynyle (C₂-C₆) linéaire ou ramifié, cycloalkyle, cycloalkyl(C₃-C₁₀)alkyle (C₁-C₆) linéaire ou ramifié, hétérocycloalkyle, aryle ou hétéroaryle, étant entendu qu'un ou plusieurs atomes de carbone des groupements précédents, ou de leurs éventuels substituants, peut(vent) être deutéré(s),
◆ R₄ représente un phényle substitué en position *para* par un groupement de formule -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃), ou -OPO(O⁻M₁⁺)(O[CH₂CH₂O]ₙCH₃), ou bien R₄ représente un groupement pyrimidin-5-yle, substitué en position *para* par un groupement de formule -OPO(O⁻M₁⁺)(O-M₂⁺), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5, étant entendu que le groupement phényle peut être optionnellement substitué par un ou plusieurs atomes d'halogène,
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ, R_{b}, R_{c} et R_{d} représentent indépendamment les uns des autres R₇, un atome d'halogène, un groupement alkoxy (C₁-C₆) linéaire ou ramifié, un groupe hydroxy, un groupement polyhalogénoalkyle (C₁-C₆) linéaire ou ramifié, un groupe trifluorométhoxy, -NR₇R₇', nitro, R₇-CO-alkyl(C₀-C₆)-, R₇-CO-NH-alkyl(C₀-C₆)-, NR₇R₇'-CO-alkyl(C₀-C₆)-, NR₇R₇'-CO-alkyl(C₀-C₆)-O-, R₇-SO₂-NH-alkyl(C₀-C₆)-, R₇-NH-CO-NH-alkyl(C₀-C₆)-, R₇-O-CO-NH-alkyl(C₀-C₆)-, un groupement hétérocycloalkyle, ou bien les substituants de l'un des couples (Rₐ,R_{b}), (R_{b},R_{c}) ou (R_{c},R_{d}) forment ensemble avec les atomes de carbone qui les portent un cycle constitué de 5 à 7 chaînons, pouvant contenir de un à 2 hétéroatomes choisis parmi oxygène et soufre, étant aussi entendu qu'un ou plusieurs atomes de carbone du cycle précédemment défini peut(vent) être deutéré(s) ou substitués par un à 3 groupements choisis parmi halogène ou alkyle (C₁-C₆) linéaire ou ramifié,
◆ R₇ et R₇' représentent indépendamment l'un de l'autre un hydrogène, un alkyle (C₁-C₆) linéaire ou ramifié, un alkényle (C₂-C₆) linéaire ou ramifié, un alkynyle (C₂-C₆) linéaire ou ramifié, un aryle ou un hétéroaryle, ou bien R₇ et R₇' forment ensemble avec l'atome d'azote qui les porte un hétérocycle constitué de 5 à 7 chaînons,
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié éventuellement substitué, spiro (C₃-C₆), alkoxy (C₁-C₆) linéaire ou ramifié éventuellement substitué, (C₁-C₆)alkyl-S-, hydroxy, oxo (ou *N-*oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle éventuellement substitué, hétéroaryle, aryloxy, arylthio, cycloalkyle, hétérocycloalkyle éventuellement substitué par un ou plusieurs atomes d'halogène ou groupements alkyles, étant entendu que R' et R" représentent, indépendamment l'un de l'autre, un atome d'hydrogène ou un groupement alkyle (C₁-C₆) linéaire ou ramifié éventuellement substitué,
le groupement Het défini dans la formule (1) pouvant être substitué par un à trois groupements choisis parmi alkyle (C₁-C₆) linéaire ou ramifié, hydroxy, alkoxy (C₁-C₆) linéaire ou ramifié, NR₁'R₁", ou halogène, étant entendu que R₁' et R₁" ont les mêmes définitions que les groupes R' et R" mentionnés précédemment,
ses énantiomères et diastéréoisomères, ainsi que ses sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc... Parmi les composés préférés de l'invention, on trouve les composés de formule (I) pour lesquels R₄ représente un phényle substitué en position *para* par un groupement de formule -OPO(OM)(OM'), -OPO(OM)(O⁻M₁⁺), -OPO(O-M₁⁺)(O⁻M₂⁺), -OPO(O⁻)(O⁻)M₃²⁺, -OPO(OM)(O[CH₂CH₂O]ₙCH₃), ou -OPO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5, étant entendu que le groupement phényle peut être optionnellement substitué par un ou plusieurs atomes d'halogène.

Les composés de formule (I) dans lesquels R₄ représente un phényle ou un groupement pyrimidin-5-yle, tous deux substitués en position *para* par un groupement de formule -OPO(O⁻M₁⁺)(O⁻M₂⁺), et plus particulièrement encore par un groupement de formule -OPO(O⁻Na⁺)(O⁻Na⁺), sont préférés.

De manière avantageuse, X représente un atome de carbone et Y représente un atome d'azote. Plus avantageusement encore, le groupe : 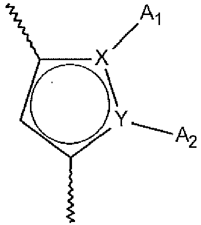 représente une 5,6,7,8-tétrahydroindolizine, une indolizine ou un pyrrole diméthylé.

De manière préférentielle, T représente un groupement méthyle, (morpholin-4-yl)méthyle ou 3-(morpholin-4-yl)propyle.

Dans les composés préférés de l'invention, Rₐ et R_{d} représentent chacun un atome d'hydrogène et (R_{b},R_{c}) forment ensemble avec les atomes de carbone qui les portent un groupe 1,3-dioxolane, un groupe 1,4-dioxane, ou bien Rₐ, R_{c} et R_{d} représentent chacun un atome d'hydrogène et R_{b} représente un hydrogène ou un halogène.

Dans un autre mode de réalisation de l'invention, Rₐ et R_{d} représentent chacun un atome d'hydrogène, R_{b} représente un atome d'halogène et R_{c} un groupement méthoxy.

Alternativement, Rₐ, R_{b} et R_{d} représentent avantageusement chacun un atome d'hydrogène et R_{c} représente un groupement NR₇R₇'-CO-alkyl(C₀-C₆)-O-, et plus préférentiellement encore R_{c} représente un groupement 2-oxo-2-(pipéridin-1-yl)éthoxy.

Par ailleurs, R₃ représente avantageusement un groupement choisi parmi phényle, 1*H-*indole, 1*H*-pyrrolo[2,3-b]pyridine, pyridine, 1*H*-pyrazole, 1*H*-pyrrole, et 2,3-dihydro-1*H* pyrrolo[2,3-*b*]pyridine, ces groupements comportant éventuellement un ou plusieurs substituants choisis parmi alkyle (C₁-C₆) linéaire ou ramifié (et plus préférentiellement méthyle), cyano, ou trideutériométhyle.

Parmi les composés préférés de l'invention, on peut citer :
- 4-[{[3-(6-{[(3*S*)-3-(morpholin-4-ylméthyl)-3,4-dihydroisoquinolin-2(1*H)-*yl]carbonyl}-1,3-benzodioxol-5-yl)-5,6,7,8-tétrahydroindolizin-1-yl]carbonyl}(phényl)amino]phényl phosphate de disodium,
- 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(pyridin-4-yl)amino]phényl phosphate de disodium,
- 4-({[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}[1-(trideutériométhyl)-1*H*-pyrazol-4-yl]amino)phényl phosphate de disodium,
- 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(5-cyano-1,2-diméthyl-1*H*-pyrrol-3-yl)amino]phényl phosphate de disodium,
- 4-[{[5-(5-chloro-2-{[(3*S*)*-*3-(morpholin-4-ylméthyl)-3,4-dihydroisoquinolin-2(1*H*)-yl]carbonyl}phényl)-1,2-diméthyl-1*H*-pyrrol-3-yl]carbonyl}(5-cyano-1-méthyl-1*H-*pyrrol-3-yl)amino]phényl phosphate de disodium,
- 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H-*pyrrol-3-yl]carbonyl}(1-méthyl-1*H*-pyrazol-4-yl)amino]phényl phosphate de disodium,
- 4-[(5-cyano-1,2-diméthyl-1*H*-pyrrol-3-yl){[5-(5-fluoro-2-{[(3*S*)-3-(morpholin-4-ylméthyl)-3,4-dihydroisoquinolin-2(1*H*)-yl]carbonyl}phényl)-1,2-diméthyl-1H-pyrrol-3-yl]carbonyl}amino]phényl phosphate de disodium,
- 4-[{[5-(5-fluoro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(1-méthyl-1H-pyrazol-4-yl)amino]phényl phosphate de disodium,
leurs énantiomères et diastéréoisomères, ainsi que leurs sels d'addition à un acide ou à une base pharmaceutiquement acceptable.

L'étude pharmacocinétique des composés phosphates de formule (I) a montré qu'ils se convertissaient *in vivo* en composés de formule (I') caractérisés en ce que la fonction phosphate a été métabolisée en une fonction hydroxy. Les composés de formule (I) se comportent donc comme des prodrogues des composés de formule (I') de formule suivante : 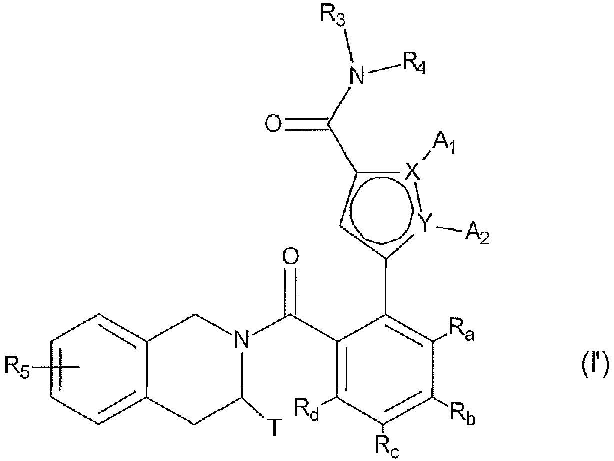 dans laquelle :
◆ X et Y représentent un atome de carbone ou un atome d'azote, étant entendu qu'ils ne peuvent représenter simultanément deux atomes de carbone ou deux atomes d'azote,
◆ A₁ et A₂ forment ensemble avec les atomes qui les portent un hétérocycle Het éventuellement substitué, aromatique ou non, constitué de 5, 6 ou 7 chaînons, et pouvant contenir, en plus de l'azote représenté par X ou par Y, un à 3 hétéroatomes choisis indépendamment parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié, ou bien, A₁ et A₂ représentent indépendamment l'un de l'autre un atome d'hydrogène, un polyhalogénoalkyle (C₁-C₆) linéaire ou ramifié, un groupement allyle (C₁-C₆) linéaire ou ramifié ou un cycloalkyle,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
   ou bien R₁ et R₂ forment avec l'atome d'azote qui les porte un hétérocycloalkyle,
◆ R₃ représente un groupement alkyle (C₁-C₆) linéaire ou ramifié, alkényle (C₂-C₆) linéaire ou ramifié, alkynyle (C₂-C₆) linéaire ou ramifié, cycloalkyle, cycloalkyl(C₃-C₁₀)alkyle (C₁-C₆) linéaire ou ramifié, hétérocycloalkyle, aryle ou hétéroaryle, étant entendu qu'un ou plusieurs atomes de carbone des groupements précédents, ou de leurs éventuels substituants, peut(vent) être deutéré(s),
◆ R₄ représente un groupement aryle, hétéroaryle, cycloalkyl ou alkyle (C₁-C₆) linéaire ou ramifié, étant entendu qu'un ou plusieurs atomes de carbone des groupements précédents, ou de leurs éventuels substituants, peut(vent) être deutéré(s),
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ, R_{b}, R_{c} et R_{d} représentent indépendamment les uns des autres R₇, un atome d'halogène, un groupement alkoxy (C₁-C₆) linéaire ou ramifié, un groupe hydroxy, un groupement polyhalogénoalkyle (C₁-C₆) linéaire ou ramifié, un groupe trifluorométhoxy, -NR₇R₇', nitro, R₇-CO-alkyl(C₀-C₆)-, R₇-CO-NH-alkyl(C₀-C₆)-, NR₇R₇'-CO-alkyl(C₀-C₆)-, NR₇R₇'-CO-alkyl(C₀-C₆)-O-, R₇-SO₂-NH-alkyl(C₀-C₆)-, R₇-NH-CO-NH-alkyl(C₀-C₆)-, R₇-O-CO-NH-alkyl(C₀-C₆)-, un groupement hétérocycloalkyle, ou bien les substituants de l'un des couples (Rₐ,R_{b}), (R_{b},R_{c}) ou (R_{c},R_{d}) forment ensemble avec les atomes de carbone qui les portent un cycle constitué de 5 à 7 chaînons, pouvant contenir de un à 2 hétéroatomes choisis parmi oxygène et soufre, étant aussi entendu qu'un ou plusieurs atomes de carbone du cycle précédemment défini peut(vent) être deutéré(s) ou substitués par un à 3 groupements choisis parmi halogène ou alkyle (C₁-C₆) linéaire ou ramifié,
◆ R₇ et R₇' représentent indépendamment l'un de l'autre un hydrogène, un alkyle (C₁-C₆) linéaire ou ramifié, un alkényle (C₂-C₆) linéaire ou ramifié, un alkényle (C₂-C₆) linéaire ou ramifié, un aryle ou un hétéroaryle, ou bien R₇ et R₇' forment ensemble avec l'atome d'azote qui les porte un hétérocycle constitué de 5 à 7 chaînons,
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié éventuellement substitué, spiro (C₃-C₆), alkoxy (C₁-C₆) linéaire ou ramifié éventuellement substitué, (C₁-C₆)alkyl-S-, hydroxy, oxo (ou *N*-oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle éventuellement substitué, hétéroaryle, aryloxy, arylthio, cycloalkyle, hétérocycloalkyle éventuellement substitué par un ou plusieurs atomes d'halogène ou groupements alkyles, étant entendu que R' et R" représentent, indépendamment l'un de l'autre, un atome d'hydrogène ou un groupement alkyle (C₁-C₆) linéaire ou ramifié éventuellement substitué,
le groupement Het défini dans la formule (I') pouvant être substitué par un à trois groupements choisis parmi alkyle (C₁-C₆) linéaire ou ramifié, hydroxy, alkoxy (C₁-C₆) linéaire ou ramifié, NR₁'R₁", ou halogène, étant entendu que R₁' et R₁" ont les mêmes définitions que les groupes R' et R" mentionnés précédemment,
leurs énantiomères et diastéréoisomères, ainsi que leurs sels d'addition à un acide ou à une base pharmaceutiquement acceptable.

Les composés de formule (I') possèdent des propriétés pro-apoptotiques et présentent de ce fait un intérêt thérapeutique majeur dans le traitement des cancers, des maladies auto-immunes et du système immunitaire. Dans la présente invention, il a été montré qu'en administrant les composés phosphates de formule (I), l'exposition *in vivo* des composés de formule (I') était optimisée. En effet, la solubilité des composés de formule (I) est bien supérieure à celle des composés de formule (I'). Par conséquent, l'utilisation des composés de formule (I) pour la fabrication de compositions pharmaceutiques, est particulièrement avantageuse sur le plan galénique.

L'invention s'étend également au procédé de préparation des composés de formule (I) caractérisé en ce que l'on utilise comme produit de départ le composé de formule (II) : 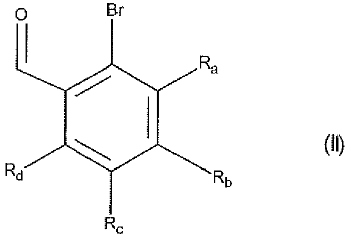 dans laquelle Rₐ, R_{b}, R_{c} et R_{d} sont tels que définis dans la formule (I'),
composé de formule (II) qui est soumis à une réaction de Heck, en milieu aqueux ou organique, en présence d'un catalyseur au palladium, d'une base, d'une phosphine et du composé de formule (III) : 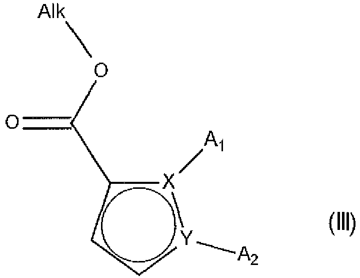 dans laquelle les groupements A₁, A₂, X et Y sont tels que définis dans la formule (I') et Alk représente un alkyle (C₁-C₆) linéaire ou ramifié,
pour obtenir le composé de formule (IV) : 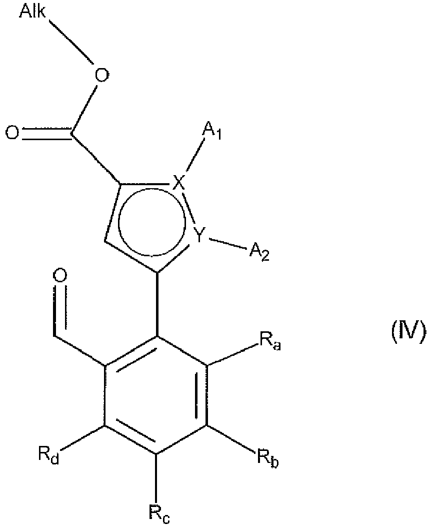 dans laquelle A₁, A₂, X, Y, Rₐ, R_{b}, R_{c} et R_{d} sont tels que définis dans la formule (I') et Alk est tel que défini précédemment,
composé de formule (IV) dont la fonction aldéhyde est oxydée en acide carboxylique pour former le composé de formule (V) : 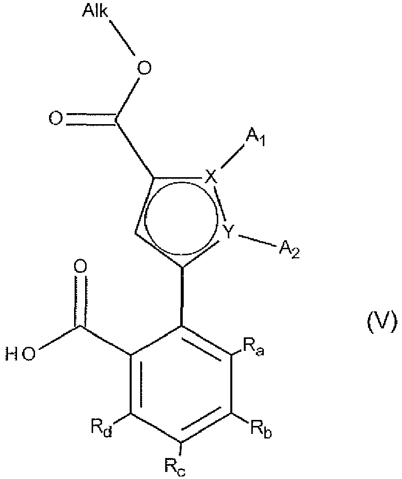 dans laquelle A₁, A₂, X, Y, Rₐ, R_{b}, R_{c} et R_{d} sont tels que définis dans la formule (I') et Alk est tel que défini précédemment,
composé de formule (V) qui subit ensuite un couplage peptidique avec un composé de formule (VI) : 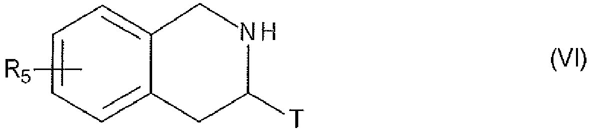 dans laquelle T et R₅ sont tels que définis dans la formule (I'),
pour conduire au composé de formule (VII) : 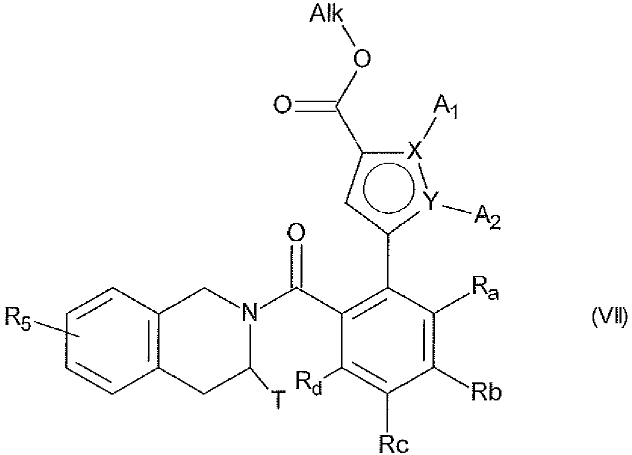 dans laquelle A₁, A₂, X, Y, Rₐ, R_{b}, R_{c}, R_{d}, T et R₅ sont tels que définis dans la formule (I') et Alk est tel que défini précédemment,
composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I'), avant d'être soumis à l'action d'un dérivé pyrophosphate, phosphonate ou phosphoryle dans des conditions basiques, le composé ainsi obtenu pouvant être optionnellement hydrolysé ou hydrogénolysé, pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

Les composés de formules (II), (III), (VI), ainsi que l'amine NHR₃R₄, sont soit commerciaux, soit accessibles à l'homme du métier par des réactions chimiques classiques et décrites dans la littérature.

Plus précisément, les composés phosphate de formule (I) selon l'invention seront utiles dans le traitement des cancers chimio ou radiorésistants, ainsi que dans les hémopathies malignes et le cancer du poumon à petites cellules.
Parmi les traitements des cancers envisagés on peut citer, sans s'y limiter, le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules. Parmi les lymphomes non hodgkiniens, on peut citer plus préférentiellement les lymphomes folliculaires, les lymphomes des cellules du manteau, les lymphomes diffus à grandes cellules B, les petits lymphomes lymphocytiques et les lymphomes à cellules B de la zone marginale.

La présente invention a également pour objet les compositions pharmaceutiques contenant au moins un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Par ailleurs, la présente invention concerne également une combinaison d'un composé de formule (I) avec un agent anticancéreux choisi parmi les agents génotoxiques, les poisons mitotiques, les anti-métabolites, les inhibiteurs du protéasome, les inhibiteurs de kinase, ou les anticorps, ainsi que les compositions pharmaceutiques contenant ce type d'association et leur utilisation pour la fabrication de médicaments utiles dans le traitement du cancer. Les composés de l'invention peuvent également être utilisés en association avec une radiothérapie dans le traitement du cancer.

Les Préparations et Exemples suivants illustrent l'invention et ne la limitent en aucune façon.

### Préparation 1 : Acide 6-[1-(méthoxycarbonyl)-5,6,7,8-tétrahydro-3-indolizinyl]-1,3-benzodioxole-5-carboxylique

### Stade A : Acide 1-formyl-2-pipéridine carboxylique

A une solution de 40 g d'un mélange racémique d'acide 2-pipéridine carboxylique (0,310 mmol) dans 300 mL d'acide formique placée à 0°C, on ajoute goutte à goutte 200 mL (2,15 mmol) d'anhydride acétique. L'ensemble est ensuite agité à température ambiante durant une nuit. Puis, le milieu réactionnel est refroidi à 0°C, hydrolysé par l'addition de 250 mL d'eau, et agité pendant une demi-heure à 0°C avant d'être concentré à sec. L'huile ainsi obtenue est reprise dans 200 mL de méthanol puis concentrée à sec. Le produit du titre est obtenu sous la forme d'une huile avec un rendement de 98%. Il est utilisé directement, sans autre purification, pour l'étape suivante.
**RMN ¹H :** δ (400 MHz; dmso-d6; 300°K): 13,0 (m, 1H OH); 8,0-8,05 (2s, 1H aldéhyde) ; 4,9-4,5 (2d, 1H α de N et COOH) ; 4,1-2,6 (m, 2H en α du N); 2,2-1,2 (m, 6H pipéridine)
**IR :** *v* : -OH : 2000-3000 cm⁻¹ acide ; ν : >C=O 1703 cm⁻¹ bande large

### Stade B : 5,6,7,8-Tétrahydro-1-indolizine carboxylate de méthyle

A une solution de 10 g d'acide carboxylique obtenu au Stade A (63,6 mmol) dans 65 mL de dichloroéthane sont ajoutés successivement 13,4 g de chlorure de tosyle (70,4 mmol), 11,5 mL de 2-chloroacrylate de méthyle (113,5 mmol), puis, au goutte à goutte, 17,8 mL de *N.N,N*-triéthylamine (127,2 mmol). Le milieu réactionnel est ensuite porté au reflux pendant 1h30. On se place ensuite à température ambiante, puis on ajoute 5 mL de 2-chloroacrylate de méthyle (48,9 mmol) et, au goutte à goutte, 9 mL de *N,N*,*N-*triéthylamine (64 mmol). L'ensemble est chauffé au reflux pendant une nuit.
Le milieu réactionnel est ensuite dilué avec du chlorure de méthylène, lavé successivement avec une solution HCl 1M, une solution aqueuse saturée en NaHCO₃, puis avec de la saumure jusqu'à obtenir un pH neutre. La phase organique est alors séchée sur MgSO₄, filtrée, concentrée à sec, et purifiée par chromatographie sur gel de silice (gradient heptane/ AcOEt). On obtient le produit du titre sous la forme d'une huile.
**RMN ¹H :** δ (400 MHz ; CDCl₃ ; 300°K) : 6,55-6,40 (d, 2H, tétrahydroindolizine) ; 3,91 (t, 3H méthyl ester); 3,78 (s, 3H tétrahydroindolizine); 3,08 (t, 2H, tétrahydroindolizine); 1,95-1,85 (m, 4H, tétrahydroindolizine)
**IR :** ν :>C=O 1692 cm⁻¹ ester

### Stade C : 3-(6-Formyl-1,3-benzodioxol-5-yl)-5,6,7,8-tétrahydro-1-indolizine carboxylate de méthyle

A une solution de 6,4 g de l'ester obtenu au Stade B (35,7 mmol) dans 12 mL de *N,N-*diméthylacétamide, on ajoute successivement 12,3g de 6-bromo-1,3-benzodioxole-5-carbaldéhyde (53,6 mmol), 7 g d'acétate de potassium (71,4 mmol), puis l'ensemble est agité sous argon pendant 20 minutes. On ajoute alors 1,3 g de catalyseur au palladium dichlorobis(triphénylphosphine)palladium(II) (PdCl₂(PPh₃)₂) (1,8 mmol). Le milieu réactionnel est ensuite chauffé à 130°C pendant une heure avant d'y ajouter 139 µL d'H₂O. Le chauffage est maintenu à cette même température pendant la nuit. On laisse le milieu revenir à température ambiante, puis on le dilue avec de l'AcOEt. On ajoute du noir animal (25 g par g de produit) et l'ensemble est agité à température ambiante pendant 1 heure, puis filtré. La phase organique est alors lavée avec de l'eau, séchée sur sulfate de magnésium et concentrée à sec. Le produit brut ainsi obtenu est purifié par chromatographie sur gel de silice (gradient heptane/ AcOEt). Le produit du titre est obtenu sous la forme d'une huile.
**RMN¹H** : δ (400 MHz ; dmso-d6 ; 353°K) : 9,65 (s, 1H, H aldéhyde) ; 7,3-7,15 (2s, 2H, H aromatiques) ; 6,45 (s, 1H tétrahydroindolizine) ; 6,20 (s, 2H méthylénedioxy); 3,70 (s, 3H méthyl ester) ; 3,5-4,0 (m, 2H tétrahydroindolizine) ; 3,05 (m, 2H tétrahydroindolizine) ; 1,85 (m, 4H tétrahydroindolizine)
**IR :** ν : >C=O 1695 cm⁻¹ ester; ν : >C=O 1674 cm⁻¹

### Stade D : Acide 6-[1-(méthoxycarbonyl)-5,6,7,8-tétrahydro-3-indolizinyl]-1,3-benzodioxole-5-carboxylique

On prépare une solution contenant 3,37 g du composé obtenu au Stade C (10,3 mmol) dans 9,3 mL d'acétone et 8,8 mL (80,24 mmol) de 2-méthyl-2-butène, laquelle est placée à 0°C. On ajoute, goutte à goutte, 9,3 mL d'une solution aqueuse contenant un mélange de 3,3 g de chlorite de sodium (NaClO₂) (36,05 mmol) et de 3,6 g de dihydrogénophosphate de sodium monohydrate (NaH₂PO₄) (25,75 mmol). L'ensemble est ensuite agité à température ambiante durant 7 heures. Puis, le milieu réactionnel est concentré pour éliminer l'acétone. Le solide alors obtenu est filtré, lavé à l'eau puis séché sous vide à 40°C pendant une nuit. Le produit du titre est obtenu sous la forme d'un solide, qui est utilisé pour la suite sans autre purification.
**RMN¹H :** δ (400 MHz ; dmso-d6 ; 300°K) : 12,10 (m, 1H, H acide carboxylique) ; 7,40-6,88 (2s, 2H, H aromatiques); 6,20 (s, 1H, H tétrahydroindolizine) ; 6,18 (s, 2H, H méthylènedioxy); 3,70 (s, 3H, méthyl ester) ; 3,55 (t, 2H tétrahydroindolizine) ; 3,00 (t, 2H tétrahydroindolizine) ; 1,80 (m, 4H, H tétrahydroindolizine)
**IR :** ν : -OH : 3000-2000 cm⁻¹ acide ; ν : >C=O 1686-1676 cm⁻¹ ester+acide ; ν : >C=C< 1608 cm⁻¹

### Préparation 2 : Acide 2-[1-(méthoxycarbonyl)-5,6,7,8-tétrahydro-3-indolizinyl]benzoïque

On procède selon le protocole décrit à la Préparation 1 en remplaçant 6-brome-1,3-benzodioxole-5-carbaldéhyde utilisé au Stade C par le 2-bromo-benzaldéhyde.

### Préparation 3 : Acide 6-[1-(méthoxycarbonyl)-3-indolizinyl]-1,3-benzodioxole-5-carboxylique

### Stade A : Bromure de 1-(carboxyméthyl)-1,2-dihydropyridinium

A une solution de 16,2 mL de pyridine (200 mmol) dans 120 mL d'acétate d'éthyle, on ajoute par portions 27,8 g (200 mmoles) d'acide bromoacétique. L'ensemble est ensuite agité à température ambiante pendant une nuit. Le précipité ainsi obtenu est filtré, puis lavé avec de l'acétate d'éthyle froid. Après séchage, on obtient le produit du titre sous la forme d'une poudre qui est utilisée directement pour l'étape suivante.
**RMN ¹H:** δ (400 MHz ; dmso-d6 ; 300°K) : 9,15 (d, 2H, H aromatiques pyridine) ; 8,7 (t, 1H, H aromatique) ; 8,25 (t, 2H, H aromatique); 5,65 (s, 2H, H CH₂COOH)
**IR :** ν : C=O: 1732 cm⁻¹ ; -OH acide : 2800 cm⁻¹

### Stade B : 1-Indolizinecarboxylate de méthyle

A une suspension de 6,55 g du sel de pyridinium obtenu au Stade A (30 mmol) dans 240 mL de toluène, on ajoute successivement 16,7 mL d'acrylate de méthyle (150 mmol), 4,2 mL de triéthylamine (30 mmol), puis par portions 20,9g de MnO₂ (240 mmol). L'ensemble est ensuite chauffé à 90°C durant 3 h. Après refroidissement, le milieu réactionnel est filtré sur un gâteau de célite et concentré à sec. Le produit du titre est alors isolé par purification sur gel de silice (gradient heptane / AcOEt : 0-10%) sous la forme d'une huile qui cristallise à froid.
**RMN ¹H:** δ (300 MHz ; dmso-d6 ; 300°K) : 8,5 (d, 1H, H indolizine) ; 8,05 (d, 1H, H indolizine); 7,6 (s, 1H, H indolizine); 7,15 (m, 2H, H indolizine); 6,85 (m, 1H, H indolizine); 4,25 (q, 2H, -C(O)CH₂CH₃); 1,35 (t, 3H, -C(O)CH₂CH₃)
**IR :** ν : C=O ester : 1675 cm⁻¹ ; C=C aromatiques : 1634 cm⁻¹

### Stades C : Acide 6-[1-(méthoxycarbonyl)-3-indolizinyl]-1,3-benzodioxole-5-carboxylique

On procède selon le protocole décrit aux Stades C et D de la Préparation 1.

### Préparation 4 : Acide 4-chloro-2-[4-(éthoxycarbonyl)-1,5-diméthyl-1H-pyrrol-2-yl]benzoïque

### Stale A : 1,2-Diméthyl-1H-pyrrole-3-carboxylate d'éthyle

A une solution de 10 g de **2-méthyl-1*H* pyrrole-3-carboxylate** d'éthyle (65,3 mmol) et 8,95 mL (130,6 mmol) d'iodure de méthyle dans 70 mL de diméthylformamide placée à 0°C, on ajoute en trois portions 2,61 g (65,3 mmol) d'hydrure de sodium à 60%. L'ensemble est ensuite agité à 0°C pendant 1 heure. Puis, le milieu réactionnel est hydrolysé par l'addition de 420 mL d'eau glacée. Le milieu réactionnel est ensuite dilué avec de l'acétate d'éthyle, lavé successivement avec une solution HCl 0,1 M, une solution aqueuse saturée en LiCl, puis de la saumure. La phase organique est alors séchée sur MgSO₄, filtrée, concentrée à sec, et purifiée par chromatographie sur gel de silice (gradient éther de pétrole/ AcOEt).
**RMN ¹H :** δ (400 MHz ; dmso-d6 ; 300K) : 6,65 (d, 1H pyrrole) ; 6,3 (1d, 1H pyrrole) ; 4,1 (1q, 2H, O**CH₂**CH₃); 3,5 (s, 3H N-pyrrole); 2,4 (s, 3H pyrrole) ; 1,5 (1t, 3H OCH₂**CH₃**) **IR :** ν : >C=O: 1688 cm⁻¹ ; ν : C-O-C: 1172 cm⁻¹

### Stade B : 5-(5-Chloro-2-formylphényl)-1,2-diméthyl-1H-pyrrole-3-carboxylate d'éthyle

A une solution de 10,5 g du composé obtenu au Stade A (62,8 mmol) dans 65 mL de *N*,*N-*diméthylacétamide, on ajoute successivement 15,2 g de 2-bromo-4-chlorobenzaldéhyde (69 mmol), 12,3 g d'acétate de potassium (125,6 mmol), puis l'ensemble est agité sous argon pendant 20 minutes. On ajoute alors 2,2 g de catalyseur au palladium PdCl₂(PPh₃)₂ (3,14 mmol). Le milieu réactionnel est ensuite chauffé à 130°C pendant la nuit. On laisse le milieu revenir à température ambiante, puis on le dilue avec du dichlorométhane. On ajoute du noir animal (30 g) et l'ensemble est agité à température ambiante pendant 1 heure, puis filtré. La phase organique est alors lavée avec de l'eau, séchée sur sulfate de magnésium et concentrée à sec. Le produit brut ainsi obtenu est purifié par chromatographie sur gel de silice (gradient éther de pétrole/ AcOEt). Le produit du titre est obtenu sous la forme d'un solide.
**RMN ¹H :** δ (400 MHz ; dmso-d6 ; 300K) : 9,8 (s, 1H, formyle) ; 7,91-7,69-7,61 (d, 3H aromatiques); 6,5 (s, 1H pyrrole); 4,2 (q, 2H, O**CH**₂CH₃ ); 3,4 (s, 3H, **CH₃**-N-pyrrole) ; 2,55 (s, 3H pyrrole) ; 1,28 (t, 3H, OCH₂**CH₃**)

### Stade C : Acide 4-chloro-2-[4-(éthoxycarbonyl)-1,5-diméthyl-1H-pyrrol-2-yl]benzoïque

On prépare une solution contenant 12,85 g du composé obtenu au Stade B (42 mmol) et 35,7 mL (336 mmol) de 2-méthyl-2-butène dans un mélange constitué de 20 mL d'acétone et 20 mL de tétrahydrofurane. On ajoute, goutte à goutte, 200 mL d'une solution aqueuse contenant un mélange de 13,3 g de chlorite de sodium (NaClO₂) (147 mmol) et de 14,5 g de dihydrogénophosphate de sodium monohydrate (NaH₂PO₄H₂O) (105 mmol). L'ensemble est ensuite agité énergiquement à température ambiante durant 7 heures. Puis, le milieu réactionnel est concentré pour éliminer l'acétone. On ajoute de l'acétate d'éthyle et on lave à l'eau la phase organique, puis on concentre à sec. Le résidu est ensuite repris dans un minimum d'éther éthylique. Le solide alors obtenu est filtré, lavé à l'éther puis séché sous vide à 40°C pendant une nuit. Le produit du titre est obtenu sous la forme d'un solide, qui est utilisé pour la suite sans autre purification.
**RMN¹H:** δ (400 MHz ; dmso-d6 ; 300K) : 13 (m, 1H COO**H**), ; 7,85-7,6-7,41(d,dd,df, 3H, H aromatiques) ; 6,3 (s, 1H, H pyrrole) ; 4,15 (q, 2H, **OCH₂**CH₃) ; 3,25 (s, 3H, **CH₃**-N-pyrrole); 2,5 (s, 3H, **CH**₃-pyrrole) ; 1,25 (t, 3H, OCH₂**CH₃)**
**IR :** ν : -OH : 3100-2500 cm⁻¹ acide ; ν : >C=O : 1681 cm⁻¹ ester + acide

### Préparation 5 : Acide 6-[4-(éthoxycarbonyl)-1,5-diméthyl-1H-pyrrol-2-yl]-1,3-benzodioxole-5-carboxylique

On procède selon le procédé de la Préparation 4 en remplaçant le 2-brome-4-chlorobenzaldéhyde utilisé au Stade B par le 6-bromo-1,3-benzodioxole-5-carbaldéhyde.

### Préparation 6 : Acide 4-fluoro-3-méthoxy-2-[4-(éthoxycarbonyl)-1,5-diméthyl-1H-pyrrol-2-yl]benzoïque

On procède selon le procédé de la Préparation 4 en remplaçant le 2-bromo-4-chlorobenzaldéhyde utilisé au Stade B par le 2-bromo-4-fluoro-3-méthoxybenzaldéhyde.

### Préparation 7 : Acide 4-fluoro-2-[4-(éthoxycarbonyl)-1,5-diméthyl-1H-pyrrol-2-yl]benzoïque

On procède selon le procédé de la Préparation 4 en remplaçant le 2-bromo-4-chlorobenzaldéhyde utilisé au Stade B par le 2-bromo-4-fluorobenzaldéhyde.

### Préparation 8 : Acide 7-[4-(méthoxycarbonyl)-1,5-diméthyl-1H-pyrrol-2-yl]-2,3-dihydro-1,4-benzodioxine-6-carboxylique

On procède selon le procédé de la Préparation 4 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 7-bromo-2,3-dihydro-1,4-benzodioxine-6-carbaldéhyde.

### Préparation 9: Acide 5-benzyloxy-2-(1-méthoxycarbonyl-5,6,7,8-tétrahydroindolizin-3-yl)benzoïque

### Stade A: 3-(4-Benzyloxy-2-formyl-phényl)-5,6,7,8-tétrahydroindolizine-1-carboxylate de méthyle

Le 5-benzyloxy-2-bromo-benzaldéhyde (12,3 g, 42,2 mmol) est introduit dans un ballon en présence d'acétate de potassium (8,3 g ; 84,2 mmol) et 120 mL de diméthylacétamide. Après dégazage sous argon, le dichlorobis(triphenylphosphine)palladium (II) (1,04 g, 1,5 mmol) est ajouté, puis le mélange est dégazé sous argon avant d'être chauffé à 100°C pendant 16 h. Après retour à température ambiante, le milieu réactionnel est versé sur 200 mL d'acétate d'éthyle, filtré sur célite, lavé avec de l'eau, puis avec de la saumure. Les phases aqueuses réunies sont extraites à l'acétate d'éthyle. Les phases organiques sont séchées sur sulfate de sodium, filtrées et concentrées sous pression réduite. Le résidu obtenu est purifié par chromatographie sur gel de silice afin d'obtenir le produit du titre.

### Stade B: Acide 5-benzyloxy-2-(1-méthoxycarbonyl-5,6,7,8-tétrahydroindolizin-3-yl)benzoïque

A une solution du composé obtenu au Stade B (4,63 g, 11,89 mmol) dans 300 mL d'acétone est ajouté le 2-méthyl-2-butène (6,31 mL, 59 mmol). Une solution de dihydrogénophosphate de sodium monohydrate (6,56 g, 47,6 mmol) et de chlorite de sodium (2,69 g, 23,8 mmol) dans 40 mL d'eau est ensuite coulée goutte à goutte en maintenant la température inférieure à 20°C. Après 30 min d'agitation à température ambiante, le mélange est acidifié par une solution de HCl 2M, puis décanté. La phase aqueuse est extraite à l'acétate d'éthyle. Les phases organiques réunies sont séchées sur sulfate de sodium, filtrées et évaporées à sec pour fournir le composé attendu.

### Préparation 1' : (3S)-3-(4-Morpholinylméthyl)-1,2,3,4-tétrahydroisoquinoline

### Stade A: (3S)-3-(4-Morpholinylcarbonyl)-3,4-dihydro-2(1H)-isoquinoline carboxylate de benzyle

A une solution de 5 g d'acide (3*S*)-2-[(benzyloxy)carbonyl]-1,2,3,4-tétrahydro-3-isoquinolinecarboxylique (16 mmol) dans 160 mL de dichlorométhane sont ajoutés 1,5 mL de morpholine (17,6 mmol), puis 9 mL de *N,N,N*-triéthylamine (64 mmol), 3,3 g de 1-éthyl-3-(3'-diméthylaminopropyl)-carbodiimide (EDC) (19,2 mmol), et 2,6 g d'hydroxybenzotriazole (HOBT) (19,2 mmol). Le milieu réactionnel est agité à température ambiante pendant une nuit, puis il est versé sur une solution aqueuse de chlorure d'ammonium et extrait avec de l'acétate d'éthyle. La phase organique est ensuite séchée sur sulfate de magnésium, puis filtrée et évaporée à sec. Le produit brut ainsi obtenu est alors purifié par chromatographie sur gel de silice (gradient dichlorométhane/ méthanol). Le produit est obtenu sous la forme d'une mousse.
**RMN¹H:** δ (400 MHz ; dmso-d6 ; 353°K) : 7,30 (m, 5H benzyl); 7,15 (m, 4H aromatiques) ; 5,2-5,0 (m, 3H, 2H benzyl, 1H dihydroisoquinoline); 4,75-4,5 (2d, 2H dihydroisoquinoline); 3,55-3,3 (m, 8H morpholine); 3,15-2,9 (2dd, 2H dihydroisoquinoline)
**IR** : ν : >C=O : 1694 ; 1650 cm⁻¹

### Stade B : (3S)-3-(4-Morpholinylméthyl)-3,4-dihydro-2(1H)-isoquinoline carboxylate de benzyle

A une solution de 5,3 g du produit obtenu au Stade A (13,9 mmol) dans 278 mL de tétrahydrofurane sont ajoutés 14 mL de complexe borane-diméthylsulfure (BH₃-Me₂S) (27,8 mmol) à température ambiante. Le tout est chauffé pendant 4 heures à 80°C. On laisse revenir à température ambiante, puis on ajoute 7 mL (14 mmol) de BH₃-Me₂S. Le milieu réactionnel est de nouveau chauffé à 80°C pendant 2 heures. On évapore ensuite le tétrahydrofurane, puis on ajoute lentement du méthanol, puis 5,6 mL d'acide chlorhydrique 5M (27,8 mmol). Le mélange est agité à température ambiante pendant une nuit, puis à 80°C pendant 1h. On ajoute ensuite une solution aqueuse saturée en NaHCO₃ sur le milieu réactionnel placé à 0°C jusqu'à atteindre un pH=8, puis on extrait avec de l'acétate d'éthyle. La phase organique est ensuite séchée sur sulfate de magnésium, puis filtrée et évaporée à sec. Le produit du titre est obtenu sous la forme d'une huile.
**RMN¹H :** δ (400 MHz ; dmso-d6 ; 353°K) : 7,43-7,30 (massif, 5H benzyl) ; 7,19 (m, 4H aromatiques) ; 5,16 (m, 2H, 2H benzyl) ; 4,79-4,29 (d, 2H dihydroisoquinoline) ; 4,58 (m, 1H dihydroisoquinoline) ; 3,50 (m, 4H morpholine) ; 3,02-2,80 (dd, 2H dihydroisoquinoline) ; 2,42-2,28 (massif, 5H, 4H morpholine, 1H morpholine) ; 2,15 (dd, 1H morpholine)
**IR :** ν : >CH : 2810 cm⁻¹ ; ν : >C=O : 1694 cm⁻¹ ; ν : >C-O-C< : 1114 cm⁻¹ ; ν : >CH-Ar : 751 ; 697 cm⁻¹

### Stade C : (3S)-3-(4-Morpholinylméthyl)-1,2,3,4-tétrahydroisoquinoline

A une solution de 4,9 g du composé du Stade B (13,4 mmol) dans 67 mL d'éthanol est ajouté 0,980 g de dihydroxyde de palladium (20% massique) à température ambiante. Le milieu réactionnel est placé sous 1,2 bar de d'hydrogène à température ambiante pendant 4 heures. Il est ensuite passé sur une filtre Wathman, puis le palladium est rincé plusieurs fois avec de l'éthanol. Le filtrat est évaporé à sec, Le produit du titre est obtenu sous la forme d'une huile.
**RMN¹H:** δ (400 MHz ; dmso-d6 ; 300°K) : 7,12-7,0 (massif, 4H aromatiques) ; 3,92 (s, 2H tétrahydroisoquinoline) ; 3,60 (t, 4H morpholine) ; 2,98 (m, 1H tétrahydroisoquinoline) ; 2,68 (dd, 1H tétrahydroisoquinoline) ; 2,5-2,3 (massif, 8H, 1H tétrahydroisoquinoline, 6H morpholine, 1H NH)
**IR :** ν : >NH : 3322 cm⁻¹ ; ν : >C-O-C< : 1115 cm⁻¹ ; ν : >CH-Ar : 742 cm⁻¹

### Préparation 2' : Chlorhydrate de (3R)-3-méthyl-1,2,3,4-tétrahydroisoquinoline

### Stade A : {(3S)-2-[(4-Méthylphényl)sulfonyl]-1,2,3,4-tétrahydroisoquinolin-3-yl}méthyl 4-méthylbenzènesulfonate

A une solution de 30,2 g de [(3*S*)-1,2,3,4-tétrahydroisoquinolin-3-yl]méthanol (185 mmol) dans 750 mL de dichlorométhane sont ajoutés successivement 91,71 g de chlorure de tosyle (481 mmol), puis, au goutte à goutte, 122,3 mL de *N,N,N*-triéthylamine (740 mmol). Le milieu réactionnel est ensuite agité à température ambiante durant 20 h. Il est ensuite dilué avec du dichlorométhane, lavé successivement avec une solution HCl 1M, une solution aqueuse saturée en NaHCO₃, puis de la saumure jusqu'à neutralité. La phase organique est alors séchée sur MgSO₄, filtrée, concentrée à sec. Le solide obtenu est alors solubilisé dans un volume minimum de dichlorométhane, puis est ajouté du cyclohexane jusqu'à formation d'un précipité. Ce précipité est alors filtré et lavé avec du cyclohexane. Après séchage, le produit du titre est obtenu sous forme de cristaux.
**RMN ¹H :** δ (400 MHz ; dmso-d6 ; 300°K) : 7,75 (d, 2H , H aromatiques, *ortho* O-tosyle); 7,6 (d, 2H, H aromatiques, *ortho N*-tosyle); 7,5 (d, 2H, H aromatiques, *méta* O-tosyle); 7,3 (d, 2H, H aromatiques, *méta N*-tosyle); 7,15-6,9 (m, 4H, H aromatiques, tétrahydro isoquinoline); 4,4-4,15 (dd, 2H, H aliphatiques, tétrahydroisoquinoline); 4,25 (m, 1H, H aliphatique, tétrahydroisoquinoline); 4,0-3,8 (2dd, 2H, H aliphatiques, **CH₂**-O-tosyle) ; 2,7 (2dd, 2H, H aliphatiques, tétrahydroisoquinoline); 2,45 (s, 3H, O-SO₂-Ph-**CH₃**); 2,35 (s, 3H, *N*-SO₂-Ph-**CH₃)**
**IR** : ν : -SO₂ : 1339-1165 cm⁻¹

### Stade B : (3R)-3-Méthyl-2-[(4-méthylphényl)sulfonyl]-1,2,3,4-tétrahydroisoquinoline

A une suspension de 8,15 g (214,8 mmol) de LiAlH₄ dans 800 mL de méthyl *tert*-butyl éther (MTBE), on ajoute 101,2 g du dérivé ditosylé obtenu au Stade A (214,8 mmol) en solution dans 200 mL de MTBE. L'ensemble est ensuite chauffé à 50°C pendant 2h. On laisse refroidir et on se place à 0°C, puis on ajoute, goutte à goutte, 12 mL d'une solution de NaOH 5M. L'ensemble est agité à température ambiante pendant 45 minutes. Le solide ainsi obtenu est alors filtré, lavé avec du MTBE puis avec du dichlorométhane. Le filtrat est ensuite concentré à sec. On obtient alors le produit du titre sous la forme d'un solide
**RMN ¹H :** δ (400 MHz ; dmso-d6 ; 300°K) : 7,70 (d, 2H, H aromatiques, *ortho N-*tosyle); 7,38 (d, 2H, H aromatiques, *méta N-*tosyle); 7,2-7,0 (m, 4H, H aromatiques, tétrahydroisoquinoline); 4,4 (m, 2H, H aliphatiques, tétrahydroisoquinoline); 4,3 (m, 1H, H aliphatique, tétrahydroisoquinoline); 2,85-2,51 (2dd, 2H, H aliphatiques, tétrahydro isoquinoline); 2,35 (s, 3H, *N*-SO₂-Ph- **CH₃**) ; 0,90 (d, 3H, tétrahydroisoquinoline-**CH₃)**
**IR :** ν : -SO₂ : 1332-1154 cm⁻¹

### Stade C : (3R)-3-Méthyl-1,2,3,4-tétrahydroisoquinoline

A une solution de 31,15 g (103,15 mmol) du dérivé monotosylé obtenu au Stade B dans 500 mL de méthanol anhydre, on ajoute 3,92g (161 mmol) de tournure de magnésium par pelletés. L'ensemble est agité en présence d'ultra-sons pendant 96 h. Le milieu réactionnel est ensuite filtré et le solide est lavé plusieurs fois avec du méthanol. Le filtrat est ensuite concentré à sec. Après purification par chromatographie sur gel de silice (gradient dichlorométhane /EtOH /NH₄OH) on obtient le produit du titre sous la forme d'une huile.
**RMN ¹H :** δ (400 MHz ; dmso-d6 ; 300°K) : 7,05 (m, 4H, H aromatiques, tétrahydroisoquinoline); 3,90 (m, 2H, H aliphatiques, tétrahydroisoquinoline); 2,85 (m, 1H, H aliphatique, tétrahydroisoquinoline); 2,68-2,4 (2dd, 2H, H aliphatiques, tétrahydro isoquinoline); 1,12 (d, 3H, tétrahydroisoquinoline-**CH₃**) ; 2,9-2,3 (m, large, 1H, **HN**(tétrahydroisoquinoline))
**IR** : ν : -NH : 3248 cm⁻¹

### Stades D : Chlorhydrate de (3R)-3-méthyl-1,2,3,4-tétrahydroisoquinoline

A une solution de 14,3 g (97.20 mmol) du composé obtenu au Stade C dans 20 mL d'éthanol anhydre, on ajoute, goutte à goutte, 100 mL d'une solution d'éther chlorhydrique 1M. L'ensemble est agité à température ambiante pendant 1h, puis filtré. Les cristaux ainsi obtenus sont lavés avec de l'éther éthylique. Après séchage, le produit du titre sous la forme de cristaux.
**RMN ¹H :** δ (400 MHz ; dmso-d6 ; 300°K) : 9,57 (m, large, 2H, **NH₂**⁺ (tétrahydro isoquinoline) ; 7,22 (m, 4H, H aromatiques, tétrahydroisoquinoline); 4,27 (s, 2H, H aliphatiques, tétrahydroisoquinoline); 3,52 (m, 1H, H aliphatique, tétrahydroisoquinoline); 3,03-2,85 (2dd, 2H, H aliphatiques, tétrahydroisoquinoline); 1,39 (d, 3H, tétrahydro isoquinoline**-CH₃**)
**IR :** *ν* : -NH₂⁺ : 3000-2300 cm⁻¹; *ν* : -CH aromatique : 766 cm⁻¹

### Préparation 3' : (3R)-3-[3-(Morpholin-4-yl)propyl]-1,2,3,4-tétrahydroisoquinoline

### Stade A : {(3S)-2-[(4-Méthylphényl)sulfonyl]-1,2,3,4-tétrahydroisoquinolin-3-yl}méthyl 4-méthylbenzènesulfonate

Le procédé est identique à celui du Stade A de la Préparation 2'.

### Stade B : 2-({(3R)-2-[(4-Méthylphényl)sulfonyl]-1,2,3,4-tétrahydroisoquinolin-3-yl}méthyl)-3-(morpholin-4-yl)-3-oxopropanoate de tert-butyle

A une suspension de 1 g de NaH (60%) (25,08 mmol) dans 30 mL de MTBE, on ajoute goutte à goutte une solution de 5 g de 3-morpholino-3-oxopropanoate de *tert*-butyle (21,81 mmol) dans 20 mL de MTBE anhydre. Cette suspension est agitée à température ambiante pendant 1h, puis on ajoute le composé obtenu au Stade A sous forme d'une poudre. L'ensemble est agité à 60°C pendant 30h. Une solution de 100 mL d'une solution aqueuse saturée en chlorure d'ammonium est ajoutée. Cette solution est extraite au dichlorométhane. La phase organique est alors séchée sur MSO₄, filtrée et concentrée à sec. Après purification par chromatographie sur gel de silice (gradient dichlorométhane/MeOH), on obtient le produit attendu sous la forme d'une huile.
**RMN ¹H** (500 MHz, dmso-d6) δ ppm : 7,63/7,59 (2d, 2 H) ; 7,3/7,26 (2d, 2 H) ; 7,13 (m, 2 H) ; 7,09/6,97 (2t, 2 H) ; 4,64/4,55/4,36/4,28 (2AB, 2 H} ; 4,25/4,11 (2m, 1 H) ; 3,81 (m, 1 H); 3,73-3,48 (m, 4 H); 3,57-3,32 (m, 4 H); 2,51 (m, 2 H); 2,32/2,31 (2s, 3 H); 1,88/1,79 (2m, 2 H) ; 1,39/1,38 (2s, 9 H)
**IR (ATR)** cm⁻¹: *ν* : >C=O : 1731 (ester); *ν* : >C=O : 1644 (amide); *ν* : -SO2 : 1334-1156 ; *ν* : >C-O-C< : 1115; γ : >CH-Ar: 815-746-709

### Stade C : Acide 2-({(3R)-2-[(4-méthylphényl)sulfonyl]-1,2,3,4-tétrahydroisoquinolin-3-yl}méthyl)-3-(morpholin-4-yl)-3-oxopropanoique

A une solution de 9,5 g (17,97 mmol) du composé obtenu au Stade B dans 40 mL de dioxane, on ajoute goutte à goutte 20 mL d'une solution d'acide chlorhydrique 4M dans le dioxane. L'ensemble est agité à température ambiante pendant 48h, puis la solution est concentrée à sec. Après séchage, on obtient le produit attendu sous la forme d'une huile.
**RMN ¹R** (400 MHz, dmso-d6) δ ppm : 12,75 (m, 1 H) ; 7,6 (2*d, 2 H) ; 7,3 (2*d, 2 H) ; 7,1/6,95 (2*m, 4 H) ; 4,7-4,2 (d, 2 H) ; 4,25/4,12 (2*m, 1 H) ; 3,9-3,3 (m, 9 H) ; 2,55 (d, 2 H); 2,3 (2*s, 3 H) ; 1,8 (t, 2 H).
**IR (ATR)** cm⁻¹ : *ν* : -OH : 3500 à 2000 ; *ν* : >C=O : 1727 (acide) ; *ν* : >C=O : 1634 (amide); *ν* : -S02 : 1330-1155

### Stade D : 3-{(3R)-2-[(4-Méthylphényl)sulfonyl]-1,2,3,4-tétrahydroisoquinolin-3-yl}-1-(morpholin-4-yl)propan-1-one

A une solution de 7,80 g (16,51 mmol) du composé obtenu au Stade C dans 100mL de DMSO, on ajoute 1,16 g (19,83 mmol) de chlorure de sodium solide, puis goutte à goutte 5 mL d'eau. L'ensemble est agité à 130°C pendant 1h, puis la solution est concentrée au ¾. Le milieu réactionnel est ensuite dilué avec du dichlorométhane, lavé successivement avec une solution aqueuse saturée de chlorure de lithium, puis de la saumure. La phase organique est alors séchée sur MgSO₄, filtrée, concentrée à sec. Après purification par chromatographie sur gel de silice (gradient cyclohexane/acétate d'éthyle), on obtient le produit attendu sous la forme d'une huile.
**RMN ¹H** (400 MHz, dmso-d6) δ ppm : 7,65 (d, 2 H) 7,3 (d, 2 H) ; 7,15/7 (2 m, 4 H) ; 4,6 (d, 1 H) ; 4,25 (d, 1 H) ; 4,2 (m, 1 H) ; 3,5 (m, 4 H) ; 3,4 (2 m, 4 H) ; 2,6 (2 dd, 2 H) ; 2,35 (s, 3 H) ; 2,3 (m, 2 H) ; 1,5 (quad, 2 H)
**IR (ATR)** cm⁻¹ : *ν* : >C=O : 1639 ; *ν* : -SO2 : 1331-1156 ; γ : >CH-Ar : 815-675

### Stade E : (3R)-2-[(4-Méthylphényl)sulfonyl]-3-[3-(morpholin-4-yl)propyl]-1,2,3,4-tétrahydroisoquinoline

A une solution de 6,0 g (14,0 mmol) du composé obtenu au Stade D dans 60mL de MTBE et 14 mL de dichlorométhane, on ajoute 1,06g (28 mmol) de LAH par portion sur 5 minutes. L'ensemble est agité à température ambiante pendant 15 h. On ajoute goutte à goutte 1,5 mL d'eau et on agite pendant 15min. Ensuite, on ajoute goutte à goutte 1,5mL de soude 5 M et on agite 15min. Le milieu réactionnel est ensuite dilué avec du MTBE et du dichlorométhane. Puis, la suspension est filtrée et le précipité est lavé avec du MTBE et du dichlorométhane. La phase organique est alors séchée sur MgSO₄, filtrée, et concentrée à sec. Après purification par chromatographie sur gel de silice (gradient dichlorométhane /EtOH /NH₄OH), on obtient le produit attendu sous la forme d'une huile.
**RMN ¹H** (400 MHz, dmso-d6) δ ppm : 7,68 (d, 2 H) ; 7,32 (d, 2 H) ; 7,1 (massif, 4 H) ; 4,65/4,23 (AB, 2 H) ; 4,2 (m, 1 H) ; 3,55 (t, 4 H) ; 2,7/2,6 (ABx, 2 H) ; 2,35 (s, 3 H) ; 2,25 (t, 4 H) ; 2,2 (t, 2 H) ; 1,4/1,3 (2m, 4 H).
**IR (ATR)** cm⁻¹ : *ν* : -SO2 : 1333-1158

### Stade F : (3R)-3-[3-(Morpholin-4-yl)propyl]-1,2,3,4-tétrahydroisoquinoline

A une solution de 1,50 g (3,62 mmol) du dérivé obtenu au Stade E dans 20 mL de méthanol anhydre, on ajoute 2,0 g (82,3 mmol) de tournure de magnésium par pelletés. L'ensemble est agité en présence d'ultrasons pendant 96 h. Le milieu réactionnel est ensuite filtré, le solide est lavé plusieurs fois avec du méthanol, et le filtrat est concentré à sec. Après purification par chromatographie sur gel de silice (gradient dichlorométhane /EtOH /NH₄OH), on obtient le produit attendu sous la forme d'une huile.
**RMN ¹H** (400 MHz, dmso-d6) δ ppm : 7,3 (d, 2 H) ; 7,1 (t, 2 H) ; 7,1 (d+t, 3 H) ; 7 (d, 2 H) ; 3,9 (s, 2 H) ; 3,55 (t, 4 H) ; 2,75 (m, 1 H) ; 2,72/2,45 (dd, 2 H) ; 2,35 (t, 4 H) ; 2,25 (t, 2 H); 1,6 (m, 2 H); 1,45 (m, 2 H)
**IR (ATR)** cm⁻¹ : *ν* : >NH2+/NH+ : 3500-2300 ; *ν* : >C-O-C< : 1115

### Masse haute résolution (ESI+-/FIA/HR) :

Formule brute : C₁₆ H₂₄ N₂ O
[M+H]⁺ calculé: 261,1961
[M+H]⁺ mesuré : 261,1959

### Préparation 4': (3R)-3-(4-Morpholinylméthyl)-1,2,3,4-tétrahydroisoquinoline

On procède selon le procédé de la Préparation 1' en remplaçant l'acide (3*S*)-2-[(benzyloxy)carbonyl]-1,2,3,4-tétrahydro-3-isoquinolinecarboxylique utilisé au Stade A par l'acide (3*R*)-2-[(benzyloxy)carbonyl]-1,2,3,4-tétrahydro-3-isoquinolinecarboxylique.

### Préparation 1" : 4-{[tert-Butyl(diméthyl)silyl]oxy}-N-phénylaniline

A une solution de 12 g de 4-anilinophénol (64,7 mmol) dans 200 mL d'acétonitrile sont ajoutés à température ambiante 6,7 g d'imidazole (97,05 mmol) et 11,7 g de *tert-*butyl(chloro)diméthylsilane (77,64 mmol). L'ensemble est mis sous agitation à 70°C pendant 4 heures. Puis, le milieu réactionnel est versé sur de l'eau et extrait avec de l'éther. La phase organique est ensuite séchée sur sulfate de magnésium, puis filtrée et évaporée à sec. Le produit brut ainsi obtenu est alors purifié par chromatographie sur gel de silice (gradient éther de pétrole / dichlorométhane). Le produit du titre est obtenu sous la forme d'une poudre.
**RMN¹H:** δ (400 MHz ; dmso-d6 ; 300°K) : 7,84 (s, 1H NH) ; 7,17 (t, 2H aniline) ; 6,98 (d, 2H phénoxy); 6,94 (d, 2H aniline) ; 6,76 (d, 2H phénoxy) ; 6,72 (t, 1H aniline) ; 0,95 (s, 9H *tert*-butyl) ; 0,15 (s, 6H diméthyl)
**IR :** *ν* : >NH : 3403 cm⁻¹ ;>Ar : 1597 cm⁻¹

### Préparation 2" : N-(4-{[tert-Butyl(diméthyl)silyl]oxy}phényl)-1-méthyl-1H-indol-5-amine

On procède selon le procédé de la Préparation 5" en remplaçant le 4-bromo-1-méthyl-1*H-*pyrazole utilisé au Stade B par le le 5-bromo-1-méthyl-1*H*-indole.

### Préparation 3" : N-(4-{[tert-Butyl(diméthyl)silyl]oxy}phényl)-1-méthyl-1H-pyrrolo[2,3-b]pyridin-5-amine

On procède selon le procédé de la Préparation 5" en remplaçant le 4-bromo-1-méthyl-1*H-*pyrazole utilisé au Stade B par la 5-bromo-1-méthyl-1*H*-pyrrolo[2,3-*b*]pyridine (obtenue selon un protocole de la littérature : Heterocycles, 60(4), 865, 2003).
**IR :** *ν* :-NH- : 3278 cm⁻¹; *ν* :-C=C- aromatiques: 1605 cm⁻¹

### Préparation 4": N-(4-{[tert-Butyl(diméthyl)silyl]oxy}phényl)pyridin-4-amine

On procède selon le procédé de la Préparation 5" en remplaçant le 4-bromo-1-méthyl-1*H-*pyrazole utilisé au Stade B par la 4-bromopyridine.
**IR :** *ν* -NH- : 3200 et 2500 cm⁻¹; *ν* -Si-O-: 902 cm⁻¹; *ν* -Si-C-: 820 cm⁻¹

### Préparation 5" : N-(4-{[tert-Butyl(diméthyl)silyl]oxy}phényl)-1-méthyl-1H-pyrazol-4-amine

### Stade A : 4-{[tert-Butyl(diméthyl)silyl]oxy}aniline

Le composé du titre est obtenu à partir de la 4-aminophénol dans le THF en présence d'imidazole et de *tert*-butyl(chloro)diméthylsilane selon le protocole décrit dans la littérature (S. Knaggs et al, Organic & Biomolecular Chemistry, 3(21), 4002-4010; 2005**).**
**RMN ¹H.** δ (400 MHz ; dmso-d6 ; 300K) : 6,45-6,55 (dd, 4H, H aromatiques); 4,60 (m, 2H, **NH₂**-Ph) ; 0,90 (s, 9H, Si (CH₂)₂CH(**CH₃**)₂); 0,10 (s, 6H, Si (**CH₂**)₂CH(CH₃)₂)
**IR :** *ν* : -NH₂⁺ : 3300-3400 cm⁻¹

### Stade B: N-[4-[tert-Butyl(diméthyl)silyl]oxyphényl]-1-méthyl-pyrazol-4-amine

A une solution de 30,8g (0,137 mol) du composé du Stade A dans 525 mL de toluène anhydre, on ajoute successivement 29,8g de *tert*-butylate de sodium (0,310 mol), 4,55 g de Pd₂(dba)₃ (aussi appelé tris(dibenzylideneacetone)dipalladium(0)) (4,96 mmol), 4,81 g de 2-di-*tert*-butylphosphino-2',4',6'-tri-isopropyl-1,1'-biphényle (9,91 mmol) et 12,8 mL de 4-bromo-1-méthyl-1*H-*pyrazole (0,124 mol). L'ensemble est dégazé sous argon durant 30 mn puis chauffé à reflux pendant 3 h. On laisse refroidir. Le milieu réactionnel est concentré à sec, puis repris dans le dichlorométhane, filtré sur célite, puis de nouveau concentré à sec. Le résidu est alors purifié par chromatographie sur gel de silice (gradient CH₂Cl₂/AcOEt) pour fournir le produit attendu sous la forme d'un solide.
**RMN ¹H :** δ (400 MHz ; dmso-d6 ; 300K) : 7,55 (s, 1H, pyrazole); 7,23 (s, 1H, pyrazole); 7,18 (large s, 1H, **NH₂**-Ph), 6,64 (m, 4H, H aromatiques); 3,77 (s, 3H, **CH₃**-pyrazole); 0,90 (s, 9H, Si (CH₂)₂CH(**CH₃**)₂); 0,12 (s, 6H, Si (**CH₂**)₂CH(CH₃)₂)
**IR :** *ν* -NH⁺ : 3275 cm⁻¹; *ν* Ar et C=N : 1577 et 1502 cm⁻¹ ; *ν* -Si-C-: 1236 cm⁻¹; *ν* -Si-O-: 898 cm⁻¹; *ν* -Si-C-: 828, 774 cm⁻¹

### Préparation 6" : N-{4-[(tert-Butyldiméthylsilyl)oxylphényl}-1-trideutériométhyl-1H-pyrazol-4-amine

### Stade A : 4-Bromo-1-trideutériométhyl-1H-pyrazole

Le 4-bromo-1*H*-pyrazole (9,05 g, 61,6 mmol) est ajouté par portions à une suspension d'hydrure de sodium (60% dans l'huile) (2,83 g, 70,8 mmol) dans du tétrahydrofurane (90 mL) refroidie dans un bain de glace. Après avoir retiré le bain de glace, la solution est agitée à la température ambiante pendant 0,5 h. Celle-ci est à nouveau refroidie dans un bain de glace et de l'iodométhane-*d*₃ (5,0 mL, 80,3 mmol) est ajouté. La solution est agitée à la température ambiante durant 19 h. La suspension est ensuite concentrée. Le résidu d'évaporation est trituré avec du *tert*-butyl méthyl éther (90 mL) et filtré. Le filtrat est concentré sous vide pour obtenir le composé attendu sous la forme d'une huile.
**RMN ¹H** (400 MHz, CDCl₃) δ ppm : 7,37 (s, 1 H) ; 7,43 (s, 1 H)

### Stade B: N-{4-[(tert-Butyldiméthylsilyl)oxy]phényl}-1-trideutériométhyl-1H-pyrazol-4-amine

Du 4-bromo-1-trideutériométhyl-1*H-*pyrazole (9,6 g, 58,5 mmol), de la 4-[(*tert-*butyldiméthylsilyl)oxy]aniline (14,4 g, 64,6 mmol) et du toluène (150 mL) sont ajoutés dans un tricol de 500 mL. La solution est dégazée avec de l'azote durant 15 minutes, puis du *tert*-butylate de sodium (11,4 g, 0,12 mol), du 2-di-*tert*-butylphosphino-2',4',6'-triisopropylbiphényle (0,77 g, 1,81 mmol) et du tris(dibenzylideneacétone)dipalladium(0) (1,64 g, 1,79 mmol) sont successivement ajoutés. La suspension est chauffée à 85°C durant 1.5 h. Le mélange réactionnel est ensuite refroidi à la température ambiante et de l'eau (270 mL) est ajoutée. Le mélange est agité durant 30 minutes. Puis, on additionne de la Célite (30 g) et la suspension est filtrée sur un lit de Célite. Les phases du filtrat sont séparées et la phase aqueuse est extraite avec de l'acétate d'éthyle (3 x 200 mL). Les phases organiques combinées sont séchées avec du sulfate de sodium et filtrées. Le produit est purifié par chromatographie sur gel de silice (gradient acétate d'éthyle/heptane). Le produit obtenu est recristallisé dans l'heptane (80 mL) pour obtenir le composé attendu.
**RMN ¹H** (400 MHz, CDCl₃ δ ppm: 0,16 (s, 6 H); 0,97 (s, 9 H); 4,92 (s, 1 H); 6,61-6,73 (m, 4 H); 7,25 (s, 1 H); 7,36 (s, 1 H)
**RMN ¹³C** (100 MHz, CDCl₃) δ ppm: -4,37; 18,28; 25,86; 38,67 (sept, ¹*J*_{C-D} = 21,0 Hz); 115,12; 120,73; 123,76; 126,52; 134,74; 141,07; 148,43
**MS (ESI):** [M+H]⁺ 307,08

### Préparation 7": 4-({4-[(tert-Butyldiméthylsilyl)oxy]phényl}amino)-1,5-diméthyl-1H-pyrrole-2-carbonitrile

### Stade A : 4-Bromo-1,5-diméthyl-1H-pyrrole-2-carbonitrile

Une solution de brome (6,58 mL, 0,13 mol) dans l'acide acétique (60 mL) est ajoutée goutte-à-goutte à l'aide d'une ampoule à addition dans une solution de 1,5-diméthyl-1*H* pyrrole-2-carbonitrile (15,0 g, 0,12 mol) dans l'acide acétique (300 mL). L'ensemble est agité à la température ambiante pendant 24 h. Le mélange réactionnel est ensuite versé dans un bécher contenant 300 mL d'eau. Le solide formé est filtré et rincé avec de l'eau. Puis, il est solubilisé dans le dichlorométhane (300 mL) et la phase organique est lavée avec de la saumure, séchée avec du sulfate de sodium, filtrée et concentrée sous vide pour donner le produit attendu sous la forme d'un solide.
**RMN ¹H** (CDCl₃) δ ppm : 2,25 (s, 3 H); 3,67 (s, 3 H); 6,74 (s, 1 H)

### Stade B : 4-({4-[(tert-Butyldiméthylsilyl)oxy]phényl}amino)-1,5-diméthyl-1H-pyrrole-2-carbonitrile

Une solution du composé du stade précédent (1,5 g, 7,53 mmol), de 4-[(*tert-*butyldiméthylsilyl)oxy]aniline (2,02 g, 9,04 mmol), de *tert*-butylate de sodium (1,45 g, 15,06 mmol) et de 2-di-*tert*-butylphosphino-2',4',6'-triisopropylbiphényle (0,13 g, 0,30 mmol) dans le toluène (20 mL) est purgée à l'azote. Le tris(dibenzylideneacétone)dipalladium(0) (0,28 g, 0,30 mmol) est ajouté, puis le mélange réactionnel est chauffé à 90°C jusqu'à ce que la réaction soit complète (suivi par CCM). Le chauffage est arrêté et on laisse le mélange revenir à la température ambiante. De l'eau (75 mL) est ajoutée et le mélange est extrait avec de l'acétate d'éthyle (3 x 75 mL). Les phases organiques réunies sont lavées avec de la saumure puis concentrées. Le produit brut est purifié par chromatographie éclair sur gel de silice (gradient acétate d'éthyle/heptane). Le produit ainsi obtenu est solubilisé à chaud dans l'heptane et est laissé précipiter sous agitation à température ambiante, puis à 0°C. Le solide est filtré et l'opération est répétée sur le filtrat pour donner le composé attendu sous la forme d'un solide.
**RMN ¹H** (400 MHz, CDCl₃) δ ppm : 0,15 (s, 6 H) ; 0,97 (s, 9 H) ; 2,13 (s, 3 H) ; 3,66 (s, 3 H) ; 4,68 (large s, 1 H) ; 6,49 (d, *J*= 8,5 Hz, 2 H) ; 6,64 (s, 1H) ; 6,66 (d, *J* = 8,7 Hz, 2 H) **RMN ¹³C** (100 MHz, CDCl₃) δ ppm: 4,34 ; 9,72 ; 18,30 ; 25,88 ; 32,94 ; 101,27 ; 114,37 ; 114,70 ; 116,41 ; 120,73 ; 124,52 ; 131,23 ; 141,54 ; 148,27
**MS (ESI+):** [M+H]⁺ mesuré : 342,3

### Préparation 8": 4-[(4-{[tert-Butyl(diméthyl)silyl]oxy}phényl)amino]-1-méthyl-1H-pyrrole-2-carbonitrile

### Stade A : 1-Méthyl-1H-pyrrole-2-carbonitrile

Du *N,N-*diméthylformamide (3 mL) et du 1,4-diazabicyclo[2.2.2]octane (0,49 g, 4,3 mmol) sont ajoutés à une solution de pyrrole-2-carbonitrile (4 g, 43,4 mmol) dans le diméthylcarbonate (56 mL). La solution est agitée à 90°C pendant 15 h, puis chauffée à 110°C pendant 8 h. Le mélange est refroidi jusqu'à température ambiante, puis de l'acétate d'éthyle (80 mL) est ajouté. Les phases sont séparées et la phase organique est lavée avec de l'eau (2 x 80 mL) et une solution aqueuse 1 M d'acide chlorhydrique (1 x 80 mL). Les phases aqueuses combinées sont extraites à nouveau avec de l'acétate d'éthyle (1 x 80 mL). Les phases organiques combinées sont lavées avec de la saumure (1 x 80 mL), séchées sur sulfate de magnésium, filtrées et concentrées sous vide pour obtenir le produit attendu sous la forme d'un liquide.
**RMN ¹H** (400 MHz, CDCl₃) δ ppm : 3,78 (m, 2 H) ; 6,12-6,18 (m, 1 H) ; 6,74-6,82 (m, 1 H)

### Stade B : 4-Bromo-1-méthyl-1H-pyrrole-2-carbonitrile

Du *N*-bromosuccinimide (6,2 g, 34,9 mmol) est ajouté à une solution de 1-méthyl-1*H-*pyrrole-2-carbonitrile (3,7 g, 34,9 mmol) dans le *N,N*-diméthylformamide (150 mL). La solution est agitée pendant 15 h à température ambiante. Une nouvelle quantité de *N-*bromosuccinimide (2,0 g, 11 mmol) est ajoutée et le mélange est agité pendant 3 h. Le produit est purifié par chromatographie sur gel de silice (gradient AcOEt/heptane) pour obtenir le produit attendu sous la forme d'un solide.
**RMN ¹H** (400 MHz, CDCl₃) δ ppm : 3,77 (s, 3 H) ; 6,75 (d, *J*= 1,7 Hz, 1 H) ; 6,80 (d, *J*= 1,7 Hz, 1 H)

### Stade C : 4-[(tert-Butyldiméthylsilyl)oxy]phényl}amino)-1-méthyl-1H-pyrrole-2-carbonitrile

On laisse buller de l'azote pendant 5 minutes dans une solution de 4-bromo-1-méthyl-1*H-*pyrrole-2-carbonitrile (2,82 g, 15,2 mmol) et de 4-[(*tert*-butyldiméthylsilyl)oxy]aniline (4,08 g, 18,3 mmol) dans le toluène (55 mL). Du *tert*-butylate de sodium (2,92 g, 30,4 mmol), du tris(dibenzylideneacétone)dipalladium(0) (556 mg, 0,6 mmol) et du 2-di-*tert-*butylphosphino-2',4',6'-triisopropylbiphényle (255 mg, 0.6 mmol) sont ensuite ajoutés au mélange réactionnel. Le milieu est agité 1 h à 80°C sous azote. La suspension est ensuite refroidie jusqu'à la température ambiante et filtrée sur Célite. Le tampon de Célite est ensuite rincé avec de l'acétate d'éthyle. Le filtrat est lavé avec de l'eau puis de la saumure. La phase organique est séchée sur sulfate de magnésium, filtrée et concentrée sous vide. Le produit est purifié deux fois par chromatographie sur gel de silice (gradient AcOEt/heptane), puis ensuite par trituration dans l'heptane pour obtenir le produit attendu sous la forme d'un solide.
**RMN ¹H** (400 MHz, CDCl₃) δ ppm: 0,16 (s, 6 H) ; 0,97 (s, 9 H) ; 3,73 (s, 3 H) ; 6,57 (d, J = 1,9 Hz, 1 H) ; 6,64-6,66 (m, 1 H) ; 6,70 (s, 4 H)
**RMN ¹³C** (100 MHz, CDCl₃) δ ppm: -4,48 ; 18,17 ; 25,72 ; 35,4G ; 103,01 ; 113,56 ; 113,69 ; 115,92 ; 119,55 ; 120,67 ; 129,04 ; 139,94 ; 148,85
**MS (ESI+):** [M+H]⁺ 328,25

Les amines NHR₃R₄ dans lesquelles R₃ et R₄ représentent indépendamment l'une de l'autre un groupement aryle ou hétéroaryle sont obtenues selon les procédés décrits dans la littérature (Surry D.S et al., Chemical Science, 2011, 2, 27-50, Charles M.D. et al., Organic Letters, 2005, 7, 3965-3968). La réaction de protection de la fonction hydroxy du 4-anilinophénol décrite à la Préparation 1 " peut être appliquée à diverses amines secondaires NHR₃R₄ (telles que définies précédemment), comportant une ou plusieurs fonctions hydroxy, lorsque celles-ci sont disponibles commercialement. Alternativement, les amines secondaires comportant au moins un substituant hydroxy peuvent être synthétisées directement sous une forme protégée, i.e. à partir de réactifs dont la fonction hydroxy a été préalablement protégée. Parmi les groupements protecteurs, le *tert*-butyl(diméthyl)silyloxy et le benzyloxy sont particulièrement préférés.

Parmi les amines NHR₃R₄ comportant un substituant hydroxy qui sont utilisées pour synthétiser les composés de l'invention, on peut citer : le 4-(4-toluidino)phénol, le 4-(4-chloroanilino)phénol, le 4-(3-fluoro-4-méthylanilino)phénol, le 4-[4-(trifluorométhoxy)anilino]phénol, le 4-(4-hydroxyanilino]phénol, le {4-[(1-méthyl-1*H-*indol-6-yl)amino]phényl}méthanol, le 4-(2,3-dihydro-1*H*-indol-6-ylamino)phénol, le 4-[(1-méthyl-2,3-dihydro-1*H*-indol-6-yl)amino]phénol, 4-[(1-méthyl-1*H*-indol-6-yl}amino]phénol, le 4-[(1-méthyl-1*H*-indol-6-yl)amino]cyclohexanol, le 4-[(1-méthyl-1,2,3,4-tétrahydro-6-quinolinyl)amino]phénol, le 4-[(4-méthyl-3,4-dihydro-2*H*-1,4-benzoxazin-7-yl)amino]phénol, le 4-[4-(diéthylamino)anilino]phénol, le 4-(2,3-dihydro-1*H*-indén-5-ylamino)phénol, le 4-[(1-méthyl-1*H*-indazol-5-yl)amino]phénol, le 4-[(1'-méthyl-1',2'-dihydrospiro[cyclopropane-1,3'-indol]-5'-yl)amino]phénol, le 4-[(1,3,3-triméthyl-2,3-dihydro-1*H*-indol-5-yl) amino] phénol, le 4-[4-méthoxy-3-(trifluorométhyl)anilino]phénol, le 4-[4-(méthylsulfanyl)-3-(trifluorométhyl)anilino]phénol, le 2-fluoro-4-[(1-méthyl-1H-indol-5-yl)amino]phénol, le 4-[{1-éthyl-1*H-*indol-5-yl)amino]phénol, le 4-[(1-éthyl-2,3-dihydro-1*H*-indol-5-yl)amino]phénol, le 4-[(1-isopropyl-2,3-dihydro-1*H-*indol-5-yl)amino]phénol, le 4-(butylamino)phénol, le 3-[(1-méthyl-1*H*-indol-5-yl)amino]-1-propanol, le 4-[(1-methyl-1*H*-indol-5-yl)amino]-1-butanol, le 4-[(3-fluoro-4-méthylphényl)amino]phénol, 4-[(3-chloro-4-méthylphényl)amino]phénol, 4-[(4-fluorophényl)amino]phénol, 4-[(1-méthyl-1*H-*pyrrolo[2_{,}3-*b*]pyridin-5-yl)amino]phénol, 4-[(4-fluorophényl)amino]phénol, 4-[(2-fluorophényl)amino]phénol, 4-[(3-fluorophényl)amino]phénol, 4-[(2,4-difluorophényl) amino]phénol, 4-[(3,4-difluorophényl)amino]phénol, 3-[(4-hydroxyphényl)amino] benzonitrile, 4-[(3-méthoxyphényl)amino]phénol, 4-[(3,5-difluorophényl)amino]phénol, 4-[(3-méthylphényl)amino]phénol, 4-[(4-hydroxyphényl)amino]benzonitrile, 4-[(3-chlorophényl)amino]phénol, 4-(pyrimidin-2-ylamino)phénol, 4-[(cyclobutylméthyl) amino]phénol, 2-[(4-hydroxyphényl)amino]benzonitrile, 4-{[{1-méthyl-1*H-*pyrazol-4-yl)méthyl]amino}phénol, 4-[(cyclopropylméthyl)amino]phénol, 4-{[(1-méthyl-1*H-*pyrazol-3-yl)méthyl]amino}phénol, 4-(but-2-yn-1-ylamino)phénol, 4-(pyrazin-2-ylamino) phénol, 4-(pyridin-2-ylamino)phénol, 4-(pyridazin-3-ylamino)phénol, 4-(pyrimidin-5-ylamino)phénol, 4-(pyridin-3-ylamino)phénol, 4-[(3,5-difluoro-4-méthoxyphényl) amino]phénol, 4-(pyridin-4-ylamino)phenol, 4-[(3-fluoro-4-méthoxyphényl)amino] phénol, 2-(phénylamino)pyrimidin-5-ol, 5-[(4-hydroxyphényl)amino]-2-méthoxy benzonitrile, 4-{[3-(trifluorométhyl)phényl]amino}phénol.

La ou les fonction(s) hydroxy des amines secondaires listées ci-dessus est (sont) préalablement protégée(s) par un groupement protecteur adapté avant tout couplage à un dérivé d'acide du composé de formule (VII) tel que défini dans le procédé général précédent.

### Exemple 1. 4-[{[3-(6-{[(3S)-3-(Morpholin-4-ylméthyl)-3,4-dihydroisoquinolin-2(1H)-yl]carbonyl}-1,3-benzodioxol-5-yl)-5,6,7,8-tétrahydroindolizin-1-yl)carbonyl}(phényl) amino]phényl phosphate de disodium

### Stade A : 3-{6-[((3S)-3-(4-Morpholinylméthyl)-3,4-dihydro-2(1H)-isoquinolinyl) carbonyl]-1,3-benzodioxol-5-yl}-5,6,7,8-tétrahydro-1-indolizine carboxylate de méthyle,

A une solution de 2 g du composé de la Préparation 1 (5,83 mmol) dans 20 mL de dichlorométhane, on ajoute à température ambiante 5,5 mL de *N,N,N*-triéthylamine (6,96 mmol), 2,12 g du composé de la Préparation 1' (6,96 mmol), puis 0,94 g d'hydroxybenzotriazole (HOBT) et 1,34 g d'l-éthyl-3-(3'-diméthylaminopropyl)-carbodiimide (EDC) (6,96 mmol). Le milieu réactionnel est ensuite agité à température ambiante pendant 1 nuit, puis il est versé sur une solution aqueuse saturée en chlorure d'ammonium et extrait avec de l'acétate d'éthyle. La phase organique est ensuite séchée sur sulfate de magnésium, puis filtrée et évaporée à sec. Le produit brut ainsi obtenu est alors purifié par chromatographie sur gel de silice (gradient heptane/ AcOEt). Le produit du titre est obtenu sous la forme d'une huile.
**RMN¹H:** δ (500 MHz ; dmso-d6 ; 300°K) : 7,2-6,9 (m, 4H, H aromatiques) ; 7,04-7,03-7,00 (m, 1H, H aromatique); 6,85 (m, 1H, H aromatique); 6,35-6,26-6,06 (m, 1H, H tétrahydroindolizine) ; 6,15-6,12 (m, 2H, H méthylènedioxy); 5,06-4,84 (m, 1H, H dihydroisoquinoline) ; 4,86-4,17 (m, 2H, H dihydroisoquinoline) ; 3,65-3,6-3,55 (m, 3H, H méthyl ester) ; 3,43-4,26 (m, 2H, H tétrahydroindolizine) ; 3,58-3,5 (m, 4H, H morpholine) ; 2,37-3,05 (m, 4H, 2H dihydroisoquinoline, 2H tétrahydroindolizine) ; 1,68-2,56 (m, 4H, H morpholine) ; 1,4-2,0 (m, 4H, H tétrahydroindolizine)
**IR :** *ν* : >C=O 1695 cm⁻¹ ester ; *ν* : >C=O 1625 cm⁻¹ amide ; *ν* : >C-O-C< 1214-1176-1115 cm⁻¹ ; >CH-Ar 772-744 cm⁻¹

### Stade B: 3-[6-[(3S)-3-(Morpholinométhyl)-3,4-dihydro-1H-isoquinoline-2-carbonyl]-1,3-benzodioxol-5-yl]-5,6,7,8-tétrahydro-1-indolizine carboxylate de lithium

A une solution de 4,6 g du composé du Stade A (8,26 mmol) dans 24 mL de dioxane est ajoutée une solution d'hydroxyde de lithium (675 mg, 16,1 mmol). L'ensemble est placé dans un four à micro-ondes à 140W, 100°C pour une durée de 2h30. Le milieu réactionnel est ensuite filtré et évaporé. Le solide ainsi obtenu est séché à 40°C dans une étuve en présence de P₂O₅.
**RMN¹H :** δ (400 MHz ; dmso-d6 ; 353°K) : 6,7-7,15 (massif, 6H, H aromatiques); 6,21 (s, 1H, H aromatique) ; 6,03 (s, 2H, H méthylènedioxy); 4,0-5,0 (massif, 3H dihydroisoquinoline); 3,4-3,6 (massif, 3H tétrahydroindolizine, 3H morpholine); 2,5-3,1 (massif, 4H, 2H tétrahydroindolizine, 2H morpholine) ; 1,5-2,4 (massif, 10H morpholine) **IR :** *ν* :>C=O 1567 large cm⁻¹ acétate ; *ν* : 1236 cm⁻¹

### Stade C: N-(4-{[tert-Butyl(diméthyl)silyl]oxy}phényl)-3-{6-[((3S)-3-(4-morpholinyl méthyl)-3,4-dihydro-2(1H)-isoquinolinyl)carbonyl]-1,3-benzodioxol-5-yl}-N-phényl-5,6,7,8-tétrahydro-1-indolizine carboxamide

A une solution de 2,6 g du composé du Stade B (4,73 mmol) dans 47 mL de dichlorométhane sont ajoutés, au goutte à goutte, 1,2 mL de chlorure d'oxalyle (14,2 mmol) à 0°C. Le milieu réactionnel est agité à température ambiante pendant 11 heures, puis co-évaporé plusieurs fois avec du dichlorométhane. Le produit ainsi obtenu est mis en suspension dans 37 mL de dichlorométhane, puis est additionné sur une solution de 2,1 g du composé obtenu à la Préparation 1" (7,1 mmol) dans 10 mL de dichlorométhane en présence de 0,6 mL de pyridine (7,1 mmol). L'ensemble est agité à température ambiante pendant une nuit.

Le milieu réactionnel est concentré, purifié par chromatographie sur gel de silice (gradient dichlorométhane/méthanol). Le produit du titre est obtenu sous forme d'une mousse.
**RMN¹H:** δ (500MHz ; dmso-d6 ; 300°K) : 6,9-7,3 (9H aromatiques); 6,88 (2H aromatiques) ; 6,72-6,87 (2H aromatiques); 6,64 (2H aromatiques); 6,13 (2H méthylènedioxy) ; 5,05-4,74 (1H dihydroisoquinoline) ; 4,25-4,13 (2H dihydroisoquinoline) ; 3,44-3,7 (4H morpholine) ; 3,62-3,52 (2H tétrahydroindolizine) ; 3,0-2,6 (4H, 2H tétrahydroindolizine, 2H dihydroisoquinoline) ; 2,54-1,94 (6H morpholine) ; 1,91-1,53 (4H tétrahydroindolizine) ; 0,92 (9H *tert*-butyl) ; 0,17 (6H diméthyl)
**IR :** *ν* :>C=O : 1632 cm⁻¹ ; *ν* : >C-O-C< : 1237 cm⁻¹; *ν* : -Si-O-C- : 1035 cm⁻¹, -Si-C- : 910 cm⁻¹ ; >CH-Ar : 806 cm⁻¹

### Stade D: Chlorhydrate de N-(4-hydroxyphényl)-3-{6-[((3S)-3-(4-morpholinylméthyl)-3,4-dihydro-2(1H)-isoquinolinyl)carbonyl]-1,3-benzodioxol-5-yl}-N-phényl-5,6,7,8-tétrahydro-1-indolizine carboxamide

A une solution de 1,9 g du composé obtenu au Stade C (2,3 mmol) dans 4 mL de méthanol, on ajoute 0,646 g (11,5 mmol) d'hydroxyde de potassium solubilisé dans 8 mL de méthanol. L'ensemble est agité à température ambiante pendant 30 min. Le milieu réactionnel est ensuite dilué dans le dichlorométhane et lavé successivement avec une solution HCl 1M, une solution aqueuse saturée en NaHCO₃ saturée, puis de la saumure jusqu'à atteindre un pH neutre. La phase organique est ensuite séchée sur sulfate de magnésium, filtrée et évaporée. Le produit brut ainsi obtenu est purifié par chromatographie sur gel de silice (gradient dichlorométhane/méthanol). Le solide est ensuite solubilisé dans le dichlorométhane et 2 mL d'éther chlorhydrique 1M sont ajoutés. Le tout est agité pendant 1 heure, puis évaporé à sec. Le chlorhydrate ainsi obtenu est dissout dans un mélange eau / acétonitrile jusqu'à solubilisation totale, puis est lyophilisé.
***Microanalyse élémentaire : (% théorique : mesuré)*** %C=69,11:68,95; %H=5,8:5,46; %N=7,5:7,51; %Cl-=4,74:4,48
***Pouvoir rotatoire :*** (α) _{D}²⁰= + 50,8° (c = 9 mg/mL, MeOH)

### Stade E: 4-[{[3-(6-{[(3S)-3-(Morpholin-4-ylméthyl)-3,4-dihydroisoquinolin-2(1H)-yl]carbonyl}-1,3-benzodioxol-5-yl)-5,6,7,8-tétrahydilizin-1-yl]carbonyl}(phényl) amino]phényl phosphate de dibenzyle

A une suspension de 82 mg d'hydrure de sodium (2,06 mmol) dans 10 mL de THF anhydre est ajouté par portions et à 0°C 700 mg du composé du Stade D. Après 30 minutes d'agitation à 0°C et 30 min à température ambiante, le pyrophosphate de tétrabenzyle est ajouté à 0°C et le milieu réactionnel est agité une nuit à température ambiante. Après évaporation du solvant, le brut réactionnel est dilué au dichlorométhane (30 mL), lavé par une solution aqueuse saturée de NaHCO₃, puis de la saumure. La phase organique est alors séchée sur MgSO₄, filtrée, concentrée à sec, et purifiée par chromatographie sur gel de silice (gradient CH₂Cl₂/MeOH). On obtient alors le produit du titre sous la forme d'un solide.
**RMN ¹H** : δ (500 MHz ; DMSO-d6 ; 300K) : 7,34 (m, 10H, phényl) ; 7,30-6,71 (m, 15H, aryl); 6,06 (s, 1H, méthylenedioxy) ; 5,30-4,97 (m, 1H, pyrrole); 5,11 (m, 4H, benzyle) : 5,03-3,64 (m, 1H, Ctertiaire THIQ) ; 4,91-4,09 (m, 2H, Csecondaire THIQ) ; 3,99-3,48 (m, 2H, Csecondaire THIQ) ; 3,54-3,44 (m, 4H, morpholine) ; 2,89-2,65 (m, 3H, Csecondaire THIQ) ; 2,51-1,87 (m, 4H, Csecondaire THID) ; 2,36-1,85 (m, 2H, Csecondaire THIQ) ; 1,91-1,45 (m, 4H, CsecondaireTHID)

### Stade F: 4-[{[3-(6-{[(3S)-3-(Morpholin-4-ylméthyl)-3,4-dihydroisoquinolin-2(1H)-yl]carbonyl}-1,3-benzodioxol-5-yl)-5,6,7,8-tétrahydroindolizin-1-yl]carbonyl}(phényl) amino]phényl phosphate de disodium

A une solution du produit obtenu au Stade A (505 mg ; 0.52 mmol) dans du méthanol (10 mL) est ajouté 50 mg de Pd(OH)₂, puis le milieu réactionnel est placé sous atmosphère d'hydrogène (1 bar) pendant 5h. Apres filtration du catalyseur et concentration à sec, le brut réactionnel est solubilisé dans le méthanol (5 mL) et traité par 0.95 mL de soude 1M. Les solvants sont alors évaporés et le brut réactionnel est purifié par chromatographie sur phase OASIS® (gradient Acétonitrile/H₂O pour obtenir un solide blanc.

**Microanalyse élémentaire :**

| | %*C* | *%H* | *%N* | %*Na* |
|---|---|---|---|---|
| *Calculé* | *61, 87* | *4*,*95* | *6,71* | *5,51* |
| *Trouvé* | *61,45* | *4,46* | *6,61* | *5,38* |

**IR** : *ν* : -C=O: 1628 cm⁻¹ ; *ν* : C-O-C : 1234 cm⁻¹ ; *ν* : P=O : 115 cm⁻¹ ; *ν* : P-O : 985 cm⁻¹;
*ν* : CH-Ar : 876 cm⁻¹

### Masse haute résolution (ESI+) :

Formule brute : C₄₃ H₄₁ N₄ Na₂ O₉ P
[M+H]+ calculé : 835,2479
[M+H]+ mesuré : 835,2467

### Exemple 2. 4-[{[3-(6-{[(3R)-3-Méthyl-3,4-dihydroisoquinolin-2(1H)-yl]carbonyl}-1,3-benzodioxol-5-yl)-5,6,7,8-tétrahydroindolizin-1-yl]carbonyl}(phényl)amino] phényl phosphate de disodium

### Stade A : N-(4-Hydroxyphényl)-3-(6-{[(3R)-3-méthyl-3,4-dihydroisoquinolin-2(1H)-yl]carbonyl}-1,3-benzodioxol-5-yl)-N-phényl-5,6,7,8-tétrahydroindolizine-1-carboxamide

On procède selon un protocole analogue à celui décrit aux Stades A-D de l'Exemple 1 en remplaçant au Stade A le produit de la Préparation 1' par celui de la Préparation 2', étant entendu que le produit ainsi obtenu n'est pas soumis à une étape de salification en présence d'éther chlorhydrique.
***Microanalyse élémentaire : (% théorique : mesuré)***
%C=74,86:74,88; %H=5,64:5,31; %N=6,72:6,78

### Stade B : 4-[{[3-(6-{[(3R)-3-Méthyl-3,4-dihydroisoquinolin-2(1H)-yl]carbonyl}-1,3-benzodioxol-5-yl)-5,6,7,8-tétrahydroindolizin-1-yl]carbonyl}(phényl)amino]phényl phosphate de disodium

On procède selon un protocole analogue à celui décrit aux Stades E et F de l'Exemple 1.

### Masse haute résolution (ESI+) :

Formule brute : C₃₉H₃₆N₃O₈P
[M+H]⁺ calculé : 706,2313
[M+H]+ mesuré : 706,2324

### Exemple 3. 4-[(1-Méthyl-1H-indol-5-yl){[3-(2-{[(3S)-3-(morpholin-4-ylméthyl)-3,4-dihydroisoquinolin-2(1H)-yl]carbonyl}phényl)-5,6,7,8-tétrahydroindolizin-1-yl] carbonyl}amino]phényl phosphate de disodium

### Stade A : Chlorhydrate de 3-{5'-chloro-2-[((3S)-3-(4-morpholinylméthyl)-3,4-dihydro-2(1H)-isoquinolinyl)carboyl]phényl}-N-(4-hydroxyphényl)-N-(1-méthyl-1H-indol-5-yl)-5,6,7,8-tétrahydro-1-indolizine carboxamide

On procède selon un protocole analogue à celui décrit aux Stades A-D de l'Exemple 1 en remplaçant d'une part le composé de la Préparation 1 utilisé au Stade A par le composé de la Préparation 2, et d'autre part le composé de la Préparation 1" utilisé au Stade C par celui de la Préparation 2".

**Microanalyse élémentaire :**

| | %C | %*H* | *%N* | *%Cl⁻* |
|---|---|---|---|---|
| *Calculé* | *68,04* | *5,72* | *8,82* | *4,91* |
| *Trouvé* | *67,84* | *5,46* | *8,64* | *5*,*21* |

***Pouvoir rotatoire :*** (α) _{D}²⁰ = + 55,9° (c = 7 mg/mL, MeOH)

### Stade B : 4-[(1-Méthyl-1H-indol-5-yl){[3-(2-{[(3S)-3-(morpholin-4-ylméthyl)-3,4-dihydroisoquinolin-2(1H)yl]carbonyl}phényl)-5,6,7,8-tétrahydroindolizin-1-yl] carbonyl} amino]phényl phosphate de disodium

On procède selon un protocole analogue à celui décrit aux Stades E et F de l'Exemple 1.

### Masse haute résolution (ESI+) :

Formule brute : C₄₅H₄₄N₅Na₂O₇P
[M-2Na+3H]⁺ calculé : 800,3208
[M-2Na+3H]+ mesuré: 800,3211

### Exemple 4. 4-[{[3-(6-{[(3R)-3-Méthyl-3,4-dihydroisoquinolin-2(1H)-yl]carbonyl}-1,3-benzodioxol-5-yl)indolizin-1-yl]carbonyl}(1-méthyl-1H-pyrrolo[2,3-b]pyridin-5-yl)amino]phényl phosphate de disodium

### Stade A : Chlorhydrate de N-(4-hydlroxyphényl)-3-(6-{[(3R)-3-méthyl-3,4-dihydroisoquinolin-2(1H)-yl]carbonyl}-1,3-benzodioxol-5-yl)-N-(1-méthyl-1H-pyrrolo[2,3-b]pyridin-5-yl)indolizine-1-carboxamide

On procède selon un protocole analogue à celui décrit aux Stades A-D de l'Exemple 1 en remplaçant d'une part les composés des Préparation 1 et 1' utilisés au Stade A par les composés des Préparations 3 et 2', et d'autre part le composé de la Préparation 1" utilisé au Stade C par celui de la Préparation 3".

### Microanalyse élémentaire : (% théorique : mesuré)

### %C=69,14:70,09; %H=4,81:4,55; %N=9,83:10,09; %Cl-=4,98:3,26

### Stade B : 4-[{[3-(6-{[(3R)-3-Méthyl-3,4-dihydroisoquinolin-2(1H)-yl]carbonyl}-1,3-benzodioxol-5-yl)indolizin-1-yl]carbonyl}(1-méthyl-1H-pyrrolo[2,3-b]pyridin-5-yl)amino]phényl phosphate de diéthyle

A une suspension du composé obtenu au Stade A (1,5 mmol) dans 10 mL de CH₂Cl₂ anhydre est ajouté de la triéthylamine (0,42 mL ; 3 mmol), puis le diéthylcyanophosphonate (0,24 mL; 1,65 mmol) au goutte à goutte à température ambiante. Après une nuit d'agitation à température ambiante, le milieu réactionnel est dilué avec du CH₂Cl₂, lavé par une solution aqueuse saturée en NaHCO₃, puis de la saumure. La phase organique est alors séchée sur MgSO₄, filtrée, concentrée à sec, et purifiée par chromatographie sur gel de silice (gradient CH₂Cl₂/MeOH). On obtient alors le produit du titre sous la forme d'un solide.

### Stade C: 4-[{[3-(6-{[(3R)-3-Méthyl-3,4-dihydroisoquinolin-2(1H)-yl]carbonyl}-1,3-benzodioxol-5-yl)indolizin-1-yl]carbonyl}(1-méthyl-1H-pyrrolo[2,3-b]pyridin-5-yl)amino]phényl phosphate de disodium

A une solution du produit obtenu au Stade B (0,78 mmol) dans le CH₂Cl₂ (12 mL) est ajouté 0,4 mL de bromure de triméthylsilyle (3 mmol) au goutte à goutte à température ambiante. Le milieu réactionnel est agité pendant 5 h, puis une solution de Na₂CO₃ (580 mg) dans l'eau (4 mL) est lentement ajoutée à 0°C. Apres 30 min d'agitation, le milieu réactionnel est concentré à sec, dilué au méthanol anhydre (25 mL), et µ-filtré. Le filtrat est mis à sec et purifié par chromatographie sur phase OASIS® (gradient acétonitrile/H₂O).

### Masse haute résolution (ESI+) :

Formule brute : C₄₅H₄₄N₅Na₂O₇P
[M-2Na+3H]⁺ calculé : 800,3211
[M-2Na+3H]+ mesuré : 800,3201

### Exemple 5. 4-[{[3-(6-{[(3R)-3-Méthyl-3,4-dihydroisoquinolin-2(1H)-yl]carbonyl}-1,3-benzodioxol-5-yl)indolizin-1-yl]carbonyl}(pyridin-4-yl)amino]phényl phosphate de disodium

### Stade A : Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(pyridin-4-yl)indolizine-1-carboxamide

On procède selon un protocole analogue à celui décrit aux Stades A-D de l'Exemple 1 en remplaçant d'une part les composés des Préparation 1 et 1' utilisés au Stade A par les composés des Préparations 3 et 2', et d'autre part le composé de la Préparation 1" utilisé au Stade C par celui de la Préparation 4".
***Microanalyse élémentaire : (% théorique : mesuré)***
%C=69,24:69,12; %H=4,74:4,23; %N=8,5:8,45; %Cl-=5,38:5,2

### Stade B : 4-[{[3-(6-{[(3R)-3-Méthyl-3,4-dihydroisoquinolin-2(1H)-yl]carbonyl}-1,3-benzodioxol-5-il)indolizin-1-yl]carbonyl}(pyridin-4-yl)amino]phényl phosphate de diéthyle

A une suspension de 950 mg du composé obtenu au Stade A (1,5 mmol) dans 10 mL de CH₂Cl₂ anhydre est ajouté de la triéthylamine (0,42 mL ; 3 mmol), puis le diéthylcyanophosphonate (0,24 mL; 1,65 mmol) au goutte à goutte à température ambiante. Après une nuit d'agitation à température ambiante, le milieu réactionnel est dilué avec du CH₂Cl₂, lavé par une solution aqueuse saturée en NaHCO₃, puis de la saumure. La phase organique est alors séchée sur MgSO₄, filtrée, concentrée à sec, et purifiée par chromatographie sur gel de silice (gradient CH₂Cl_{2/}MeOH). On obtient alors le produit du titre sous la forme d'un solide.
**RMN ¹H :** δ (500 MHz ; DMSO-d6 ; 300K) : 8,5-8, 0 (m, 5H); 7,2-7,1 (m, 1H) ; 6,85-6,65 (m, 1H); 7,3-6,8 (m, 10H); 6,25-6,10 (s1, 1H) ; 6,2 (s1, 2H); 5,1-3,7 (6d, 2H); 4,7-3,8 (m,1H); 4,15 (m, 4H); 3,0-1,7 (m,2H); 1,25 (m, 6H); 0,85-0,24 (m,3H)

### Stade C : 4-[{[3-(6-{[(3R)-3-Méthyl-3,4-dihydroisoquinolin-2(1H)-yl]carbonyl}-1,3-benzodioxol-5-yl)indolizin-1-yl]carbonyl}(pyridin-4-yl)amino]phényl phosphate de disodium

A une solution du produit obtenu au Stade B (591 mg ; 0,78 mmol) dans le CH₂Cl₂ (12 mL) est ajouté 0,4 mL de bromure de triméthylsilyle (3 mmol) au goutte à goutte à température ambiante. Le milieu réactionnel est agité pendant 5 h, puis une solution de Na₂CO₃ (580 mg) dans l'eau (4 mL) est lentement ajoutée à 0°C. Apres 30 min d'agitation, le milieu réactionnel est concentré à sec, dilué au méthanol anhydre (25 mL), et µ-filtré. Le filtrat est mis à sec et purifié par chromatographie sur phase OASIS® (gradient acétonitrile/H₂O).
RMN ¹H : δ (500 MHz ; D₂O ; 300K) : 8,23-7,98 (m, 2H, pyridyl) ; 7,01-6,97 (m, 2H, pyridyl); 7,88-7,80 (m, 1H, indolizine) ; 7,18-6,57 (m, 13H, aromatiques THIQ+aryl+indolizine+phenol); 6,17-6,15 (m, 1H, indolizine): 5,96 (m, 2H, méthylenedioxy) ; 4,61-3,76 (m, 1H, C ternieire THIQ) ; 4,16 (m, 2H, Csecondaire THIQ) ; 2,86-2,31 (m, 2H, Csecondaire THIQ) ; 0,94-0,76 (m, 3H, Cprimaire THIQ)
**IR :** ν : -C=O: 1620 cm⁻¹ ; v : C-O-C : 1218 cm⁻¹ ; v : P=O : 1107 cm⁻¹ v : P-O : 981 cm⁻¹ v : CH-Ar : 881-741 cm⁻1

### Masse haute résolution (ESI+) :

Formule brute : C₃₈H₂₉N₄Na₂O₈P
[M-2Na+3H]⁺ calculé : 703,1952
[M-2Na+3H]+mesuré : 703,1951

### Exemple 6. 4-[{[3-(6-{[(3S)-3-(Morpholin-4-ylméthyl)-3,4-dihydroisoquinolin-2(1H)-yl]carbonyl}-1,3-benzodioxol-5-yl)-5,6,7,8-tétrahydroindolizin-1-yl]carbonyl}(phényl) amino]phényl phosphate de dibenzyle

On procède selon le protocole décrit aux Stades A-E de l'Exemple 1.
**RMN ¹H :** δ (500 MHz ; DMSO-d6 ; 300K) : 7,34 (m, 10H, phényl) ; 7,30-6,71 (m, 15H, aryl); 6,06 (s, 1H, méthylenedioxy) ; 5,30-4,97 (m, 1H, pyrrole); 5,11 (m, 4H, benzyle) ; 5,03-3,64 (m, 1H, Ctertiaire THIQ) ; 4,91-4,09 (m, 2H, Csecondaire THIQ) ; 3,99-3,48 (m, 2H, Csecondaire THIQ) ; 3,54-3,44 (m, 4H, morpholine) ; 2,89-2,65 (m, 3H, Csecondaire THIQ) ; 2,51-1,87 (m, 4H, Csecondaire THID) ; 2,36-1,85 (m, 2H, Csecondaire THIQ) ; 1,91-1,45 (m, 4H, CsecondaireTHID)

### Exemple 7. 4-[{[3-(6-{[(3R)-3-Méthyl-3,4-dihydroisoquinolin-2(1H)-yl]carbonyl}-1,3-benzodioxol-5-yl)indolizin-1-yl]carbonyl}(pyridin-4-yl)amino]phényl phosphate de diéthyle

On procède selon le protocole décrit aux Stades A et B de l'Exemple 5.
RMN **¹H :** δ (500 MHz ; DMSO-d6 ; 300K) : 8,5-8,0 (m, 5H); 7,2-7,1 (m, 1H); 6,85-6,65 (m, 1H); 7,3-6,8 (m, 10H); 6,25-6,10 (sl, 1H); 6,2 (sl, 2H); 5,1-3,7 (6d, 2H); 4,7-3,8 (m, 1H); 4,15 (m, 4H); 3,0-1,7 (m,2H); 1,25 (m, 6H); 0,85-0,24 (m,3H)

### Exemple 8. Chlorhydrate de 4-[{[3-(6-{[(3S)-3-(morpholin-4-ylméthyl)-3,4-dihydroisoquinolin-2(1H)-yl]carbonyl}-1,3-benzodioxol-5-yl)-5,6,7,8-tétrahydroindolizin-1-yl]carbonyl}(phényl)amino]phényl dihydrogène phosphate

A une solution du produit obtenu au Stade E de l'Exemple 1 (500 mg ; 0,51 mmol) dans du méthanol (10 mL) sont ajoutés 100 mg de Pd(OH)₂, puis le milieu réactionnel est placé sous atmosphère d'hydrogène (1 bar) pendant 5 h. Apres filtration du catalyseur et concentration à sec, le brut réactionnel est immédiatement purifié par chromatographie sur phase C18 (gradient acétonitrile/H₂O +0,2% HCl) pour obtenir un solide.

### Masse haute résolution (ESI+) :

Formule brute : C₄₃H₄₃N₄O₉P
[M+H]⁺ calculé: 791,2846
[M+H]⁺ mesuré : 791,2852

### Exemple 9. 4-[{[5-(5-Chloro-2-{[(3R)-3-méthyl-3,4-dihydroisoquinolin-2(1H)-yl]carbonyl}phényl)-1,2-diméthyl-1H-pyrrol-3-yl]carbonyl}(pyridin-4-yl)amino]phényl phosphate de disodium

### Stade A : Chlorhydrate de 5-(5-chloro-2-{[(3R)-3-méthyl-3,4-dihydroisoquinolin-2(1H)-yl]carbonyl}phényl)-N-(4-hydroxyphényl)-1,2-diméthyl-N-(pyridin-4-yl)-1H-pyrrole-3-carboxamide

On procède selon un protocole analogue à celui décrit aux Stades A-D de l'Exemple 1 en remplaçant d'une part les composés des Préparation 1 et l'utilisés au Stade A par les composés des Préparations 4 et 2', et d'autre part le composé de la Préparation 1" utilisé au Stade C par celui de la Préparation 4",
***Microanalyse élémentaire :* (% *théorique : mesuré)***
%C=66,99:66,88; %H=5,14:5,28; %N=8,93:8,87; %Cl-=5,65:4,98

### Masse haute résolution (ESI+) :

Formule brute : C₃₅H₃₂ClN₄O₃
[M+H]⁺ calculé : 591,2157
[M+H]⁺ mesuré : 591,2178

### Stade B : 4-({[5-(5-Chloro-2-{[(3R)-3-méthyl-3,4-dihydroisoquinolin-2(1H)-yl]carbonyl}phényl)-1,2-diméthyl-1H-pyrrol-3-yl]carbonyl}(pyridin-4-yl)amino]phényl phosphate de disodium

On procède selon un protocole analogue à celui décrit aux Stades B et C de l'Exemple 4.

### Exemple 10. 4-[{[1,2-Diméthyl-5-(6-{[{3S)-3-(morpholin-4-ylméthyl)-3,4-dihydroisoquinolin-2(1H)-yl]carbonyl}-1,3-benzodioxol-5-yl)-1H-pyrrol-3-yl]carbonyl}(1-méthyl-1H-pyrrolo[2,3-b]pyridin-5-yl)amino]phényl phosphate de disodium

### Stade A : Chlorhydrate de N-(4-hydroxyphényl)-1,2-diméthyl-N-(1-méthyl-1H-pyrrolo[2,3-b]pyridin-5-yl)-5-(6-{[3S)-3-(morpholin-4-ylméthyl)-3,4-dihydroisoquinolin-2(1H)-yl]carbonyl}-1,3-benzodioxol-5-yl}-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 5, et d'autre part le composé de la Préparation 1" utilisé au Stade C par celui de la Préparation 3".
***Microanalyse élémentaire : (%mesuré (théorique))***
%C=66,41(66,62); %H=5,08(5,59); %N=10,85(10,84); %Cl-=4,68(4,57)

### Stade B : 4-[{[1,2-Diméthyl-5-(6-{[(3S)-3-(morpholin-4-ylméthyl)-3,4-dihydroisoquinolin-2(1H)-yl]carbonyl}-1,3-benzodioxol-5-yl)-1H-pyrrol-3-yl]carbonyl}(1-méthyl-1H-pyrrolo[2,3-b]pyridin-5-yl)amino]phényl phosphate de disodium

On procède selon un protocole analogue à celui décrit aux Stades B et C de l'Exemple 4.

### Exemple 11. 4-[{[1,2-Diméthyl-5-(6-{[(3S)-3-(morpholin-4-ylméthyl)-3,4-dihydroisoquinolin-2(1H)-yl]carbonyl}-1,3-benzodioxol-5-yl)-1H-pyrrol-3-yl]carbonyl}(1-méthyl-1H-pyrazol-4-yl)amino]phényl phosphate de disodium

### Stade A : Chlorhydrate de N-(4-hydroxyphényl)-1,2-diméthyl-N-(1-méthyl-1H-pyrazol-4-yl)-5-(6-{[(3S)-3-(morpholin-4 ylméthyl)-3,4-dihydroisoquinolin-2(1H)-yl]carbonyl}-1,3-benzodioxol-5-yl)-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 5, et d'autre part le composé de la Préparation 1" utilisé au Stade C par celui de la Préparation 5".
***Microanalyse élémentaire : (%mesuré (théorique))***
%C=64,25(64,59); %H=5,4(5,7); %N=11,41(11,59); %Cl-=4,93(4,89)

### Stade B : 4-[{[1,2-Diméthyl-5-(6-{[(3S)-3-(morpholin-4-ylméthyl)-3,4-dihydroisoquinolin-2(1H)-yl]carbonyl}-1,3-benzodioxol-5-yl)-1H-pyrrol-3-yl]carbonyl} (1-méthyl-1H-pyrazol-4-yl)amino]phényl phosphate de disodium

On procède selon un protocole analogue à celui décrit aux Stades E et F de l'Exemple 1.
**IR (cm⁻¹):** v : C=O : 1628; v : (phosphate; éther):1238, 1143,1113, 985; γ : >CH Ar :740

**Microanalyse élémentaire :**

| | %*C* | *%H* | *%N* |
|---|---|---|---|
| *Calculé* | *57, 64* | *4,84* | *10,34* |
| *Trouvé* | *56,62* | *4,54* | *10,14* |

### Masse haute résolution (ESI+-/FIA/HR] :

Formule brute : C₃₉H₃₉ClN₆Na₂O₉P
[M-Na+H]⁺ calculé : 791,2565
[M-Na+H]⁺ mesuré : 791,2564

### Exemple 12. 4-[{[1,2-Diméthyl-5-(6-{[(3R)-3-[3-(morpholin-4-yl)propyl]-3,4-dihydroisoquinolin-2(1H)-yl]carbonyl}-1,3-benzodioxol-5-yl)-1H-pyrrol-3-yl]carbonyl}(1-méthyl-1H-pyrrolo[2,3-b]pyridin-5-yl)amino]phényl phosphate de disodium

### Stade A : Chlorhydrate de N-(4-hydroxyphényl)-1,2-diméthyl-N-(1-méthyl-1H-pyrrolo[2,3-b]pyridin-5-yl)-5-(6{[(3R)-3-[3-(morpholin-4-yl)propyl]-3,4-dihydroisoquinolin-2(1H)-yl]carbonyl}-1,3-benzodioxol-5-yl)-1H-pyrrole-3-carboxamide

On procède selon un protocole analogue à celui décrit aux Stades A-D de l'Exemple 1 en remplaçant d'une part les composés des Préparation 1 et 1' utilisés au Stade A par les composés des Préparations 5 et 3', et d'autre part le composé de la Préparation 1" utilisé au Stade C par celui de la Préparation 3".
***Microanalyse élémentaire : (%mesuré (théorique))***
%C=67,63(68,06); %H=5,27(5,95); %N=10,08(10,13); %Cl-=4,53(4,27)

### Masse haute résolution (ESI+) :

Formule brute : C₃₅H₃₂ClN₄O₃
[M+H]⁺ calculé : 793,3708
[M+H]⁺ mesuré : 793,3704

### Stade B : 4-[{[1,2 Diméthyl-5-(6-{[(3R)-3-[3-(morpholin-4-yl)propyl]-3,4-dihydroisoquinolin-2(1H)-yl]carbonyl}-1,3-benzodioxol-5-yl)-1H-pyrrol-3-yl]carbonyl}(1-méthyl-1H-pyrrolo[2,3-b]pyridin-5-yl)amino]phényl phosphate de disodium

On procède selon un protocole analogue à celui décrit aux Stades E et F de l'Exemple 1.

**Sauf mention contraire, les composés des Exemples suivants sont synthétisés selon le procédé de l'Exemple 1 en utilisant : (i) l'acide approprié obtenu selon l'une des Préparation 1 à 9 et (ii) le dérivé de tétrahydroisoquinoline approprié obtenu selon l'une des Préparations 1' à 4', ainsi qu'au Stade C : (iii) l'amine NHR₃R₄ adéquate (une liste non exhaustive est proposée aux Préparations 1" à 8").**

### Exemple 13. 4-[{[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(pyridin-4-yl)amino]phényl phosphate de disodium

### Stade A : Chlorhydrate de 5-(5-chloro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(pyridin-4-yl)-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 4, et d'autre part le composé de la Préparation 1" utilisé au Stade C par celui de la Préparation 4". Le produit obtenu est enfin soumis à une étape de salification en présence d'acide chlorhydrique 1M dans l'éther. Après filtration et lyophilisation dans un mélange acétonitrile/eau, on obtient le composé attendu.

### Masse haute résolution (ESI+) :

Formule brute : C₃₉H₃₈ClN₅O₄
[M+H]⁺ calculé : 676,2685
[M+H]⁺ mesuré : 676,2684

### Stade B : 4-[{[5-(5-Chloro-2-{[(3S)-3-(morpholin-4-ylméthyl)-3,4-dihydroisoquinolin-2(1H)-yl]carbonyl}phényl)-1,2-diméthyl-1H-Pyrrol-3-yl]carbonyl}(pyridin-4-yl)amino]phényl phosphate de disodium

On procède selon un protocole analogue à celui décrit aux Stades B et C de l'Exemple 16.
**RMN ³¹P** (500 MHz, D₂O) δ ppm: -0,05
**IR (cm⁻¹):** v : C=O: 1631; v : (phosphate; éther):1243, 1136, 1112, 982; γ : >CH Ar : 883, 745

**Microanalyse élémentaire:**

| | %C | %H | *%N* |
|---|---|---|---|
| *Calculé* | *58,54* | *4*,*66* | *8,75* |
| *Trouvé* | *58,23* | *4,51* | *8*,*76* |

### Masse haute résolution (ESI+) :

Formule brute : C₃₉H₃₇ClN₅Na₂O₇P
[M-Na+2H]⁺ calculé : 778,2168
[M-Na+2H]⁺ mesuré: 778,2169

### Exemple 14. 4-[{[5-(5-Fluoro-4-méthoxy-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(1-méthyl-1H-pyrazol-4-yl)amino]phényl phosphate de disodium

### Exemple 15. 4-[{[5-(5-Fluoro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl} (1-méthyl-1H-pyrazol-4-yl)amino]phényl phosphate de disodium

### Stade A : Chlorhydrate de 5-(5-fluoro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(1-méthyl-1H-pyrazol-4-yl)-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 7, et d'autre part le composé de la Préparation 1" utilisé au Stade C par celui de la Préparation 5". Le produit obtenu est enfin soumis à une étape de salification en présence d'éther chlorhydrique dans l'éther. Après filtration et lyophilisation dans un mélange acétonitrile/eau, on obtient le composé attendu.
***Microanalyse élémentaire : (%mesuré (théorique))***
%C=65,69(65,28); %H=5,38(5,77); %N=11,18(12,02); %Cl-=5,61(5,07)

### Stade B : 4-[{[5-(5-Fluoro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(1-méthyl-1H-pyrazol-4-yl)amino]phényl phosphate de disodium

On procède selon un protocole analogue à celui décrit aux Stades B et C de l'Exemple 16.
RMN **³¹P (400/500 MHz, CD₃OD) δ ppm:** -0,5
**IR (cm⁻¹):** ν: C=O: 1628; v : (phosphate; éther):1238, 1114, 983

**Microanalyse élémentaire :**

| | %*C* | %*H* | *%N* |
|---|---|---|---|
| *Calculé* | *58,02* | *4*,*87* | *10,68* |
| *Trouvé* | *59,03* | *4,98* | *10,14* |

### Masse haute résolution (ESI+) :

Formule brute : C₃₈H₃₈FN₆Na₂O₇P
[M-2Na+3H]⁺ calculé : 743,2752
[M-2Na+3H]⁺ mesuré : 743,2760

### Exemple 16. 4-[{[1,2-Diméthyl-5-(7-{[(3S)-3-(morpholin-4-ylméthyl)-3,4-dihydroisoquinolin-2(1H)-yl]carbonyl}-2,3-dihydro-1,4-benzodioxin-6-yl)-1H-pyrrol-3-yl]carbonyl}(1-méthyl-1H-pyrazol-4-yl)amino]phény] phosphate de disodium

### Stade A : Chlorhydrate de N-(4-hydroxyphényl)-1,2-diméthyl-N-(1-méthyl-1H-pyrazol-4-yl}-5-(7-{[(3S)-3-(morpholin-4-ylméthyl)-3,4-dihydroisoquinolin-2(1H)-yl]carbonyl}-2,3-dihydro-1,4-benzodioxin-6-yl)-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 8, et d'autre part le composé de la Préparation 1" utilisé au Stade C par celui de la Préparation 5". Le produit obtenu est enfin soumis à une étape de salification en présence d'éther chlorhydrique 1M dans l'éther. Après filtration et lyophilisation dans un mélange acétonitrile/eau, on obtient le composé attendu.
***Microanalyse élémenaire : (% théorique : mesuré)***
%C=64,99:64,67; %H=5,86:5,67; %N=11,37:11,27; %Cl-=4,8:4,71

### Masse haute résolution (ESI+) :

Formule brute : C₄₀H₄₃N₆O₆
[M+H]⁺ calculé : 703,3236
[M+H]⁺ mesuré : 703,3239

### Stade B : N,N,N',N'-Tétraméthylphosphorodiamidate de 4-[{[1,2-diméthyl-5-(7-{[(3S)-3-(morpholin-4-ylméthyl)-3,4-dihydroisoquinoléin-2(1H)-yl]carbonyl}-2,3-dihydro-1,4-benzodioxin-6-yl)-1H-pyrrol-3-yl]carbonyl}(1-méthyl-1H-pyrazol-4-yl)amino]phényle

A une solution de 125 mg du composé du Stade A (0,18 mmol) dans du dichlorométhane (6 mL) est ajouté 55 µL de diazabicyclo[5.4.0]undec-7-ène (DBU ; 0,36 mmol), puis 33 µL de chlorure de bisdiméthylaminophosphoryle (0,19 mmol) et 2 mg de diméthylamino-4-pyridine (0,02 mmol). La réaction est laissée sous agitation pendant 15 heures, diluée dans du dichlorométhane puis dans une solution aqueuse saturée de carbonate de sodium. La phase aqueuse est extraite au dichlorométhane, puis les phases organiques sont rassemblées, lavées à l'eau, avec de la saumure et séchées au sulfate de magnésium. Après évaporation des solvants, le brut réactionnel est engagé directement dans l'étape suivante.

### Stade C : 4-[{[1,2-Diméthyl-5-(7-{[(3S)-3-(morpholin-4-ylméthyl)-3,4-dihydro isoquinolin-2(1H)-yl]carbonyl}-2,3-dihydro-1,4-benzodioxin-6-yl)-1H-pyrrol-3-yl] carbonyl}(1-méthyl-1H-pyrazol-4-yl)amino]phényl phosphate de disodium

A une solution de 125 mg de composé du Stade B (0,15 mmol) dans un mélange 1:1 d'acétonitrile et d'eau (5 mL) sont ajoutés 4 mL d'acide trifluoroacétique au goutte à goutte. Après 20 heures d'agitation à température ambiante, le milieu réactionnel est évaporé à sec en maintenant la température du bain-marie inférieure à 40°C, puis le résidu est traité par une solution de carbonate de sodium (95 mg ; 0,9 mmol) dans de l'eau (4 mL). Après 2 heures d'agitation à température ambiante, le milieu réactionnel est évaporé à sec puis 6 mL d'éthanol anhydre sont ajoutés. Le solide est filtré, et le filtrat est concentré à sec, puis purifié sur phase OASIS® (gradient acétonitrile/eau).
**RMN ³¹P** (500 MHz, D₂O) δ ppm: 0,9
**IR (cm⁻¹):** v : C=O: 1623; v : (phosphate; éther):1235, 1162,1115, 1065, 985; γ : >CH Ar :745

### Masse haute résolution (ESI+) :

Formule brute : C₄₀H₄₁N₆Na₂O₉P
[M-2Na+3H]⁺ calculé : 783,2902
[M-2Na+3H]⁺ mesuré : 783,2907

### Exemple 17. 5-[{[5-(5-Fluoro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl{(pyridin-4-yl)amino]pyrimidin-2-yl phosphate de disodium

### Exemple 18. 5-[{[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(pyridin-4-yl)amino]pyrimidin-2-yl phosphate de disodium

### Exemple 19. 4-({[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl{[1-(trideutériométhyl)-1H-pyrazol-4-yl]amino)phényl phosphate de disodium

### Stade A : Chlorhydrate de 5-(5-chloro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-[1-(trideutériométhyl)-1H-pyrazol-4-yl]-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 4, et d'autre part le composé de la Préparation 1" utilisé au Stade C par celui de la Préparation 6". Le produit obtenu est enfin soumis à une étape de salification en présence d'éther chlorhydrique 1M. Après filtration et lyophilisation dans un mélange acétonitrile/eau, on obtient le composé attendu.
***Microanalyse élémentaire : (% théorique : mesuré**)*
%C=63,51:63,41; %H=5,63:5,42, %N=11,69:11,61; %Cl⁻=4,93:4,85

### Masse haute résolution (ESI+-/FIA/HR ,ESI-/FIA) :

Formule brute : C₃₈ H₃₆ Cl D₃ N₆ O₄
[M+H]⁺ calculé: 682,2982
[M+H]⁺ mesuré : 682,2986

### Stade B: 4-({[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}[1-(trideutérométhyl)-1H-pyrazol-4-yl]amino)phényl phosphate de disodium

On procède selon un protocole analogue à celui décrit aux Stades B et C de l'Exemple 16.
**RMN ³¹P** (500 MHz, D₂O) δ ppm: 4,8
**IR (cm⁻¹):** v : C=O: 1626; v : (phosphate; éther):1243, 1141,1115, 982; γ : >CH Ar :880, 831

### Masse haute résolution (ESI/FIA/HR et MS/MS) :

Formule brute : C₃₈ H₃₅ Cl D₃ N₆ Na₂ O₇ P
[M+H]⁺ calculé : 806,2285
[M+H]⁺ mesuré : 806,2280

### Exemple 20. 4-[{(5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(5-cyano-1,2-diméthyl-1H-pyrrol-3-yl)amino]phényl phosphate de disodium

### Stade A : Chlorhydrate de 5-(5-chloro-2-{[(3S)-3-(morpholin-4-ylméthyl)-3,4-dihydroisoquinolin-2(1H)-yl]carbonyl}phényl)-N-(5-cyano-1,2-diméthyl-1H-pyrrol-3-yl)-N-(4-hydroxyphényl)-1,2-diméthyl-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 4, et d'autre part le composé de la Préparation 1" utilisé au Stade C par celui de la Préparation 7". Le produit obtenu est enfin soumis à une étape de salification en présence d'éther chlorhydrique 1M. Après filtration et lyophilisation dans un mélange acétonitrile/eau, on obtient le composé attendu.
**RMN ¹H** (500 MHz, dmso-d6) δ ppm: 11,2 (sl, 1H) ; 9,39 (sl,1H) ; 7,83 (d, 1 H) ; 7,54 (d, 1 H) ; 7,33 (s, 1 H) ; 7,14 (m, 2 H) ; 7 (m, 2 H) ; 6,8 (d, 2 H) ; 6,62 (d, 2 H) ; 6,57 (sl, 1 H); 5,26 (s, 1 H); 5,26 (m, 1 H); 4,64/4,03 (AB, 2 H); 4,01/3,92 (2m, 4 H); 3,75/3,43/3,15/3,02 (4m, 4 H) ; 3,59 (s, 3 H) ; 3,3/3,15 (2m, 2 H) ; 2,97 (s, 3 H) ; 2,69/2,52 (dd+d, 2 H) ; 2,06 (s, 3 H) ; 1,91 (s, 3 H)
***Microanalyse élémentaire :*** (% ***théorique : mesuré)***
%C=65,34:65,50; %H=5,62:5,15; %N=11,15:10,84 ; %Cl-=4,70:4,44

### Masse haute résolution (ESI+) :

Formule brute : C₄₁ H₄₁ Cl N₆ O₄
[M+H]⁺ calculé : 717,2952
[M+H]⁺ mesuré : 717,2951

### Stade B : 4-[{[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(5-cyano-1,2-diméthyl-1H-pyrrol-3-yl)amino]phényl phosphate de disodium

On procède selon un protocole analogue à celui décrit aux Stades B et C de l'Exemple 16,
**RMN ³¹P** (500 MHz, dmso-d6) δ ppm : 3,7
**IR (cm⁻¹):** v : -CN: 2210 cm⁻¹ ; v : C=O : 1623; v : (phosphate; éther): 1227,1133,1110, 982; γ : >CH Ar :884-741

**Microanalyse élémentaire :**

| | %*C* | *%H* | %*N* |
|---|---|---|---|
| *Calculé* | *58,54* | *4,79* | *9*,*99* |
| *Trouvé* | *58,75* | *4,71* | *10,18* |

### Masse haute résolution (ESI+-/FIA/HR) :

Formule brute : C₄₁ H₄₀ Cl N₆ Na₂ O₇ P
[M-2Na+H]⁺ calculé : 797,2614
[M-2Na+H]⁺ mesuré : 797,2618

### Exemple 21. 4-[{[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(5-cyano-1-méthyl-1H-pyrrol-3-yl)amino]phényl phosphate de disodium

### Stade A : Chlorhydrate de 5-(5-chloro-2-{[(3S)-3-(morpholin-4-ylméthyl)-3,4-dihydroisoquinolin-2(1H)-yl]carbonyl}phényl)-N-(5-cyano-1-méthyl-1H-pyrrol-3-yl)-N-(4-hydroxyphényl)-1,2-diméthyl-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 4, et d'autre part le composé de la Préparation 1" utilisé au Stade C par celui de la Préparation 8". Le produit obtenu est enfin soumis à une étape de salification en présence d'éther chlorhydrique 1M. Après filtration et lyophilisation dans un mélange acétonitrile/eau, on obtient le composé attendu.
***Microanalyse élémentaire :* (% *théorique : mesuré)***
%C=64,95:65,09; %H=5,45:5,20; %N=11,36:11,26; %Cl-=4,79:4,62

### Masse haute résolution (ESI/+) :

Formule brute : C₄₀ H₃₉ Cl N₆ O₄
[M+H]⁺ calculé : 703,2794
[M+H]⁺ mesuré : 703,2789

### Stade B : 4-[{[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(5-cyano-1-méthyl-1H-pyrrol-3-yl)amino]phényl phosphate de disodium

On procède selon un protocole analogue à celui décrit aux Stades B et C de l'Exemple 16.
**RMN ³¹P** (500 MHz, dmso-d6) δ ppm : 4,5
**IR (cm⁻¹):** ν : -CN: 2215 cm⁻¹ ; v : C=O 1626; v : (Phosphate; éther):1227, 1141,1112, 982; γ : >CH Ar :826-742

### Masse haute résolution (ESI+-/FIA/HR) :

Formule brute : C₄₀ H₃₈ Cl N₆ Na₂ O₇ P
[M-2Na+3H]⁺ calculé : 783,2457
[M-2Na+3H]⁺ mesuré : 783,2462

### Exemple 22. 4-[{[3-(6-{[(3R)-3-(Morpholin-4-ylméthyl)-3,4-dihydroisoquinolin-2(1H)-yl]carbonyl}-1,3-benzodioxol-5-yl)-5,6,7,8-tétrahydroindolizin-1-yl]carbonyl}(phényl)amino]phényl phosphate de disodium

### Stade A : Chlorhydrate de N-(4-hydroxyphényl)-3-{6-[((3R)-3-(4-morpholinylméthyl)-3,4-dihydro-2(1H)-isoquinolinyl)carbonyl]-1,3-benzodioxol-5-yl}-N-phényl-5,6,7,8-tétrahydro-1-indolizine carboxamide

On procède selon un protocole analogue à celui décrit aux Stades A-D de l'Exemple 1 en remplaçant le composé de la Préparation 1' utilisé au Stade A par le composé de la Préparation 4'. Le solide est ensuite solubilisé dans le dichlorométhane et 2 mL d'éther chlorhydrique 1 M sont ajoutés. Le tout est agité pendant 1 heure, puis évaporé à sec. Le chlorhydrate ainsi obtenu est dissous dans un mélange eau/ acétonitrile jusqu'à solubilisation totale, puis est lyophilisé.

**Microanalyse élémentaire :**

| | %*C* | *%H* | *%N* | *%Cl⁻* |
|---|---|---|---|---|
| *Calculé* | *69,11* | *5,80* | *7,50* | *4,74* |
| *Trouvé* | *68,89* | *5,23* | 7,41 | *4,62* |

***Pouvoir rotatoire :*** (α) D²⁰ = - 45,1 ° (c = 9 mg/mL, MeOH)

### Stade B : 4-[{[3-(6-{[(3R)-3-(Morpholin-4-ylméthyl)-3,4-dihydroisoquinolin-2(1H)-yl]carbonyl}-1,3-benzodioxol-5-yl)-5,6,7,8-tétrahydroindolizin-1-yl]carbonyl} (phényl)amino]phényl phosphate de disodium

On procède selon un protocole analogue à celui décrit aux Stades B et C de l'Exemple 16.
**RMN ³¹P** (400/500 MHz, dmso-d6) δ ppm : 2,6

**Microanalyse élémentaire :**

| | %*C* | %*H* | %*N* |
|---|---|---|---|
| *Calculé* | *61,87* | *4,95* | *6,71* |
| *Trouvé* | *61,33* | *4,93* | *7,14* |

### Masse haute résolution (ESI+-/FIA/HR) :

Formule brute : C₄₃ H₄₁ N₄ Na₂ O₉ P
[M-2Na+H]⁺calculé : 791,2840
[M-2Na+H]⁺ mesuré : 791,2845

### Exemple 23. 4-[(1-Méthyl-2,3-dihydro-1H-pyrrolo[2,3-b]pyridin-5-yl){[3-(2-{[(3S)-3-(morpholin-4-ylméthyl)-3,4-dihydroisoquinolin-2(1H-yl]carbonyl}-4-[2-oxo-2-(pipéridin-1-yl)éthoxy]phényl)-5,6,7,8-tétrahydroindolizin-1-yl]carbonyl}amino] phényl phosphate de disodium

### Stade A: 3-[(4-Benzyloxy-2-[(3S)-3-(morpholinométhyl)-3,4-dihydro-1H-isoquinoline-2-carbonyl]phényl]-5,6,7,8-tétrahydroindolizine-1-carboxylate de méthyle

A une solution de 14,19 g (35,0 mmol) de composé obtenu à la Préparation 9 dans 200 mL de diméthylformamide, sont ajoutés successivement le 4-[[(3*S*)-1,2,3,4-tétrahydroisoquinolin-3-yl]méthyl]morpholine (Préparation 1' ; 8,13 g; 35,0 mmol), l'hydroxybenzotriazole (6,15 g; 45,5 mmol), le chlorhydrate de 1-éthyl-3-(3'-diméthyl aminopropyl)-carbodiimide (6,70 g,; 45,5 mmol) et la triéthylamine (21.95 mL ; 0,16 mol). L'ensemble est ensuite agité pendant une nuit à température ambiante. Le milieu réactionnel est alors versé sur 400 mL d'acétate d'éthyle, puis lavé successivement avec une solution aqueuse saturée de NaHCO₃, de l'eau et de la saumure. Les phases aqueuses réunies sont extraites à l'acétate d'éthyle. Les phases organiques résultantes sont séchées sur sulfate de sodium, filtrées et concentrées sous pression réduite. Le produit obtenu est purifié par chromatographie sur gel de silice pour fournir le composé du titre.
**RMN ¹H** (500 MHz, dmso-d6, 300K) δ ppm: 7,5-7,3 (m, 5 H) ; 7,38 (d, 1 H) ; 7,2-6,9 (m, 4 H); 7,15 (dd, 1 H) ; 6,9 (d, 1 H); 6,35/6,25/6,08 (3*s, 1 H) ; 5,21/5,12 (3*s, 2 H) ; 5,09/4,85/3,7 (3*m, 1 H) ; 4,9-3,8 (8*d, 2 H) ; 4,2-3,4 (m, 2 H) ; 3,65/3,6/3,55 (3*s, 3 H) ; 3,6-3,4 (m, 4 H) ; 3-2,4 (m, 2 H) ; 2,9-1,8 (6*dd, 2 H) ; 2,5-1,95 (4*m, 4 H) ; 2,35-1,7 (6*m, 2 H) ; 2-1,45 (6*m, 4 H)

### Stade B: Acide 3-[4-benzyloxy-2-[(3S)-3-(morpholinométhyl)-3,4-dihydro-1H-isoquinoline-2-carbonyl]phényl]-5,6,7,8-tétrahydroindolizine-1-carboxylique

A une solution de 12,7 g (20 mmol) du composé obtenu au stade précédent dans 40 mL de dioxane sont ajoutés 40,1 mL d'une solution aqueuse de LiOH 1 M. L'ensemble est chauffé à 100°C pendant 1 nuit. Le milieu réactionnel est versé sur de l'eau, puis extrait à l'éther éthylique. La phase éthérée est extraite de nouveau une fois à l'eau. Les phases aqueuses réunies sont acidifiées à pH 4 par ajout d'acide citrique en poudre, puis extraites avec du dichlorométhane. La phase dichlorométhane est lavée avec de la saumure, séchée sur sulfate de sodium, filtrée et concentrée à sec. Le produit du titre est obtenu sous la forme d'une meringue.
**RMN ¹H** (500 MHz, dmso-d6, 300K) δ ppm: 11,35 (sl, 1 H) ; 7,5-7,3 (m, 5 H) ; 7,38 (m, 1 H); 7,2-6,9 (m, 4 H); 7,15 (m, 1 H); 6,9 (m, 1 H); 6,31/6,25/6,1 (3*s, 1 H); 5,22/5,2/5,15 (3*s, 2 H) ; 5,1/4,82/3,7 (3*m, 1 H) ; 4,85-3,8 (8*d, 2 H) ; 4,2-3,4 (m, 2 H) ; 3,6-3,45 (m, 4 H) ; 3-2,3 (m, 2 H) ; 2,9-1,8 (m, 2 H) ; 2,5-1,9 (6*m, 4 H) ; 2,35-1,8 (6*m, 2 H); 1,9-1,3 (m, 4 H)

### Stade C: 3-[4-Benzyloxy-2-[(3S)-3-(morpholinométhyl)-3,4-dihydro-1H-isoquinoline-2-carbonyl]phényl]-N-[4-[tert-butyl(diméthyl)silyl]oxyphényl]-N-(1-méthylpyrrol[2,3-b]pyridin-5-yl)-5,6,7,8-tétrahydroindolizine-1-carboxamide

L'acide obtenu au Stade B (9 g, 11,8 mmol) est mis en solution dans 90 mL de 1,2-dichloroéthane. On y ajoute 1,9 mL de 1-chloro-*N,N*,2-triméthylpropènylamine (14 mmol). Après 3 h d'agitation à température ambiante, 90 mL de toluène et 4,62 g de N*-*[4-[*tert-*butyl(diméthyl)silyl]oxyphényl]-1-méthyl-1*H*-pyrrolo[2,3-*b*]pyridin-5-amine (Préparation 3", 13 mmol) sont additionnés. Le milieu réactionnel est chauffé à 110°C pendant 20 h. Après retour à température ambiante, le milieu réactionnel est lavé avec de la saumure, séché sur Na₂SO₄, filtré et concentré à sec. Le résidu obtenu est purifié par chromatographie sur gel de silice (gradient dichlorométhane/éthanol) pour conduire au produit attendu.
**RMN ¹H** (500 MHz, dmso-d6, 300K) δ ppm : 7,95/7,8/7,75 (3*d, 1 H) ; 7,68/7,65/7,4 (3*d, 1 H) ; 7,4/7,3 (2*d, 1 H) ; 7,25-6,8 (m, 9 H) ; 7,05/6,9 (2*m, 1 H) ; 7-6,6 (3*dl, 2 H) ; 6,9 (m, 1 H) ; 6,75-6,45 (3*dl, 2 H) ; 6,7 (m, 1 H) ; 6,3 (2*d, 1 H) ; 5,15-4,95 (m, 2 H) ; 5,15/5,1/4,8 (3*s, 1 H) ; 4,95/4,6/3,5 (3*m, 1 H) ; 4,9-3,7 (8*d, 2 H) ; 3,8-3,3 (3*m, 2 H) ; 3,75/3,7/3,5 (3*s, 3 H) ; 3,45/3,3 (2*m, 4 H) ; 3-2,5 (3*m, 2 H) ; 3-2,3 (m, 2 H) ; 2,4-1,75 (5*m, 4 H) ; 2,25-1,7 (6*m, 2 H) ; 1,75-1,3 (m, 4 H) ; 0,7 (sl, 9 H) ; 0,1 (m, 6 H)

### Stade D: N-[4-[tert-Butyl(diméthyl)silyl]oxyphényl]-3-[4-hydroxy-2-[(3S)-3-(morpholino méthyl)-3,4-dihydro-1H-isoquinoline-2-carbonyl]phényl]-N-(1-méthylpyrrolo[2,3-b]pyridin-5-yl)-5,6,7,8-tétrahydroindolizine-1-carboxamide

A une solution dans 100 mL d'éthanol de 8,88 g (8,4 mmol) du composé obtenu au Stade C est ajouté 0,9 g de Pd/C à 10% sous bullage d'argon. Le mélange réactionnel est placé sous 1,2 bar d'hydrogène à température ambiante pendant 15 h. Le catalyseur est filtré et le solvant évaporé sous pression réduite pour fournir le composé du titre.
**RMN ¹H** (500 MHz, dmso-d6, 300K) δ ppm: 8,06/7,92/7,87 (3*d, 1 H) ; 7,75/7,5/7,39 (3*d, 1 H) ; 7,5 (m, 1 H) ; 7,28-6,9 (m, 5 H) ; 6,87/6,7 (2*m, 2 H) ; 6,76 (m, 1 H) ; 6,75/6,67/6,62 (3*m, 2 H) ; 6,67/6,46 (m, 1 H) ; 6,4/6,36 (2*m, 1 H) ; 5,19/5,13/4,9 (3*sl, 1 H); 5,06/4,7/3,6 (3*m, 1 H) ; 4,97/4,2/4,15/4,07 (4*m, 2 H); 4,87/4,81 (sl, 1H) ; 3,86/3,56/3,39 (3*m, 2 H) ; 3,78/3,57 (2*m, 3 H) ; 3,59/3,44 (2*m, 4 H) ; 2,96-2,61 (2*m, 2 H) ; 2,88/2,6 (2*m, 2 H) ; 2,59-1,81 (m, 6 H) ; 1,87-1,42 (m, 4 H) ; 0,89 (s, 9 H) ; 0,12 (m, 6 H)

### Stade E: N-[4-[tert-Butyl(diméthyl)silyl]oxyphényl]-N-(1-méthylpyrrolo[2,3-b]pyridin-5-yl)-3-[2-[(3S)-3-(morpholinométhyl)-3,4-dihydro-1H-isoquinoline-2-carbonyl]-4-[2-oxo-2-(1-pipéridyl)ethoxylphényl]-5,6,7,8-tétrahydroindolizine-1-carboxamide

Le composé du Stade D (3,0 g, 2,9 mmol) est mis en solution dans 100 mL de toluène. Y sont ajoutés 1,53 g (5,8 mmol) de triphénylphosphine et 0,62 g (4,3 mmol) de 2-hydroxy-1-(1-pipéridyl)éthanone. Le mélange est chauffé à 50°C, puis 1,01 g (4,3 mmol) d'azodicarboxylate de di-*tert*-butyle est additionné. Le milieu réactionnel est agité à 50°C pendant 1h puis laissé revenir à température ambiante avant d'ajouter 1 mL d'acide trifluoroacétique. Après une nuit sous agitation à température ambiante, le mélange est lavé successivement avec de l'eau, une solution saturée de NaHCO₃ et une solution de saumure. Les phases aqueuses réunies sont extraites à l'acétate d'éthyle. Les phases organiques résultantes sont séchées sur sulfate de sodium, filtrées et concentrées sous pression réduite. Le produit brut obtenu est purifié par chromatographie sur gel de silice (dichlorométhane/éthanol 98/2) pour conduire au composé attendu.
**RMN ¹H** (500 MHz, dmso-d6, 300K) δ ppm: 8,06/7,92/7,87 (3*d, 1 H) ; 7,75/7,51/7,4 (3*d, 1 H) ; 7,49 (2*d, 1 H) ; 7,29-6,89 (m, 5 H) ; 6,93 (m, 1 H) ; 6,88/6,7 (m, 2 H) ; 6,75/6,67 (m, 1 H) ; 6,75/6,68/6,59 (3*m, 2 H) ; 6,4/6,36 (2*m, 1 H) ; 5,2/5,16/4,92 (3*m, 1 H) ; 5,06/4,69/3,58 (3*m, 1 H) ; 4,97/4,25/4,16/4,03 (4*d, 2 H) ; 4,89/4,81 (2*m, 2 H) ; 3,79/3,59 (2*m, 3 H) ; 3,59/3,43/3,4 (3*m, 6 H) ; 3,58/3,43 (2*m, 4 H) ; 3,03-2,61 (m, 2 H) ; 2,97-2,65 (m, 2 H) ; 2,57-1,74 (m, 6 H) ; 1,89-1,3 (m, 10 H) ; 0,89 (2sl, 9 H) ; 0,11 (m, 6 H)

### Stade F: N-(4-Hydroxyphényl)-N-(1-méthylpyrrolo[2,3-b]pyridin-5-yl)-3-[2-[(3S)-3-(morpholinométhyl)-3,4-dihydro-1H-isoquinoline-2-carbonyl]-4-[2-oxo-2-(1-pipéridyl)ethoxy]phényl]-5,6,7,8-tétrahydroindolizine-1-carboxamide

A une solution dans 30 mL de tétrahydrofurane du composé obtenu au Stade E (2,92 g, 2,9 mmol) est additionnée une solution de fluorure de tétrabutylammonium 1M (3,14 mL, 3 mmol) dans le tétrahydrofurane à température ambiante. Après 5 minutes sous agitation, le milieu réactionnel est versé sur un mélange 50/50 d'acétate d'éthyle et d'une solution aqueuse saturée en NaHCO₃. La phase organique décantée est lavée à l'eau, puis avec de la saumure. Les phases aqueuses réunies sont extraites à l'acétate d'éthyle. Les phases organiques résultantes sont séchées sur sulfate de sodium, filtrées et concentrées sous pression réduite. Le produit brut obtenu est purifié par chromatographie sur gel de silice (gradient dichlorométhane/éthanol/ammoniaque) pour conduire au composé du titre.
**RMN ¹H** (500 MHz, dmso-d6, 300K) δ ppm: 9,4 (m, OH) ; 8,1-7,8 (3*d, 1 H) ; 7,7-7,3 (2*m, 1 H) ; 7,5/7,4 (2*m, 1 H) ; 7,3-6,9 (m, 4 H) ; 7,2 (m, 1 H) ; 6,9 (m, 1 H) ; 6,8-6,5 (m, 2 H) ; 6,7-6,5 (m, 2 H) ; 6,7 (m, 1 H) ; 6,4 (m, 1 H) ; 5,3-5 (m, 1 H) ; 5,1/4,7/3,6 (3*m, 1 H) ; 5 -3,6 (m, 2 H) ; 5-3,6 (m, 2 H) ; 4,8 (m, 2 H) ; 3,8-3,6 (m, 3 H) ; 3,6/3,4 (m, 2 H) ; 3,4 (m, 6 H) ; 3,1-2,5 (m, 2 H) ; 2,9-1,9 (m, 2 H) ; 2,5-1,7 (m, 4 H) ; 1,8-1,4 (m, 6 H) ; 1,6-1,3 (m, 4 H)

### Stade G: N-(4-Hydroxyphényl)-N-(1-méthyl-2,3-dihydropyrrolo[2,3-b]pyridin-5-yl)-3-[2-[(3S)-3-(morpholinométhyl)-3,4-dihydro-1H-isoquinoline-2-carbonyl]-4-[2-oxo-2-(1-pipéridyl)ethoxy]pheny/]-5,6,7,8-tétrahydroindolizine-1-carboxamide

A une solution dans 20 mL d'acide acétique du composé obtenu au Stade F (2,0 g, 2,2 mmol) est additionné 0,71 g (11 mmol) de cyanoborohydrure de sodium. Après 14 h d'agitation à température ambiante, 0,36 g (5,5 mmol) de cyanoborohydrure de sodium est rajouté, puis le mélange réactionnel est chauffé à 50°C pendant 3h avant un second ajout de 0,1 éq de cyanoborohydrure de sodium pour compléter la réaction en 30 min à 50°C. L'acide acétique est évaporé sous pression réduite, puis le résidu est repris dans du dichlorométhane et lavé avec une solution aqueuse saturée en NaHCO₃, de l'eau et de la saumure. Les phases aqueuses réunies sont extraites au dichlorométhane. Les phases organiques résultantes sont séchées sur sulfate de sodium, filtrées et concentrées sous pression réduite. Le produit brut obtenu est purifié par chromatographie sur gel de silice (gradient dichlorométhane/éthanol/ammoniaque) pour conduire au composé du titre sous forme d'une meringue.
**RMN ¹H** (500 MHz, dmso-d6, 300K) δ ppm: 9,3 (sl, 1 H) ; 7,5/7,4/7,3 (3*m, 1 H) ; 7,2/6,7 (2*m, 1 H) ; 7,2 (m, 1 H) ; 7,1-6,8 (m, 4 H) ; 6,9/6,7 (m, 1 H) ; 6,9 (m, 1 H) ; 6,8-6,5 (m, 2 H) ; 6,7-6,5 (m, 2 H) ; 5,3-5,1 (2*d, 1 H) ; 5,1/4,7/3,6 (3*m, 1 H) ; 4,9/4,2-3,5 (2*m, 1 H) ; 4,9/4,2-3,5 (2*, 1 H) ; 4,9-4,8 (m, 2 H) ; 3,6/3,4 (2*m, 4 H) ; 3,4/3,3 (m, 2 H) ; 3,4 (m, 6 H) ; 3,1-2,5 (m, 4 H) ; 3-2,4 (m, 2 H) ; 2,8/2,6 (m, 3 H) ; 2,6-1,7 (m, 6 H) ; 1,9-1,3 (m, 10 H)

### Stade H: Chlorhydrate du N-(4-hydroxyphényl)-N-(1-méthyl-2,3-dihydropyrrolo[2,3-b]pyridin-5-yl)-3-[2-[(3S)-3-(morpholinométhyl)-3,4-dihydro-1H-isoquinoline-2-carbonyl]-4-[2-oxo-2-(1-pipéridyl)ethoxy]phenyl]-5,6,7,8-tétrahydroindolizine-1-carboxamide

La base obtenue au Stade G (0,60 g, 0,69 mmol) est solubilisée dans de l'acétonitrile, puis salifiée avec 0,7 mL (0,7 mmol) d'une solution de HCl 1N. La solution est filtrée, congelée puis lyophilisée pour fournir le chlorhydrate du titre sous forme d'une poudre.
***Microanalyse élémentaire :* (*% théorique : mesuré)***
%C=68,02:68,06; %H=6,49:6,21; %N=10,89:10,87; %Cl=4,14:3,94

### Masse haute résolution (ESI+) :

Formule brute : C₅₁ H₅₇ N₇ O₆
[M+H]⁺calculé : 869-,4445
[M+H]⁺ mesuré : 864,4443

### Stade I: 4-[(1-Méthyl-2,3-(dihydro-1H-pyrrolo[2,3-b]pyridin-5-yl){[3-(2-{[3S)-3-(morpholin-4-ylméthyl)-3,4-dihydroisoquinolin-2(1H)-yl]carbonyl]-4-[2-oxo-2-(pipéridin-1-yl)éthoxy]phényl)-5,6,7,8-tétrahydroindolizin-1-yl]carbonyl}amino]phényl phosphate de disodium

On procède selon un protocole analogue à celui décrit aux Stades B et C de l'Exemple 16.
**IR (cm⁻¹):** v : C=O: 1625; v : (phosphate; éther):1229, 1138,1115, 982; γ : >CH Ar :880-748-745

**Microanalyse élémentaire :**

| | *%C* | *%H* | *%N* |
|---|---|---|---|
| *Calculé* | *62,00* | *5,71* | *9,92* |
| *Trouvé* | *61,45* | *5,53* | *9,96* |

### Masse haute résolution (ESI+) :

Formule brute : C₅₁ H₅₆ N₇ Na₂ O₉P
[M-2Na+3H]⁺ calculé : 944,4106
[M-2Na+3H]⁺ mesuré : 944,4116

### Exemple 24. 4-[{[5-(5-Chloro-2-{[(3S)-3-(morpholin-4-ylméthyl)-3,4-dihydroisoquinolin-2(1H-yl]carbonyl}phényl)-1,2-diméthyl-1H-pyrrol-3-yl]carbonyl}(1-méthyl-1H-pyrazol-4-yl)amino]phényl phosphate de disodium

### Stade A : Chlorhydrate de 5-(5-chloro-2-{(3S)-3-(morpholin-4-ylméthyl)-3,4-dihydroisoquinolin-2(1H)-yl]carbonyl}phényl)-N-(4-hydroxyphényl)-1,2-diméthyl-N-(1-méthyl-1H-pyrazol-4-yl)-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 4, et d'autre part le composé de la Préparation 1" utilisé au Stade C par celui de la Préparation 5". Le produit obtenu est enfin soumis à une étape de salification en présence d'éther chlorhydrique dans l'éther. Après filtration et lyophilisation dans un mélange acétonitrile/eau, on obtient le composé attendu.
***Microanalyse élémentaire : (% théorique : mesuré)***
%C=63,77:62,83; %H=5,63:5,83; %N=11,74:11,29; %Cl-=4,95:5,42

### Stade B : 4-[{[5-(5-Chloro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}(1-méthyl-1H-pyrazol-4-yl)amino]pltényl phosphate de disodium

On procède selon un protocole analogue à celui décrit aux Stades B et C de l'Exemple 16.
**IR (cm⁻¹):** v : C=O : 1625; v : (phosphate; éther): 1241, 1146,1112,983

**Microanalyse élémentaire :**

| | *%C* | *%H* | *%N* |
|---|---|---|---|
| *Calculé* | *56,83* | *4,77* | *10,46* |
| *Trouvé* | *56,82* | *4,58* | *10,43* |

### Masse haute résolution (ESI+) :

Formule brute : C₃₈ H₃₈ Cl N₆ Na₂ O₇ P
[M-2Na+3H]⁺ calculé : 759,2457
[M-2Na+3H]⁺ mesuré : 759,2465

### Exemple 25. 4-[(5-Cyano-1,2-diméthyl-1H-pyrrol-3-yl){[5-(5-fluoro-2-{[(3S)-3-(morpholin-4-ylméthyl)-3,4-dihydroisoquinolin-2(1H)-yl]carbonyl}phényl)-1,2-diméthyl-1H-pyrrol-3-yl]carbonyl}amino]phényl phosphate de disodium

### Stade A: Chlorhydrate de N-(5-cyano-1,2-diméthyl-1H-pyrrol-3-yl)-5-(5-fluoro-2-{[(3S)-3-(morpholin-4-ylméthyl)-3,4-dihydroisaquinolin-2(1H)-yl]carbonyl}phényl)-N-(4-hydroxyphényl)-1,2-diméthyl-1H-pyrrole-3-carboxamide

On procède selon un protocole analogue à celui décrit aux Stades A-D de l'Exemple 1 en remplaçant d'une part le composé de la Préparation 1 utilisé au Stade A par le composé de la Préparation 7, et d'autre part le composé de la Préparation 1" utilisé au Stade C par celui de la Préparation 7". Le produit obtenu est enfin soumis à une étape de salification en présence d'éther chlorhydrique dans l'éther. Après filtration et lyophilisation dans un mélange acétonitrile/eau, on obtient le composé attendu.

### Masse haute résolution (ESI/FlA/HR et MS/MS) :

Formule brute : C₄₁ H₄₁ FN₆O₄
[M+H]⁺ calculé : 701,3246
[M+H]⁺ mesuré : 701,3282

### Stade B: 4-[(5-Cyano-1,2-diméthyl-1H-pyrrol-3-yl){[5-(5-fluoro-2-{[(3S)-3-(morpholin-4-ylméthyl)-3,4-dihydroisoquinolin-2(1H-yl]carbonyl}phényl}-1,2-diméthyl-1H-pyrrol-3-yl]carbonyl}amino]phényl phosphate de disodium

On procède selon un protocole analogue à celui décrit aux Stades B et C de l'Exemple 16.
**RMN ³¹P** (400/500 MHz, CD₃OD) δ ppm: -0,5
**IR (cm⁻¹):** v : -CN: 2211 cm⁻¹ v : C=O : 1629; v : (phosphate; éther): 1236, 1114, 984

**Microanalyse élémentaire :**

| | *%C* | %*H* | *%N* |
|---|---|---|---|
| *Calculé* | *59,71* | *4*,*89* | *10,19* |
| *Trouvé* | *60,09* | *4,95* | *9,88* |

### Masse haute résolution (ESI+) :

Formule brute : C₄₁ H₄₀ F N₆ Na₂ O₇P
[M-2Na+3H]⁺calculé : 781,2909
[M-2Na+3H]⁺ mesuré : 781,2898

### ETUDES PHARMACOLOGIQUES ET PHARMACOCINETIQUES

Dans un souci de clarification, dans tout ce qui suit, les composés de formule (I') seront référencés comme « drogue de l'Exemple x » dont ils dérivent. A titre d'exemple, le *N-(4-*hydroxyphényl)-3-{6-[((3*S*)-3-(4-morpholinylméthyl)-3,4-dihydro-2(1*H*)-isoquinolinyl) carbonyl]-1,3-benzodioxol-5-yl}-*N*-phényl-5,6,7,8-tétrahydro-1-indolizine carboxamide sera référencé « drogue de l'Exemple 1 ».

### EXEMPLE A1 : Induction de l'activité caspase in vivo par les composés de formule (I')

La capacité des composés de formule (I') à activer la caspase 3 est évaluée dans un modèle de xénogreffe de cellules leucémiques RS4 ;11.
1.10⁷ cellules RS4 ;11 sont greffées par voie sous-cutanée dans des souris immunodéprimées (souche SCID). 25 à 30 jours après la greffe, les animaux sont traités par voie orale par les différents composés. Seize heures après le traitement, les masses tumorales sont récupérées, lysées et l'activité caspase 3 est mesurée dans les lysats tumoraux.

Cette mesure enzymatique est réalisée en dosant l'apparition d'un produit de clivage fluorigénique (activité DEVDase, Promega). Elle est exprimée sous la forme d'un facteur d'activation correspondant au rapport entre les deux activités caspases : celle pour les souris traitées divisée par celle pour les souris contrôles.

Le *N-*(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-5,6,7,8-tétrahydro-1-indolizine carboxamide (aussi appelée drogue de l'Exemple 1) a été testé. A la dose de 100 mg/kg *p.o.,* le facteur d'activation des caspases *in vivo* est de 29,3.

Les résultats obtenus montrent que les composés de formule (I') sont capables d'induire l'apoptose dans les cellules tumorales RS4 ; 11 *in vivo.*

### EXEMPLE A2 : Quantification de la forme clivée de la caspase 3 in vivo induite par

### les composés de formule (I').

La capacité des composés de formule (I') à activer la caspase 3 est évaluée dans un modèle de xénogreffe de cellules leucémiques RS4 ;11.
1.10⁷ cellules RS4; 11 sont greffées par voie sous-cutanée dans des souris immunodéprimées (souche SCID). 25 à 30 jours après la greffe, les animaux sont traités par voie orale par les différents composés. Après le traitement, les masses tumorales sont récupérées, lysées et la forme clivée (activée) de la caspase 3 est quantifiée dans les lysats tumoraux.

Cette quantification est réalisée en utilisant l'essai « Meso Scale Discovery (MSD) ELISA platform » qui dose spécifiquement la forme clivée de la caspase 3. Elle est exprimée sous la forme d'un facteur d'activation correspondant au rapport entre la quantité de caspase 3 clivée chez les souris traitées divisée par la quantité de caspase 3 clivée chez les souris contrôles.

Les résultats montrent que les composés de formule (I') sont capables d'induire l'apoptose dans les cellules tumorales RS4 ; 11 *in vivo.*

**Tableau 1 : Facteurs d'activation des caspases (caspase 3 clivée essai MSD dans les tumeurs des souris traitées versus souris contrôles) in vivo, après traitement par voie orale**

| Composé testé | Dose (mg/kg) | Temps de prélèvement | Facteur d'activation +/S.E.M. |
|---|---|---|---|
| Drogue de l'exemple 13 | 12,5 | 2h | 24,5 +/- 7,5 |
| Drogue de l'exemple 19 | 12,5 | 2h | 13,5 +/- 1,2 |
| Drogue de l'exemple 20 | 12,5 | 2h | 52,0 +/- 8,6 |
| Drogue de l'exemple 21 | 12,5 | 2h | 22,6 +/- 2,4 |
| Drogue de l'exemple 24 | 25 | 2h | 45,7 +/- 2,0 |
| Drogue de l'exemple 25 | 12,5 | 2h | 38,7 +/- 10,7 |
| Drogue de l'exemple 15 | 25 | 2h | 29,8 +/- 4,0 |

### EXEMPLE A3 : Quantification de la forme clivée de la caspase 3 in vivo induite par les composés de formule (I).

La capacité des composés de formule (I) à activer la caspase 3 est évaluée dans un modèle de xénogreffe de cellules leucémiques RS4 ;11 selon le protocole présenté à l'Exemple A2.

**Tableau 2 : Facteurs d'activation des caspases (caspase 3 clivée essai MSD dans les tumeurs des souris traitées versus souris contrôles) in vivo, après traitement par voie orale**

| Composé testé | Dose (mg/kg) | Temps de prélèvement | Facteur d'activation +/- S.E.M. |
|---|---|---|---|
| Exemple 13 | 12,5 | 2h | 58,6 +/- 4,6 |
| Exemple 1 | 50 | 2h | 21,2 +/- 1,3 |
| Exemple 19 | 12,5 | 2h | 27,5 +/- 3,5 |
| Exemple 20 | 12,5 | 2h | 62,1 +/- 3,4 |
| Exemple 21 | 25 | 2h | 55,2 +/- 6,2 |
| Exemple 24 | 25 | 2h | 60,5 +/- 4,5 |
| Exemple 25 | 12,5 | 2h | 61,8 +/- 8,9 |
| Exemple 15 | 25 | 2h | 12,1 +/- 1,1 |

### EXEMPLE B : Solubilité des composés de formule (I)

La solubilité des composés de formule (I) a été mesurée dans l'eau et comparée à celle des composés de formule (I').

Plus précisément, le 4-[{[3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizin-1-y]carbonyl} (phényl)amino]phényl phosphate de disodium (aussi appelé composé de l'Exemple 1) a été testé et comparé au *N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N-*phényl-5,6,7,8-tétrahydro-1-indolizine carboxamide (aussi appelée drogue de l'Exemple 1).

La solubilité du composé de l'Exemple 1 dans l'eau est supérieure ou égale à 10 mg/mL (12,6 mM) alors que celle de la drogue associée n'est que de 40 µg/mL (56.2 µM). Les solubilités des composés ont également été mesurées dans un milieu tamponné à pH physiologique (cf tableau 3).

**Tableau 3 : Solubilités en milieu aqueux (solution tampon : phosphate 0,33 M, pH=7,4) des composés de formule (I) et des composés de formule (I') associés, mesurées à quatre concentrations : 10µM, 20µM, 50µM et 100µM**

| Composé testé | Solubilité à 10µM | Solubilité à 20µM | Solubilité à 50 µM | Solubilité à 100 µM |
|---|---|---|---|---|
| Exemple 19 | Soluble | Soluble | Soluble | Soluble |
| Drogue de l'Exemple 19 | Soluble | Soluble | Soluble | Insoluble |
| Exemple 20 | Soluble | Soluble | Soluble | Soluble |
| Drogue de l'Exemple 20 | Soluble | Insoluble | Insoluble | Insoluble |
| Exemple 25 | Soluble | Soluble | Soluble | Soluble |
| Drogue de l'Exemple 25 | Soluble | Soluble | Insoluble | Insoluble |
| Exemple 1 | Soluble | Soluble | Soluble | Soluble |
| Drogue de l'exemple 1 | Insoluble | Insoluble | Insoluble | Insoluble |

Les résultats montrent que les composés de formule (I) sont beaucoup plus solubles que les composés de formule (I'). Seuls les composés de formule (1) montrent des solubilités supérieures ou égales à 100µM.

### EXEMPLE C : Conversion in vivo des composés de formule (I)

Le profil pharmacocinétique des dérivés phosphates de formule (I) est évalué dans une formulation lipidique et en solution aqueuse chez des souris SCID femelles. Il est comparé au profil pharmacocinétique des composés de formule (I') dans une formulation lipidique. Plus précisément, le 4-[{[3-(6-{[(3*S*)-3-(morpholin-4-ylméthyl)-3,4-dihydroisoquinolin-2(1*H*}-yl]carbonyl}-1,3-benzodioxol-5-yl)-5,6,7,8-tétrahydroindolizin-1-yl]carbonyl} (phényl) amino]phényl phosphate de disodium (aussi appelé composé de l'Exemple 1) a été testé et comparé au *N*-(4-hydroxyphényl)-3-{6-[((3,*S*)-3-(4-morpholinyl méthyl)-3,4-dihydro-2(1*H*)-isoquinolinyl)carbonyl]-1,3-benzodioxol-5-yl}-*N*-phényl-5,6,7,8-tétrahydro-1-indolizine carboxamide (aussi appelée drogue de l'Exemple 1).

### Formulation lipidique du composé de l'Exemple 1

Le composé de l'Exemple 1 est préparé dans un mélange éthanol anhydre/polyéthylène glycol 300/ eau (10/40/50, v/v/v) destiné à une administration par voie *p.o.* L'étude est réalisée sur 2 groupes de souris SCID dans lesquelles le composé de l'Exemple 1 est administré dans les conditions suivantes :
- Groupe 1 : 3 mg/kg *p*.*o*. (gavage, 10 mL/kg),
- Groupe 2 : 25 mg/kg *p.o*. (gavage, 10 mL/kg).

Des échantillons de sang sont prélevés aux temps suivants (3 échantillons par animal et 3 animaux pour chaque temps): 0,25 h, 0,5 h, 1 h, 2 h, 6 h et 24 h après l'administration orale.

### Formulation aqueuse du composé de l'Exemple 1

Le composé de l'Exemple 1 est aussi administré par voie orale en milieu aqueux chez la souris SCID, dans les conditions suivantes :
- Groupe 1 : 30 mg/kg *p.o.* en solution dans du carbonate de sodium 1 mM (gavage, 10 mL/kg),
- Groupe 2 : 100 mg/kg *p*.*o*. dans l'eau (gavage, 10 mL/kg).

Des échantillons de sang sont prélevés au temps suivants (3 animaux pour chaque temps et 1 échantillon par animal): 0,25 h, 0,5 h, 1 h, 2 h, 4 h, 6 h and 24 h après l'administration orale.

Quelle que soit la formulation du composé de l'Exemple 1, le sang ainsi collecté est centrifugé et le plasma est transféré dans des tubes contenant de l'acide chlorhydrique 1 M. Les concentrations dans le plasma du dérivé phosphate (prodrogue) et de son homologue hydroxylé (drogue) sont déterminées simultanément en utilisant une technique de chromatographie liquide couplée à une détection par spectométrie de masse (TFC-LC-MS/MS). La limite de détection pour chacune des deux entités est de 0,5 ng/mL.

### Formulation lipidique de la drogue du composé de l'Exemple 1

La drogue de l'Exemple 1 est préparée dans un mélange éthanol anhydre/polyéthylène glycol 300/ eau (10/40/50, v/v/v) destiné à une administration par voie *p.o.* L'étude est réalisée sur plusieurs groupes de souris SCID dans laquelle la drogue de l'Exemple 1 est administrée dans les conditions suivantes :
- Groupe 1 : 3 mg/kg *p.o.* (gavage, 10 mL/kg),
- Groupe 2 : 30 mg/kg *p.o.* (gavage, 10 mL/kg),
- Groupe 3 : 25 mg/kg *p.o*. (gavage, 10 mL/kg).
- Groupe 4 : 100 mg/kg *p.o.* (gavage, 10 mL/kg).

Des échantillons de sang sont prélevés aux temps suivants (3 animaux pour chaque temps et 3 échantillons par animal pour les groupes 1-2 ou 1 échantillon par animal pour les groupes 3 et 4):
- p.o : avant administration puis 0,25 h, 0,5 h, 0,75 h, 1 h, 2 h, 4 h, 6 h, 8 h, 16 h et 24 h après l'administration orale à 3 et 30 mg/kg,
- p.o : à 0,5 h, 2 h, 6 h, 16 h et 24 h après l'administration orale à 25 mg/kg et 0,5 h, 1 h, 2 h, 6 h, 16 h, 30 h et 48 h après l'administration orale à 100 mg/kg.

Les plasmas des échantillons de sang collectés après administration des formulations lipidiques de la drogue du composé de l'Exemple 1 sont analysés par chromatographie liquide couplée à une détection par spectrométrie de masse. La limite de quantification de la drogue du composé de l'Exemple 1 est inférieure ou égale 0,5 ng/mL.

Une analyse pharmacocinétique non compartimentale est réalisée sur les valeurs moyennes des concentrations plasmatiques des composés testés. Les résultats sont présentés dans les tableaux 4 et 5 ci-dessous.

Les résultats montrent que quels que soient la dose (de 3 à 100 mg/kg) et le véhicule (formulation de type lipidique ou aqueuse), la prodrogue de formule (I) est rapidement et majoritairement convertie *in vivo* en la drogue correspondante de formule (I') (voir tableau 4). L'exposition plasmatique de la prodrogue (Cₘₐₓ, AUC) est faible par comparaison à celle de la drogue correspondante. Les résultats montrent également que la concentration plasmatique de la drogue ainsi mesurée (après administration de la prodrogue) est équivalente voire supérieure à celle mesurée après une administration directe de la drogue par voie orale (voir tableau 5).

**Tableau 4**

| **Composé administré** | **Composé mesuré** | |
|---|---|---|
| | **Exemple 1** | **Drogue de l'Exemple 1** |
| **Exemple 1,3 mg/kg** ***p.o.** Formulation lipidique* | Cₘₐₓ (ng/mL) = 16 | Cₘₐₓ (ng/mL) = 342 |
| | Tₘₐₓ (h)=0,25 | Tₘₐₓ (h)= 0,25 |
| | AUCₜ (ng.h/mL) = 5 | AUCₜ (ng.h/mL) = 314 |
| **Exemple 1**,**25 mg/kg *p.o*.** *Formulation lipidique* | Cₘₐₓ (ng/mL) = 244 | Cₘₐₓ (ng/mL) = 6204 |
| | Tₘₐₓ (h)= 0,25 | Tₘₐₓ (h)= 0,5 |
| | AUCₜ (ng.h/mL) = 92 | AUCₜ (ng.h/mL) = 20952 |
| **Exemple 1,30 mg/kg** ***p.o.** Formulalion aqueuse* | Cₘₐₓ (ng/mL) = 391 | Cₘₐₓ (ng/mL) = 11967 |
| | Tₘₐₓ (h)= 1,0 | Tₘₐₓ (h)= 0,5 |
| | AUCₜ (ng.h/mL) = 879 | AUC, (ng.h/mL) = 49416 |
| **Exemple 1, 100 mg/kg *p.o*.** *Formulation aqueuse* | Cₘₐₓ (ng/mL) = 359 | Cₘₐₓ (ng/mL) = 28066 |
| | Tₘₐₓ (h)= 2,0 | Tₘₐₓ (h)= 2,0 |
| | AUCₜ (ng.h/mL) = 797 | AUCₜ (ng.h/mL) = 168478 |

**Tableau 5**

| **Composé administré** | **Composé mesuré** |
|---|---|
| | **Drogue de l'Exemple 1** |
| **Drogue de l'Exemple 1, 3 mg/** kg *p.o. Formulation lipidique* | Cₘₐₓ (ng/mL) = 295 |
| | Tₘₐₓ (h)= 0,25 |
| | AUCₜ (ng.h/mL) = 225 |
| **Drogue de l'Exemple 1, 25 mg/kg *p.o*.** *Formulation lipidique.* | Cₘₐₓ (ng/mL) = 5070 |
| | Tₘₐₓ (h)= 2,0 |
| | AUCₜ (ng.h/mL) = 20400 |
| **Drogue de l'Exemple 1, 30 mg**/**kg** ***p**.**o***. *Formulation lipidique* | Cₘₐₓ (ng/mL) = 8580 |
| | Tₘₐₓ (h)= 1,0 |
| | AUCₜ (ng.h/mL) = 24200 |
| **Drogue de l'Exemple 1,100 mg/kg *p.o*.** *Formulation lipidique* | Cₘₐₓ (ng/mL) = 25878 |
| | Tₘₐₓ (h)= 0,5 |
| | AUCₜ (ng.h/mL) = 148046 |

Plus précisément, l'administration *p.o.* de la prodrogue dans un véhicule aqueux permet d'obtenir des concentrations plasmatiques en drogue équivalentes voires supérieures à celles obtenues après l'administration *p.o.* directe de la drogue dans un véhicule lipidique. La prodrogue offre donc des facilités de formulation par rapport à la drogue correspondante, notamment en milieu aqueux, ce qui est très avantageux dans la perspective d'un développement clinique. En effet, comme le montre l'Exemple D, la drogue de l'Exemple 1 est difficilement formulable en milieu aqueux.

### Formulation aqueuse des composés des Exemples 20 et 25

Les composés des Exemples 20 et 25 sont administrés par voie orale en milieu aqueux chez la souris SCID, dans les conditions suivantes :
- Groupe 1 : 3 mg/kg *p.o*. en solution dans du carbonate de sodium 1 M (gavage, 10 mL/kg),
- Groupe 2 : 25 mg/kg *p.o*. en solution dans du carbonate de sodium 1 M (gavage, 10 mL/kg),

Des échantillons de sang sont prélevés aux temps suivants (3 animaux pour chaque temps): 0,25 h, 0,5 h, 1 h, 2 h, 6 h and 24 h après l'administration orale.

Le sang ainsi collecté est centrifugé et le plasma est transféré dans des tubes contenant de l'acide chlorhydrique 1 M. Les concentrations dans le plasma du dérivé phosphate (prodrogue) et de son homologue hydroxylé (drogue) sont déterminées simultanément en utilisant une technique de chromatographie liquide couplée à une détection par spectrométrie de masse (TFC-LC-MS/MS). La limite de quantification pour chacune des deux entités est de 0,5 ng/mL.

### Formulation lipidique de la drogue des composés des Exemples 20 et 25

Les drogues des Exemples 20 et 25 sont préparées dans un mélange polyéthylène glycol 300/éthanol/phosal 50PG (30/10/60, v/v/v) destiné à une administration par voie p. o. chez la souris SCID, dans les conditions suivantes :
- Groupe 1 : 3 mg/kg *p.o*. (gavage, 10 mL/kg),
- Groupe 2 : 25 mg/kg *p.o*. (gavage, 10 mL/kg).

Des échantillons de sang sont prélevés aux temps suivants (3 animaux pour chaque temps): 0,25 h, 0,5 h, 1 h, 2h, 6h and 24 h après l'administration orale.

Le sang ainsi collecté est centrifugé et le plasma est transféré dans des tubes contenant de l'acide chlorhydrique 1M. Les concentrations dans le plasma de la drogue sont déterminées en utilisant une technique de chromatographie liquide couplée à une détection par spectrométrie de masse (TFC-LC-MS/MS). La limite de quantification est de 0,5 ng/mL.

Une analyse pharmacocinétique non compartimentale est réalisée. Les résultats moyens sont présentés dans les tableaux **6, 7, 8 et 9** ci-dessous.

**Tableau 6, Exemple 20**

| **Composé administré** | **Composés mesurés** | |
|---|---|---|
| | **Exemple 20** | **Drogue de l'Exemple 20** |
| **Exemple 20, 3 mg/ kg** ***p.o.** Formulation aqueuse* | Cₘₐₓ (ng/mL) = BLQ | Cₘₐₓ (ng/mL) = 56 |
| | Tₘₐₓ (h)= ND | Tₘₐₓ (h)= 1,0 |
| | AUCₜ (ng.h/mL) = ND | AUCₜ (ng.h/mL) = 51 |
| **Exemple 20,25 mg**/**kg *p***.***o***. *Formulation aqueuse* | Cₘₐₓ (ng/mL) = 127 | Cₘₐₓ (ng/mL) = 3701 |
| | Tₘₐₓ (h)= 0,25 | Tₘₐₓ (h)= 1,0 |
| | AUCₜ (ng.h/mL) = 106 | AUCₜ (ng.h/mL) = 8724 |

| | | |
|---|---|---|
| *ND : non déterminé* *BLQ : sous la limite de quantification* | | |

**Tableau 7, Exemple 20**

| **Composé administré** | **Composé mesuré** |
|---|---|
| | **Drogue de l'Exemple 20** |
| **Drogue de l'Exemple 20, 3 mg**/ **kg *p.o.*** | Cₘₐₓ (ng/mL) = 39 |
| *Formulation lipidique* | Tₘₐₓ (h)= 1,0 |
| | AUCₜ (ng.h/mL) = 55 |
| **Drogue de l'Exemple 20,25 mg/kg *p.o.*** | Cₘₐₓ (ng/mL) = 5524 |
| *Formulation lipidique* | Tₘₐₓ (h)= 2,0 |
| | AUCₜ (ng.h/mL) = 10172 |

**Tableau 8, Exemple 25**

| **Composé administré** | **Composés mesurés** | |
|---|---|---|
| | **Exemple 25** | **Drogue de l'Exemple 25** |
| **Exemple 25, 3 mg/ kg *p***.***o***. *Formulation aqueuse* | Cₘₐₓ (ng/mL) = 17 | Cₘₐₓ (ng/mL) = 29 |
| | Tₘₐₓ (h)= 1,0 | Tₘₐₓ (h)= 1.0 |
| | AUCₜ (ng.h/mL) = 14 | AUCₜ (ng.h/mL) = 31 |
| **Exemple 25,25 mg/kg** ***p.o.** Formulation aqueuse* | Cₘₐₓ (ng/mL) = 106 | Cₘₐₓ (ng/mL) = 2232 |
| | Tₘₐₓ (h)= 1,0 | Tₘₐₓ (h)= 1,0 |
| | AUCₜ (ng.h/mL) = 114 | AUCₜ (ng.h/mL) = 3965 |

**Tableau 9, Exemple 25**

| **Composé administré** | **Composé mesuré** |
|---|---|
| | **Drogue de l'Exemple 25** |
| **Drogue de l'Exemple 25, 3 mg/**kg *p.o. Formulation lipidique* | Cₘₐₓ (ng/mL) = 33 |
| | Tₘₐₓ (h)= 1,0 |
| | AUCₜ (ng.h/mL) = 37 |
| **Drogue de l'Exemple 25, 25 mg**/**kg *p***.***o***. *Formulation lipidique* | Cₘₐₓ (ng/mL) = 3004 |
| | Tₘₐₓ (h)= 1,0 |
| | AUCₜ (ng.h/mL) = 5704 |

Les résultats montrent que quels que soient la dose (de 3 et 25 mg/kg), les prodrogues de formule (I) sont rapidement et majoritairement converties *in vivo* en drogues correspondantes de formule (1') (voir tableaux 6, 7, 8 et 9). Les expositions plasmatiques des prodrogues (Cₘₐₓ, AUC) sont faibles par comparaison aux expositions des drogues correspondantes. Les résultats montrent également que les concentrations plasmatiques des drogues ainsi mesurées (après administration des prodrogues) sont équivalentes à celles mesurées après une administration directe des drogues par voie orale (voir tableaux 7 et 9).

### EXEMPLE D : Profil pharmacocinétique in vivo des composés de formule (I')

Le profil pharmacocinétique du *N*-(4-hydroxyphényl)-3-{6-[((3*S*)-3-(4-morpholinyl méthyl)-3,4-dihydro-2(1*H)*-isoquinolinyl)carbonyl]-1,3-benzodioxol-5-yl}-*N-*phényl-5,6,7,8-tétrahydro-1-indolizine carboxamide (aussi appelée drogue de l'Exemple 1) est aussi évalué dans une formulation lipidique et aqueuse chez le rat Wistar.

La drogue de l'Exemple 1 est préparée en suspension aqueuse dans l'hydroxyéthylcellulose à 1 % (m/v) dans l'eau et comparée à une formulation lipidique constituée d'un mélange éthanol anhydre/polyéthylène glycol 400/ phosal 50PG (10/30/60, v/v/v). Les deux formulations sont administrées par voie orale chez le rat mâle Wistar (3 rats/formulation) à la dose de 100 mg/kg p.o (gavage, 10 mL/kg).

Des échantillons de sang sont prélevés aux temps suivants chez chaque animal (3 animaux/temps): 0,25 h, 0,5 h, 0,75 h 1 h, 2 h, 4 h, 8 h et 24 h après l'administration orale.

Les concentrations du composé testé sont déterminées dans le plasma après extraction suivie d'une chromatographie liquide couplée à une détection par spectrométrie de masse. La limite de quantification est à 0,1 ng/mL. Les résultats sont présentés dans le tableau ci-dessous :

**Tableau 10**

| **Composé administré** | **Composé mesuré** |
|---|---|
| | **Drogue de l'Exemple 1** |
| **Drogue de l**'**Exemple 1**,**100 mg/ kg p.o** | Cₘₐₓ (ng/mL) = 816 |
| *Formulation aqueuse* | AUCₜ (ng.h/mL) = 3480 |
| **Drogue de l'Exemple 1,100 mg/ kg p.o** | Cₘₐₓ (ng/mL) = 5070 |
| *Formulation lipidique* | AUCₜ (ng.h/mL) = 42900 |

Les résultats montrent que la formulation lipidique permet une bien meilleure exposition plasmatique de la drogue de l'Exemple 1 que la formulation aqueuse.

### EXEMPLE E : Test in vitro sur cellules humaine Caco-2.

Le passage cellulaire de A vers B (Apical vers Basolatéral) des dérivés phosphates de formule (I) et des composés de formule (I') (drogues correspondantes) est étudié sur cellules humaine Caco-2. Chaque composé est déposé en apical à 1 ou 3 µM (en duplicate), puis incubé pendant 120 min.

Plusieurs prélèvements sont effectués au cours de l'expérience ;
- En apical : juste après le dépôt (t=0) et à 120 min
- En basolatéral : à la fin de l'expérience (120 min)

Les concentrations du dérivé phosphate (prodrogue) et/ou de son homologue hydroxylé (drogue) sont déterminées par chromatographie liquide couplée à une détection par spectométrie de masse (LC-MS/MS). La limite de quantification pour chacune des deux entités est de 2 ng/mL.

La perméabilité apparente (Pₐₚₚ) et la fraction absorbée (F_{abs}) prédite chez l'homme sont calculées pour la prodrogue, pour la drogue après incubation de la prodrogue et pour la drogue après incubation de la drogue (Hubatsch et al, Nat Protoc. 2007; 2(9), 2111-2119).

Le rendement de l'expérience, qui correspond au ratio (en pourcentage) de la quantité totale de composé retrouvé en fin d'expérience versus celle incubée, est aussi calculé.

Les résultats ont été rassemblés dans le tableau 11. Ils montrent que les prodrogues des composés de formule (I) sont fortement dégradées au cours de l'expérience (rendements d'expérience < 1,5%), entrainant ainsi la formation des drogues associées dans des proportions importantes.

Au final, la fraction absorbée prédite chez l'homme pour les drogues formées après incubation des prodrogues est similaire à celle obtenue après l'incubation des drogues.

**Tableau 11**

| **Composé administré** | **Composés mesurés** | |
|---|---|---|
| | **Exemple 1** | **Drogue de l'Exemple 1** |
| | Pₐₚₚ (10⁻⁶ cm/s) = 0,01 | Pₐₚₚ (10⁻⁶ cm/s) =0,83 |
| **Exemple 1** | F_{abs} (%) = ND | F_{abs} (%) = 71 |
| | Rendement (%) =0 | Rendement (%)=42 |
| | | Pₐₚₚ (10⁻⁶ cm/s) = 0,65 |
| **Drogue de l'Exemple 1** | | F_{abs} (%) = 67 |
| | | Rendement (%) =37 |

| | **Exemple 4** | **Drogue de l'Exemple 4** |
|---|---|---|
| | Pₐₚₚ (10⁻⁶ cm/s) = 0,33 | Pₐₚₚ (10⁻⁶ cm/s) = 0,43 |
| **Exemple 4** | F_{abs} (%) = ND | F_{abs} (%) =69 |
| | Rendement (%)=1,3 | Rendement (%)=38 |
| | | Pₐₚₚ (10⁻⁶ cm/s) = 0,21 |
| **Drogue de l'Exemple 4** | | F_{abs} (%) =46 |
| | | Rendement (%)=20 |

| | **Exemple 5** | **Drogue de l'Exemple 5** |
|---|---|---|
| | Pₐₚₚ (10⁻⁶ cm/s) = 0,26 | Pₐₚₚ (10⁻⁶ cm/s) = 2,3 |
| **Exemple 5** | F_{abs} (%) = ND | F_{abs} (%) = 86 |
| | Rendement (%)=1,2 | Rendement (%) =78 |
| | | Pₐₚₚ (10⁻⁶ cm/s) = 0,7 |
| **Drogue de l'Exemple 5** | | F_{abs} (%) = 68 |
| | | Rendement (%)=34 |

| | **Exemple 20** | **Drogue de l'Exemple 20** |
|---|---|---|
| | Pₐₚₚ (10⁻⁶ cm/s) = 0 | Pₐₚₚ (10⁻⁶ cm/s) = 0,16 |
| **Exemple 20** | F_{abs} (%) = ND | F_{abs} (%) = 16 |
| | Rendement (%) = 0,94 | Rendement (%) = 100 |
| | | Pₐₚₚ (10⁻⁶ cm/s) = 0,29 |
| **Drogue de l'Exemple 20** | | F_{abs} (%) = 25 |
| | | Rendement (%)= 91 |

| | **Exemple 21** | **Drogue de l'Exemple 21** |
|---|---|---|
| | Pₐₚₚ (10⁻⁶ cm/s) = 0 | Pₐₚₚ (10⁻⁶ cm/s) = 0,21 |
| **Exemple 21** | F_{abs} (%) = ND | F_{abs} (%) = 19 |
| | Rendement (%) = 0,83 | Rendement (%) = 100 |
| | | Pₐₚₚ (10⁻⁶ cm/s) = 0,27 |
| **Drogue de l'Exemple 21** | | F_{abs} (%) = 24 |
| | | Rendement (%)= 82 |

| | **Exemple 25** | **Drogue de l'Exemple 25** |
|---|---|---|
| **Exemple 25** | Pₐₚₚ (10⁻⁶ cm/s) = 0 | Pₐₚₚ (10⁻⁶ cm/s) = 0,22 |
| | F_{abs} (%) = ND | F_{abs} (%) = 20 |
| | Rendement (%)= 33 | Rendement (%) = 48 |
| | | Pₐₚₚ (10⁻⁶ cm/s)=0,49 |
| **Drogue de l'Exemple 25** | | F_{abs} (%)=40 |
| | | Rendement (%)= 100 |

| | | |
|---|---|---|
| *ND : non déterminé* | | |

### EXEMPLE F : Activité anti-tumorale in vivo.

L'activité anti-tumorale des composés de l'invention est évaluée dans un modèle de xénogreffe de cellules leucémiques RS4 ;11.
1.10⁷ cellules RS4 ; 11 sont greffées par voie sous-cutanée dans des souris immunodéprimées (souche SCID). 25 à 30 jours après la greffe, lorsque la masse tumorale a atteint environ 150 mm³, les souris sont traitées par voie orale par les différents composés dans 2 schémas différents (traitement quotidien pendant cinq jours par semaine durant deux semaines, ou deux traitements par semaine pendant deux semaines). La masse tumorale est mesurée 2 fois par semaine depuis le début du traitement.

Les composés de l'invention présentent des activités antitumorales, par voie orale, dans le modèle de leucémie RS4 ;11 (leucémie aiguë lymphoblastique). Les résultats obtenus montrent que les composés de l'invention sont capables d'induire une régression tumorale significative.

### EXEMPLE G: Composition pharmaceutique : Comprimés

| | |
|---|---|
| 1000 comprimés dosés à 5 mg d'un composé choisi parmi les exemples 1 à 25........... | 5 g |
| Amidon de blé......................................................................................................................... | 20 g |
| Amidon de maïs ..................................................................................................................... | 20 g |
| Lactose ................................................................................................................................... | 30 g |
| Stéarate de magnésium.......................................................................................................... | 2 g |
| Silice......................................................................................................................................... | 1 g |
| Hydroxypropylcellulose .......................................................................................................... | 2 g |

## Revendications

1. Composé phosphate de formule (I) : 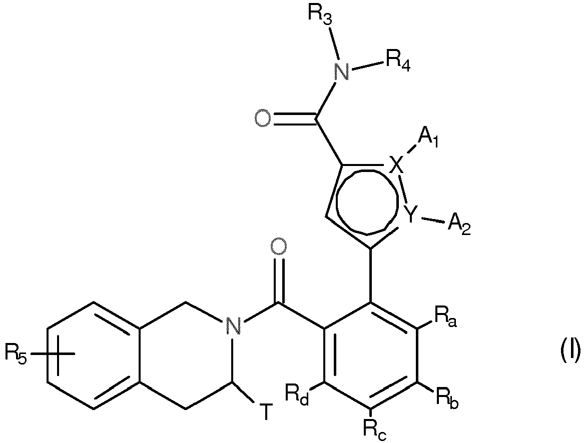 dans laquelle :
◆ X et Y représentent un atome de carbone ou un atome d'azote, étant entendu qu'ils ne peuvent représenter simultanément deux atomes de carbone ou deux atomes d'azote,
◆ A₁ et A₂ forment ensemble avec les atomes qui les portent un hétérocycle Het éventuellement substitué, aromatique ou non, constitué de 5, 6 ou 7 chaînons, et pouvant contenir, en plus de l'azote représenté par X ou par Y, un à 3 hétéroatomes choisis indépendamment parmi oxygène, soufre et azote, étant entendu que l'azote en question peut être substitué par un groupement représentant un atome d'hydrogène, un groupement alkyle (C₁-C₆) linéaire ou ramifié ou un groupement-C(O)-O-Alk dans lequel Alk est un groupement alkyle (C₁-C₆) linéaire ou ramifié, ou bien, A₁ et A₂ représentent indépendamment l'un de l'autre un atome d'hydrogène, un polyhalogénoalkyle (C₁-C₆) linéaire ou ramifié, un groupement alkyle (C₁-C₆) linéaire ou ramifié ou un cycloalkyle,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
ou bien R₁ et R₂ forment avec l'atome d'azote qui les porte un hétérocycloalkyle,
◆ R₃ représente un groupement alkyle (C₁-C₆) linéaire ou ramifié, alkényle (C₂-C₆) linéaire ou ramifié, alkynyle (C₂-C₆) linéaire ou ramifié, cycloalkyle, cycloalkyl(C₃-C₁₀)alkyle (C₁-C₆) linéaire ou ramifié, hétérocycloalkyle, aryle ou hétéroaryle, étant entendu qu'un ou plusieurs atomes de carbone des groupements précédents, ou de leurs éventuels substituants, peut(vent) être deutéré(s),
◆ R₄ représente un phényle substitué en position *para* par un groupement de formule -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃), ou -OPO(O⁻M₁⁺)(O[CH₂CH₂O]ₙCH₃), ou bien R₄ représente un groupement pyrimidin-5-yle, substitué en position *para* par un groupement de formule -OPO(O⁻M₁⁺)(O⁻M₂⁺), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5, étant entendu que le groupement phényle peut être optionnellement substitué par un ou plusieurs atomes d'halogène,
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ, R_{b}, R_{c}, et R_{d} représentent indépendamment les uns des autres R₇, un atome d'halogène, un groupement alkoxy (C₁-C₆) linéaire ou ramifié, un groupe hydroxy, un groupement polyhalogénoalkyle (C₁-C₆) linéaire ou ramifié, un groupe trifluorométhoxy, -NR₇R₇', nitro, R₇-CO-alkyl(C₀-C₆)-, R₇-CO-NH-alkyl(C₀-C₆)-, NR₇R₇'-CO-alkyl(C₀-C₆)-, NR₇R₇'-CO-alkyl(C₀-C₆)-O-, R₇-SO₂-NH-alkyl(C₀-C₆)-, R₇-NH-CO-NH-alkyl(C₀-C₆)-, R₇-O-CO-NH-alkyl(C₀-C₆)-, un groupement hétérocycloalkyle, ou bien les substituants de l'un des couples (Rₐ,R_{b}), (R_{b},R_{c}) ou (R_{c},R_{d}) forment ensemble avec les atomes de carbone qui les portent un cycle constitué de 5 à 7 chaînons, pouvant contenir de un à 2 hétéroatomes choisis parmi oxygène et soufre, étant aussi entendu qu'un ou plusieurs atomes de carbone du cycle précédemment défini peut(vent) être deutéré(s) ou substitués par un à 3 groupements choisis parmi halogène ou alkyle (C₁-C₆) linéaire ou ramifié,
◆ R₇ et R₇' représentent indépendamment l'un de l'autre un hydrogène, un alkyle (C₁-C₆) linéaire ou ramifié, un alkényle (C₂-C₆) linéaire ou ramifié, un alkynyle (C₂-C₆) linéaire ou ramifié, un aryle ou un hétéroaryle, ou bien R₇ et R₇' forment ensemble avec l'atome d'azote qui les porte un hétérocycle constitué de 5 à 7 chaînons,
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié éventuellement substitué, spiro (C₃-C₆), alkoxy (C₁-C₆) linéaire ou ramifié éventuellement substitué, (C₁-C₆)alkyl-S-, hydroxy, oxo (ou *N*-oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle éventuellement substitué, hétéroaryle, aryloxy, arylthio, cycloalkyle, hétérocycloalkyle éventuellement substitué par un ou plusieurs atomes d'halogène ou groupements alkyles, étant entendu que R' et R" représentent, indépendamment l'un de l'autre, un atome d'hydrogène ou un groupement alkyle (C₁-C₆) linéaire ou ramifié éventuellement substitué,
le groupement Het défini dans la formule (I) pouvant être substitué par un à trois groupements choisis parmi alkyle (C₁-C₆) linéaire ou ramifié, hydroxy, alkoxy (C₁-C₆) linéaire ou ramifié, NR₁'R₁", ou halogène, étant entendu que R₁' et R₁" ont les mêmes définitions que les groupes R' et R" mentionnés précédemment,
ses énantiomères et diastéréoisomères, ainsi que ses sels d'addition à un acide ou à une base pharmaceutiquement acceptable.

2. Composé de formule (I) selon la revendication 1 dans lequel R₄ représente un phényle substitué en position *para* par un groupement de formule -OPO(OM)(OM'), -OPO(OM)(O⁻M₁⁺), -OPO(O⁻M₁⁺)(O⁻M2⁺), -OPO(O⁻)(O⁻)M₃²⁺, -OPO(OM)(O[CH₂CH₂O]ₙCH₃), ou -OPO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5, étant entendu que le groupement phényle peut être optionnellement substitué par un ou plusieurs atomes d'halogène.

3. Composé de formule (I) selon la revendication 1 dans lequel R₄ représente un phényle substitué en position *para* par un groupement de formule-OPO(O⁻Na⁺)(O⁻Na⁺).

4. Composé de formule (I) selon l'une des revendications 1 à 3 dans lequel X représente un atome de carbone et Y représente un atome d'azote.

5. Composé de formule (I) selon l'une des revendications 1 à 3 dans lequel le groupe : 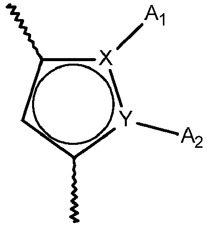 représente une 5,6,7,8-tétrahydroindolizine, une indolizine ou un pyrrole diméthylé.

6. Composé de formule (I) selon l'une des revendications 1 à 5 dans lequel T représente un groupement méthyle, (morpholin-4-yl)méthyle ou 3-(morpholin-4-yl)propyle.

7. Composé de formule (I) selon l'une des revendications 1 à 6 dans lequel Rₐ et R_{d} représentent chacun un atome d'hydrogène et (R_{b},R_{c}) forment ensemble avec les atomes de carbone qui les portent un groupe 1,3-dioxolane, un groupe 1,4-dioxane, ou bien Rₐ, R_{c} et R_{d} représentent chacun un atome d'hydrogène et R_{b} représente un hydrogène ou un halogène.

8. Composé de formule (I) selon l'une des revendications 1 à 6 dans lequel Rₐ et R_{d} représentent chacun un atome d'hydrogène, R_{b} représente un atome d'halogène et R_{c} un groupement méthoxy.

9. Composé de formule (I) selon l'une des revendications 1 à 6 dans lequel Rₐ, R_{b} et R_{d} représentent avantageusement chacun un atome d'hydrogène et R_{c} représente un groupement NR₇R₇'-CO-alkyl(C₀-C₆)-O-.

10. Composé de formule (I) selon l'une des revendications 1 à 9 dans lequel R₃ représente avantageusement un groupement choisi parmi phényle, 1*H*-indole, 1*H-*pyrrolo[2,3-*b*]pyridine, pyridine, 1*H*-pyrazole, 1*H*-pyrrole, et 2,3-dihydro-1*H*-pyrrolo[2,3-*b*]pyridine, ces groupements comportant éventuellement un ou plusieurs substituants choisis parmi alkyle (C₁-C₆) linéaire ou ramifié, cyano, ou trideutériométhyle.

11. Composé de formule (I) selon la revendication 1 choisi dans la liste suivante :
- 4-[{[3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizin-1-yl]carbonyl}(phényl)amino]phényl phosphate de disodium,
- 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(pyridin-4-yl)amino]phényl phosphate de disodium,
- 4-({[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}[1-(trideutériométhyl)-1*H*-pyrazol-4-yl]amino)phényl phosphate de disodium,
- 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(5-cyano-1,2-diméthyl-1*H*-pyrrol-3-yl)amino]phényl phosphate de disodium,
- 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(5-cyano-1-méthyl-1*H-*pyrrol-3-yl)amino]phényl phosphate de disodium,
- 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(1-méthyl-1*H*-pyrazol-4-yl)amino]phényl phosphate de disodium,
- 4-[(5-cyano-1,2-diméthyl-1*H*-pyrrol-3-yl){[5-(5-fluoro-2-{[(3*S*)-3-(morpholin-4-ylméthyl)-3,4-dihydroisoquinolin-2(1*H*)-yl]carbonyl}phényl)-1,2-diméthyl-1*H-*pyrrol-3-yl]carbonyl}amino]phényl phosphate de disodium,
- 4-[{[5-(5-fluoro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(1-méthyl-1*H-*pyrazol-4-yl)amino]phényl phosphate de disodium,
leurs énantiomères et diastéréoisomères, ainsi que leurs sels d'addition à un acide ou à une base pharmaceutiquement acceptable.

12. Composé de formule (I) selon la revendication 11 qui est le 4-[{[3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizin-1-yl]carbonyl}(phényl)amino]phényl phosphate de disodium.

13. Composé de formule (I) selon la revendication 11 qui est le 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(pyridin-4-yl)amino]phényl phosphate de disodium.

14. Composé de formule (I) selon la revendication 11 qui est le 4-({[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}[1-(trideutériométhyl)-1*H-*pyrazol-4-yl]amino)phényl phosphate de disodium.

15. Composé de formule (I) selon la revendication 11 qui est le 4-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(5-cyano-1,2-diméthyl-1*H*-pyrrol-3-yl}amino]phényl phosphate de disodium.

16. Composé de formule (I) selon la revendication 11 qui est le 4-[{[5-(5-chloro-2-{[(3*S*)-3-(morpholin-4-ylméthy1)-3,4-dihydroisoquinolin-2(1*H*)-yl]carbonyl}phényl)-1,2-diméthyl-1*H*-pyrrol-3-yl]carbonyl}(5-cyano-1-méthyl-1*H*-pyrrol-3-yl}amino]phényl phosphate de disodium.

17. Composé de formule (I) selon la revendication 11 qui est le 4-[{[5-(5-chloro-2-{[(3*S*)-3-(morpholin-4-ylméthyl}-3,4-dihydroisoquinolin-2(1*H*)-yl]carbonyl}phényl)-1,2-diméthyl-1*H*-pyrrol-3-yl]carbonyl}(1-méthyl-1*H*-pyrazol-4-yl)amino]phényl phosphate de disodium.

18. Composé de formule (I) selon la revendication 11 qui est le 4-[(5-cyano-1,2-diméthyl-1*H*-pyrrol-3-yl){[5-(5-fluoro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}amino]phényl phosphate de disodium.

19. Composé de formule (I) selon la revendication 11 qui est le 4-[[5-(5-fluoro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(1-méthyl-1*H*-pyrazol-4-yl)amino]phényl phosphate de disodium.

20. Procédé de préparation des composés de formule (I) selon la revendication **1 caractérisé en ce que** l'on utilise comme produit de départ le composé de formule (II) : 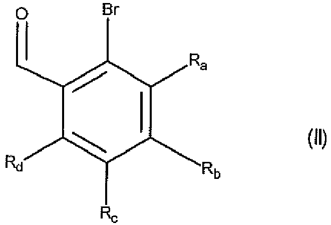 dans laquelle Rₐ, R_{b}, R_{c} et R_{d} sont tels que définis dans la revendication 1,
composé de formule (II) qui est soumis à une réaction de Heck, en milieu aqueux ou organique, en présence d'un catalyseur au palladium, d'une base, d'une phosphine et du composé de formule (III) : 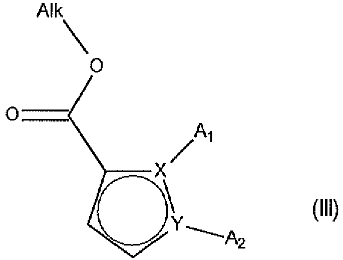 dans laquelle les groupements A₁, A₂, X et Y sont tels que définis dans la revendication 1 et Alk représente un alkyle (C₁-C₆) linéaire ou ramifié,
pour obtenir le composé de formule (IV) : 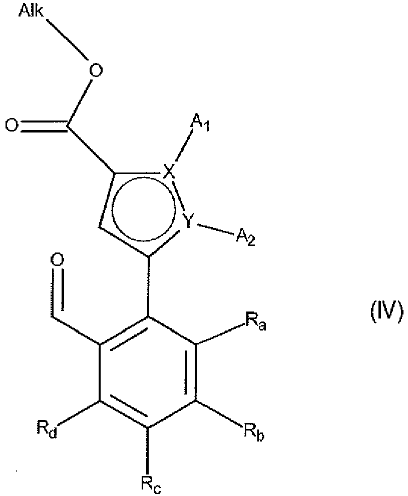 dans laquelle A₁, A₂, X, Y, Rₐ, R_{b}, R_{c} et R_{d} sont tels que définis dans la revendication 1 et Alk est tel que défini précédemment,
composé de formule (IV) dont la fonction aldéhyde est oxydée en acide carboxylique pour former le composé de formule (V) : 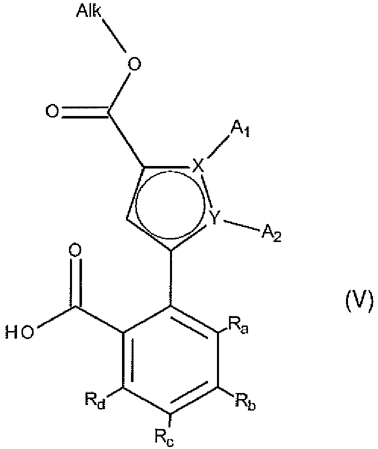 dans laquelle A₁, A₂, X, Y, Rₐ, R_{b}, R_{c} et R_{d} sont tels que définis dans la revendication 1 et Alk est tel que défini précédemment,
composé de formule (V) qui subit ensuite un couplage peptidique avec un composé de formule (VI) : 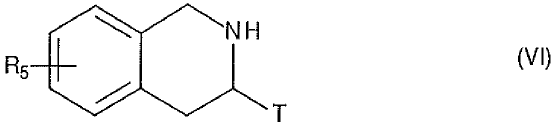 dans laquelle T et R₅ sont tels que définis dans la revendication 1,
pour conduire au composé de formule (VII) : 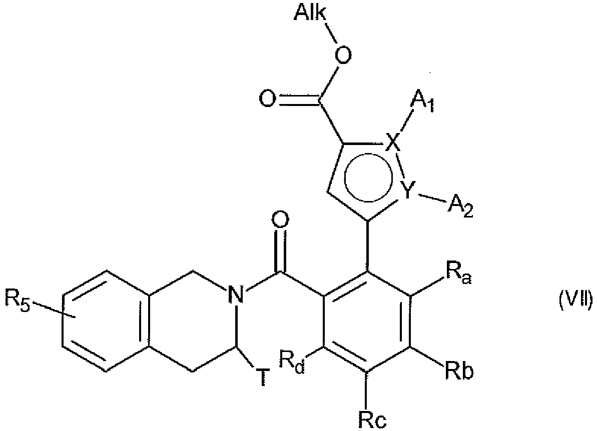 dans laquelle A₁, A₂, X, Y, Rₐ, R_{b}, R_{c}, Rd, T et R₅ sont tels que définis dans la revendication 1 et Alk est tel que défini précédemment,
composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la revendication 1, avant d'être soumis à l'action d'un dérivé pyrophosphate, phosphonate ou phosphoryle dans des conditions basiques, le composé ainsi obtenu pouvant être optionnellement hydrolysé ou hydrogénolysé, pour conduire au composé de formule (I), composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

21. Composition pharmaceutique contenant un composé de formule (I) selon l'une quelconque des revendications 1 à 19 ou un de ses sels d'addition avec un acide ou une base pharmaceutiquement acceptable en combinaison avec un ou plusieurs excipients pharmaceutiquement acceptables.

22. Composition pharmaceutique selon la revendication 21 pour son utilisation en tant que prodrogue d'un agent pro-apoptotique.

23. Composition pharmaceutique selon la revendication 21 pour son utilisation dans le traitement des cancers, des maladies auto-immunes et du système immunitaire.

24. Composition pharmaceutique selon la revendication 23 pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

25. Utilisation d'une composition pharmaceutique selon la revendication 21 pour la fabrication d'un médicament utile en tant qu'agent pro-apoptotique.

26. Utilisation d'une composition pharmaceutique selon la revendication 21 pour la fabrication d'un médicament destiné au traitement des cancers, des maladies auto-immunes et du système immunitaire.

27. Utilisation d'une composition pharmaceutique selon la revendication 26 pour la fabrication d'un médicament destiné au traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

28. Composé de formule (1) selon l'une des revendications 1 à 19, ou un de ses sels d'addition avec un acide ou une base pharmaceutiquement acceptable, pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

29. Utilisation d'un composé de formule (I) selon l'une des revendications 1 à 19, ou un de ses sels d'addition avec un acide ou une base pharmaceutiquement acceptable, pour la fabrication d'un médicament destiné au traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

30. Combinaison d'un composé de formule (I) selon l'une quelconque des revendications 1 à 19 avec un agent anticancéreux choisi parmi les agents génotoxiques, les poisons mitotiques, les anti-métabolites, les inhibiteurs du protéasome, les inhibiteurs de kinases ou les anticorps.

31. Composition pharmaceutique contenant une combinaison selon la revendication 30 en combinaison avec un ou plusieurs excipients pharmaceutiquement acceptables.

32. Combinaision selon la revendication 30 pour son utilisation dans le traitement des cancers.

33. Utilisation d'une combinaison selon la revendication 30 pour la fabrication d'un médicament utile dans le traitement des cancers.

34. Composé de formule (I) selon l'une quelconque des revendications 1 à 19 pour son utilisation en association avec une radiothérapie dans le traitement des cancers.

## Patentansprüche

1. Phosphatverbindung der Formel (I): 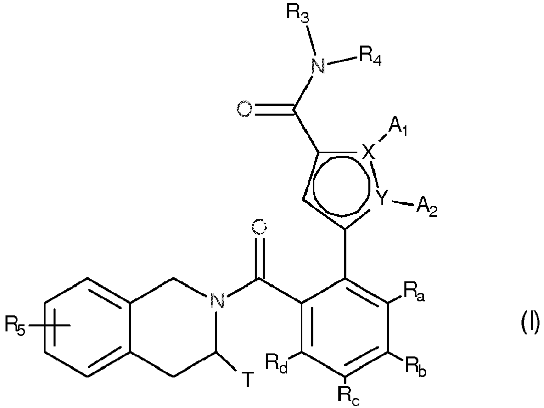 in der:
◆ X und Y ein Kohlenstoffatom oder ein Stickstoffatom bedeuten, wobei es sich versteht, dass sie nicht gleichzeitig zwei Kohlenstoffatome oder zwei Stickstoffatome bedeuten können,
◆ A₁ und A₂ gemeinsam mit den sie tragenden Atomen einen Heterocyclus Het bilden, der gegebenenfalls substituiert, aromatisch oder nicht aromatisch sein kann, aus 5, 6 oder 7 Kettengliedern gebildet ist und zusätzlich zu dem durch X oder durch Y repräsentierten Stickstoff 1 bis 3 Heteroatome enthalten kann, unabhängig voneinander ausgewählt aus Sauerstoff, Schwefel und Stickstoff, wobei es sich versteht, dass der in Rede stehende Stickstoff durch eine Gruppe substituiert sein kann, die ein Wasserstoffatom, eine geradkettige oder verzweigte (C₁-C₆)-Alkylgruppe oder eine Gruppe -C(O)-O-Alk, worin Alk eine geradkettige oder verzweigte (C₁-C₆)-Alkylgruppe bedeutet, sein kann, oder A₁ und A₂ unabhängig voneinander ein Wasserstoffatom, eine geradkettige oder verzweigte (C₁-C₆)-Polyhalogenalkylgruppe oder eine geradkettige oder verzweigte (C₁-C₆)-Alkylgruppe oder eine Cycloalkylgruppe bedeuten können,
◆ T ein Wasserstoffatom, eine gegebenenfalls durch ein bis drei Halogenatome substituierte (C₁-C₆)-Alkylgruppe, eine (C₁-C₄)-Alkyl-NR₁R₂-gruppe oder eine (C₁-C₄)-Alkyl-OR₆-gruppe bedeutet,
◆ R₁ und R₂ unabhängig voneinander ein Wasserstoffatom oder eine geradkettige oder verzweigte (C₁-C₆)-Alkylgruppe bedeuten oder
R₁ und R₂ zusammen mit dem sie tragenden Stickstoffatom eine Heterocycloalkylgruppe bilden,
◆ R₃ eine geradkettige oder verzweigte (C₁-C₆)-Alkylgruppe, geradkettige oder verzweigte (C₂-C₆)-Alkenylgruppe, geradkettige oder verzweigte (C₂-C₆)-Alkinylgruppe, Cycloalkylgruppe, geradkettige oder verzweigte (C₃-C₁₀)-Cycloalkyl-(C₁-C₆)-alkylgruppe, Heterocycloalkylgruppe, Arylgruppe oder Heteroarylgruppe bedeutet, wobei es sich versteht, dass ein oder mehrere Kohlenstoffatome der vorstehenden Gruppen oder ihre eventuellen Substituenten deuteriert sein können,
◆ R₄ eine in der *para*-Stellung durch eine Gruppe der Formel -OPO(OM)(OM'),-OPO(OM)(O⁻M₁⁺), -OPO(O⁻M₁⁺)(O⁻M₂⁺), -OPO(O⁻)(O⁻)M₃²⁺, OPO(OM)(O[CH₂CH₂O]ₙCH₃) oder -OPO(O⁻M₁⁺)(O[CH₂CH₂O]ₙCH₃ substituierten Phenylrest oder R₄ eine in der *para*-Stellung durch eine Gruppe der Formel -OPO(O⁻M₁⁺)(O⁻M₂⁺) substituierte Pyrimidin-5-yl-gruppe bedeutet, worin M und M' unabhängig voneinander ein Wasserstoffatom, eine geradkettige oder verzweigte (C₁-C₆)-Alkylgruppe, eine geradkettige oder verzweigte (C₂-C₆)-Alkenylgruppe, eine geradkettige oder verzweigte (C₂-C₆)-Alkinylgruppe, eine Cycloalkylgruppe oder eine Heterocycloalkylgruppe, welche beiden Gruppen 5 bis 6 Kettenglieder aufweisen, bedeuten, während M₁⁺ und M₂⁺ unabhängig voneinander ein pharmazeutisch annehmbares einwertiges Kation, M₃²⁺ ein pharmazeutisch annehmbares zweiwertiges Kation und n eine ganze Zahl mit einem Wert von 1 bis 5 bedeuten, wobei es sich versteht, dass die Phenylgruppe gegebenenfalls durch ein oder mehrere Halogenatome substituiert sein kann,
◆ R₅ ein Wasserstoffatom, ein Halogenatom, eine geradkettige oder verzweigte (C₁-C₆)-Alkylgruppe oder eine geradkettige oder verzweigte (C₁-C₆)-Alkoxygruppe bedeutet,
◆ R₆ ein Wasserstoffatom oder eine geradkettige oder verzweigte (C₁-C₆)-Alkylgruppe bedeutet,
◆ Rₐ R_{b}, R_{c} und R_{d} unabhängig voneinander R₇, ein Halogenatom, eine geradkettige oder verzweigte (C₁-C₆)-Alkoxygruppe, eine Hydroxygruppe, eine geradkettige oder verzweigte (C₁-C₆)-Polyhalogenalkylgruppe, eine Trifluormethoxygruppe, -NR₇R₇', Nitro, R₇-CO-(C₀-C₆)-Alkyl-, R₇-CO-NH-(C₀-C₆)-Alkyl-, NR₇R₇'-CO-(C₀-C₆)-Alkyl-, NR₇R₇'-CO-(C₀-C₆)-Alkyl-O-, R₇-SO₂-NH-(C₀-C₆)-Alkyl-, R₇-NH-CO-NH-(C₀-C₆)-Alkyl-, R₇-O-CO-NH-(C₀-C₆)-Alkyl-, oder eine Heterocycloalkylgruppe bedeuten, oder die Substituenten der Paare (Rₐ,R_{b}), (R_{b},R_{c}) oder (R_{c},R_{d}) zusammen mit den sie tragenden Kohlenstoffatomen einen Ring mit 5 bis 7 Kettengliedern bilden, der ein bis 2 Heteroatome ausgewählt aus Sauerstoff und Schwefel enthalten kann, wobei es sich weiter versteht, dass eines oder mehrere Kohlstoffatome des vorstehend definierten Ring deuteriert oder durch eine bis 3 Gruppen ausgewählt aus Halogen oder geradkettigem oder verzweigtem (C₁-C₆)-Alkyl substituiert sein können,
◆ R₇ und R₇' unabhängig voneinander Wasserstoff, geradkettiges oder verzweigtes (C₁-C₆)-Alkyl, geradkettiges oder verzweigtes (C₂-C₆)-Alkenyl, geradkettiges oder verzweigtes (C₂-C₆)-Alkinyl, Aryl oder Heteroaryl bedeuten, oder R₇ und R₇' gemeinsam mit dem sie tragenden Stickstoffatom einen Heterocyclus mit 5 bis 7 Kettengliedern bilden,
mit der Maßgabe, dass man:
- unter "Aryl" eine Phenyl-, Naphthyl-, Biphenyl- oder Indenylgruppe versteht,
- unter "Heteroaryl" jede mono- oder bicyclische aus 5 bis 10 Kettengliedern gebildete Gruppe versteht, die mindestens einen aromatischen Teil aufweist und 1 bis 4 Heteroatome ausgewählt aus Sauerstoff, Schwefel oder Stickstoff (einschließlich quaternäre Stickstoffatome) enthält,
- unter "Cycloalkyl" jede nicht-aromatische, mono- oder bicyclische carbocyclische Gruppe, die 3 bis 10 Kettenglieder aufweist, versteht,
- unter "Heterocycloalkyl" jede nicht-aromatische, mono- oder bicyclische kondensierte oder spiro-gebundene Gruppe mit 3 bis 10 Kettengliedern, die 1 bis 3 Heteroatome ausgewählt aus Sauerstoff, Schwefel, SO, SO₂ oder Stickstoff enthält, versteht,
wobei die in dieser Weise definierten Aryl-, Heteroaryl-, Cycloalkyl- und Heterocycloalkylgruppen und die Alkyl-, Alkenyl-, Alkinyl- und Alkoxygruppen substituiert sein können durch 1 bis 3 Gruppen ausgewählt aus geradkettigem oder verzweigtem, gegebenenfalls substituiertem (C₁-C₆)-Alkyl, (C₃-C₆)-spiro, geradkettigem oder verzweigtem, gegebenenfalls substituiertem (C₁-C₆)-Alkoxy, (C₁-C₆)-Alkyl-S-, Hydroxy, Oxo (oder gegebenenfalls *N*-Oxid), Nitro, Cyano, -COOR', -OCOR', NR'R", geradkettigem oder verzweigtem (C₁-C₆)-Polyhalogenalkyl, Trifluormethoxy, (C₁-C₆)-Alkylsulfonyl, Halogen, gegebenenfalls substituiertem Aryl, Heteroaryl, Aryloxy, Arylthio, Cycloalkyl und gegebenenfalls durch ein oder mehrere Halogenatome oder Alkylgruppen substituiertem Heterocycloalkyl, wobei es sich versteht, dass R' und R" unabhängig voneinander ein Wasserstoffatom oder eine geradkettige oder verzweigte, gegebenenfalls substituierte (C₁-C₆)-Alkylgruppe bedeuten,
wobei die für die Formel (I) definierte Gruppe Het substituiert sein kann durch ein bis drei Gruppen ausgewählt aus geradkettigem oder verzweigtem (C₁-C₆)-Alkyl, Hydroxy, geradkettigem oder verzweigtem (C₁-C₆)-Alkoxy, NR₁'R₁" oder Halogen, wobei es sich versteht, dass R₁' und R₁" die gleichen Bedeutungen besitzen wie die oben erwähnten Gruppen R' und R",
deren Enantiomere und Diastereoisomere sowie deren Additionssalze mit einer pharmazeutisch annehmbaren Säure oder Base.

2. Verbindung der Formel (I) nach Anspruch 1, worin R₄ eine Phenylgruppe bedeutet, die in der *para*-Stellung durch eine Gruppe der Formel -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃) oder -OPO(O⁻M₁⁺)(O[CH₂CH₂O]ₙCH₃ substituiert ist, worin M und M' unabhängig voneinander ein Wasserstoffatom, eine geradkettige oder verzweigte (C₁-C₆)-Alkylgruppe, eine geradkettige oder verzweigte (C₂-C₆)-Alkenylgruppe, eine geradkettige oder verzweigte (C₂-C₆)-Alkinylgruppe, eine Cycloalkylgruppe oder eine Heterocycloalkylgruppe, welche beiden Gruppen aus 5 bis 6 Kettengliedern gebildet sind, bedeuten, während M₁⁺ und M₂⁺ unabhängig voneinander ein pharmazeutisch annehmbares einwertiges Kation, M₃²⁺ ein pharmazeutisch annehmbares zweiwertiges Kation und n eine ganze Zahl mit einem Wert zwischen 1 und 5 bedeuten, wobei es sich versteht, dass die Phenylgruppe gegebenenfalls durch ein oder mehrere Halogenatome substituiert sein kann.

3. Verbindung der Formel (I) nach Anspruch 1, worin R₄ eine in der *para*-Stellung durch eine Gruppe der Formel -OPO(O⁻Na⁺)(O⁻Na⁺) substituierte Phenylgruppe darstellt.

4. Verbindung der Formel (I) nach einem der Ansprüche 1 bis 3, worin X ein Kohlenstoffatom und Y ein Stickstoffatom bedeuten.

5. Verbindung der Formel (I) nach einem der Ansprüche 1 bis 3, worin die Gruppe: 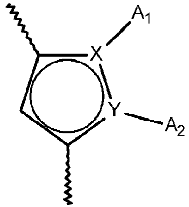 eine 5,6,7,8-Tetrahydroindolizin-, Indolizin- oder Dimethylpyrrolgruppe bedeutet.

6. Verbindung der Formel (I) nach einem der Ansprüche 1 bis 5, worin T eine Methyl-, (Morpholin-4-yl)-methyl- oder 3-(Morpholin-4-yl)-propyl-gruppe bedeutet.

7. Verbindung der Formel (I) nach einem der Ansprüche 1 bis 6, worin Rₐ und R_{d} jeweils ein Wasserstoffatom bedeuten und (R_{b},R_{c}) gemeinsam mit dem sie tragenden Kohlenstoffatom eine 1,3-Dioxolan- oder 1,4-Dioxangruppe bilden oder Rₐ, R_{c} und R_{d} jeweils ein Wasserstoffatom und R_{b} ein Wasserstoffatom oder ein Halogenatom bedeuten.

8. Verbindung der Formel (I) nach einem der Ansprüche 1 bis 6, worin Rₐ und R_{d} jeweils ein Wasserstoffatom, R_{b} ein Halogenatom und R_{c} eine Methoxygruppe bedeuten.

9. Verbindung der Formel (I) nach einem der Ansprüche 1 bis 6, worin Rₐ, R_{b} und R_{d} mit Vorteil jeweils ein Wasserstoffatom und R_{c} eine Gruppe NR₇R₇'-CO-(C₀-C₆)-Alkyl-O- bedeuten.

10. Verbindung der Formel (I) nach einem der Ansprüche 1 bis 9, worin R₃ mit Vorteil eine Gruppe ausgewählt aus Phenyl, 1*H*-Indol, 1*H*-Pyrrolo[2,3-*b*]pyridin, Pyridin, 1*H*-Pyrazol, 1*H*-Pyrrol und 2,3-Dihydro-1*H*-pyrrolo[2,3-*b*]pyridin bedeutet, wobei diese Gruppen gegebenenfalls ein oder mehrere Substituenten ausgewählt aus geradkettigem oder verzweigtem (C₁-C₆)-Alkyl, Cyano oder Trideuteromethyl aufweisen.

11. Verbindung der Formel (I) nach Anspruch 1, ausgewählt aus der folgenden Liste:
- 4-[{[3-(6-{[(3*S*)-3-(Morpholin-4-ylmethyl)-3,4-dihydroisochinolin-2(1*H*)-yl]-carbonyl}-1,3-benzodioxol-5-yl)-5,6,7,8-tetrahydroindolizin-1-yl]-carbonyl}(phenyl)-amino]-phenyl-dinatriumphosphat,
- 4-[{[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(pyridin-4-yl)-amino]-phenyl-dinatriumphosphat,
- 4-({[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}[1-(trideuteromethyl)-1*H*-pyrazol-4-yl]-amino)-phenyl-dinatriumphosphat,
- 4-[{[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(5-cyano-1,2-dimethyl-1*H*-pyrrol-3-yl)-amino]-phenyl-dinatriumphosphat,
- 4-[{[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(5-cyano-1-methyl-1*H* pyrrol-3-yl}-amino]-phenyl-dinatriumphosphat,
- 4-[{[5-(5-Chlor-2-{[(3*S*)-3-(morpholin-4-ylmethyl)-3,4-dihydroisochinolin-2(1*H*)-yl]-carbonyl}-phenyl}-1,2-dimethyl-1*H*-pyrrol-3-yl]-carbonyl}(1-methyl-1*H*-pyrazol-4-yl)-amino]-phenyl-dinatriumphosphat,
- 4-[((5-Cyano-1,2-dimethyl-1*H* pyrrol-3-yl}-{[5-(5-fluor-2-{[{3*S*)-3-(morpholin-4-ylmethyl)-3,4-dihydroisochinolin-2(1*H*)-yl]-carbonyl}-phenyl)-1,2-dimethyl-1*H* pyrrol-3-yl]-carbonyl}-amino]-phenyl-dinatriumphosphat,
- 4-[{[5-(5-Fluor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(1-methyl-1*H*-pyrazol-4-yl)-amino]-phenyl-dinatriumphosphat,
deren Enantiomere und Diastereoisomere sowie deren Additionssalze mit einer pharmazeutisch annehmbaren Säure oder Base.

12. Verbindung der Formel (I) nach Anspruch 11, nämlich 4-[{[3-(6-{](3*S*)-3-(morpholin-4-ylmethyl)-3,4-dihydroisochinolin-2(1*H*)-yl]-carbonyl}-1,3-benzodioxol-5-yl)-5,6,7,8-tetrahydroindolizin-1-yl]-carbonyl}(phenyl)-amino]-phenyl-dinatriumphosphat.

13. Verbindung der Formel (I) nach Anspruch 11, nämlich 4-[{[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(pyridin-4-yl)-amino]-phenyl-dinatriumphosphat.

14. Verbindung der Formel (I) nach Anspruch 11, nämlich 4-({[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}[1-(trideuteromethyl)-1*H*-pyrazol-4-yl]-amino)-phenyl-dinatriumphosphat.

15. Verbindung der Formel (I) nach Anspruch 11, nämlich 4-[{[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(5-cyano-1,2-dimethyl-1*H*-pyrrol-3-yl)-amino]-phenyl-dinatriumphosphat.

16. Verbindung der Formel (I) nach Anspruch 11, nämlich 4-[{[5-(5-Chlor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(5-cyano-1-methyl-1*H*-pyrrol-3-yl}-amino]-phenyl-dinatriumphosphat.

17. Verbindung der Formel (I) nach Anspruch 11, nämlich 4-[{[5-(5-Chlor-2-{[(3*S*)-3-(morpholin-4-ylmethyl)-3,4-dihydroisochinolin-2(*1*H)-yl]-carbonyl}-phenyl}-1,2-dimethyl-1*H*-pyrrol-3-yl]-carbonyl}-(1-methyl-1*H*-pyrazol-4-yl)-amino]-phenyl-dinatriumphosphat,

18. Verbindung der Formel (I) nach Anspruch 11, nämlich 4-[(5-Cyano-1,2-dimethyl-1*H-*pyrrol-3-yl}-{[5-(5-fluor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}-amino]-phenyl-dinatriumphosphat.

19. Verbindung der Formel (I) nach Anspruch 11, nämlich 4-[{[5-(5-Fluor-2-{[(3*S*)-3-(morpholin-4-ylmethyl)-3,4-dihydroisochinolin-2(1*H*)-yl]-carbonyl}-phenyl)-1,2-dimethyl-1*H*-pyrrol-3-yl]-carbonyl}(1-methyl-1*H*-pyrazol-4-yl)-amino]-phenyldinatriumphosphat.

20. Verfahren zur Herstellung der Verbindungen der Formel (I) nach Anspruch 1, **dadurch gekennzeichnet, dass** man als Ausgangsprodukt die Verbindung der Formel (II): 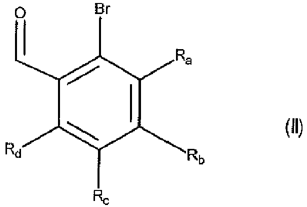 worin Rₐ, R_{b}, R_{c} und R_{d} die in Anspruch 1 angegebenen Bedeutungen besitzen, verwendet,
welche Verbindung der Formel (II) einer Heck-Reaktion unterworfen wird in wässrigem oder organischem Medium, in Gegenwart eines Palladiumkatalysators, einer Base, eines Phosphins und einer Verbindung der Formel (III): 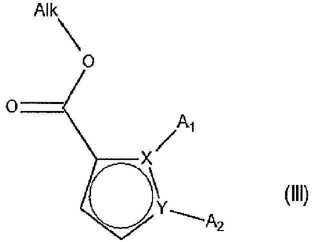 in der die Gruppen A₁, A₂, X und Y die in Anspruch 1 angegebenen Bedeutungen besitzen und Alk eine geradkettige oder verzweigte (C₁-C₆)-Alkylgruppe bedeutet, zur Bildung der Verbindung der Formel (IV): 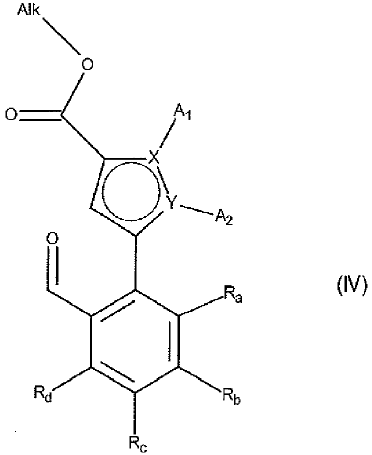 in der A₁, A₂, X, Y, Rₐ, R_{b}, R_{c} und R_{d} die in Anspruch 1 angegebenen Bedeutungen besitzen und Alk die oben angegebene Bedeutung aufweist,
die Aldehydfunktion der Verbindung der Formel (IV) zu der Carbonsäure oxidiert wird unter Bildung der Verbindung der Formel (V): 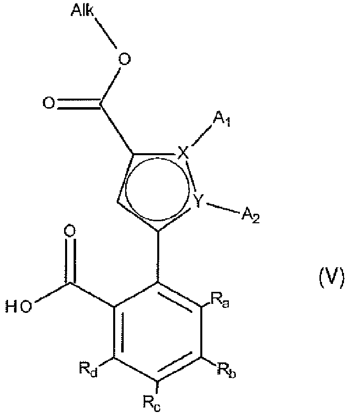 in der A₁, A₂, X, Y, Rₐ, R_{b}, R_{c} und R_{d} die in Anspruch 1 angegebenen Bedeutungen besitzen und Alk die oben angegebene Bedeutung aufweist,
welche Verbindung der Formel (V) einer Peptidkupplung unterzogen wird mit einer Verbindung der Formel (VI): 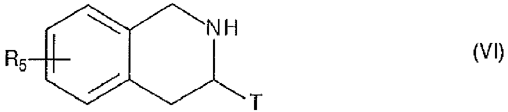 in der T und R₅ die in Anspruch 1 angegebenen Bedeutungen besitzen,
zur Bildung der Verbindung der Formel (VII): 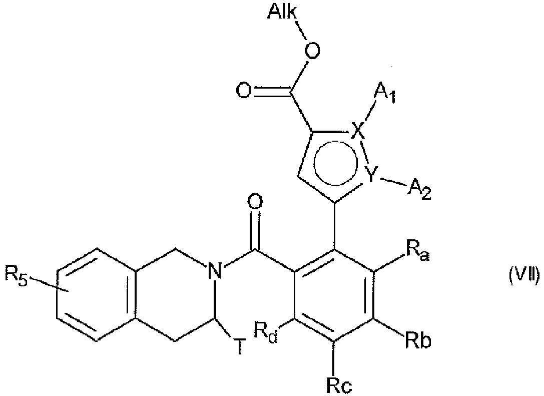 in der A₁, A₂, X, Y, Rₐ, R_{b}, R_{c}, R_{d}, T und R₅ die in Anspruch 1 angegebenen Bedeutungen besitzen und Alk die oben angegebene Bedeutung aufweist,
die Esterfunktion der Verbindung der Formel (VII) hydrolysiert wird zur Bildung der entsprechenden Carbonsäure oder des entsprechenden Carbonsäureesters, die bzw. der in ein Säurederivat, wie das entsprechende Acylchlorid oder Anhydrid umgewandelt werden kann, bevor sie mit einem Amin NHR₃R₄, worin R₃ und R₄ die in Anspruch 1 angegebenen Bedeutungen besitzen, gekuppelt wird, bevor sie der Einwirkung eines Pyrophosphat-, Phosphonat- oder Phosphoryl-Derivats unter basischen Bedingungen unterworfen wird, wobei die in dieser Weise erhaltene Verbindung gegebenenfalls hydrolysiert oder hydriert werden kann zur Bildung der Verbindung der Formel (I), welche Verbindung der Formel (I) mit Hilfe einer klassischen Trennmethode gereinigt werden kann, welche man gewünschtenfalls in ihre Additionssalze mit einer pharmazeutisch annehmbaren Säure oder Base umwandelt und die man gegebenenfalls mit Hilfe einer klassischen Trennmethode in ihre Isomeren auftrennt,
wobei es sich versteht, das bestimmte Gruppen (Hydroxy, Amino...) der Synthesereagenzien oder -zwischenprodukte zu jedem angemessen erscheinenden Augenblick des oben beschriebenen Verfahrens zum Zwecke der Synthese geschützt und dann wieder von ihren Schutzgruppen befreit werden können.

21. Pharmazeutische Zubereitung, enthaltend eine Verbindung der Formel (I) nach einem der Ansprüche 1 bis 19 oder eines ihrer Additionssalze mit einer pharmazeutisch annehmbaren Säure oder Base in Kombination mit einem oder mehreren pharmazeutisch annehmbaren Hilfsstoffen.

22. Pharmazeutische Zubereitung nach Anspruch 21 zur Verwendung als Vorläuferwirkstoff eines pro-apoptotischen Mittels.

23. Pharmazeutische Zubereitung nach Anspruch 21 zur Verwendung bei der Behandlung von Krebs, Autoimmunkrankheiten und Erkrankungen des Immunsystems.

24. Pharmazeutische Zubereitung nach Anspruch 23 zur Verwendung bei der Behandlung von Blasenkrebs, Gehirnkrebs, Brustkrebs, Uteruskrebs, chronisch-lymphatischen Leukämien, Darmkrebs, Speiseröhrenkrebs, Leberkrebs, lymphoblastischen Leukämien, Nicht-Hodgkin-Lymphomen, Melanomen, malignen Hämopathien, Myelomen, Ovarialkrebs, nicht-kleinzelligem Lungenkrebs, Prostatakrebs und kleinzelligem Lungenkrebs.

25. Verwendung einer pharmazeutischen Zubereitung nach Anspruch 21 zur Herstellung eines Arzneimittels, das nützlich ist als pro-apoptotisches Mittel.

26. Verwendung einer pharmazeutischen Zubereitung nach Anspruch 21 zur Herstellung eines Arzneimittels, das bestimmt ist zur Behandlung von Krebst, Autoimmunerkrankungen und Erkrankungen des Immunsystems.

27. Verwendung einer pharmazeutischen Zubereitung nach Anspruch 26 zur Herstellung eines Arzneimittels zur Behandlung von Blasenkrebs, Gehirnkrebs, Brustkrebs, Uteruskrebs, chronisch-lymphatischen Leukämien, Darmkrebs, Speiseröhrenkrebs, Leberkrebs, lymphoblastischen Leukämien, Nicht-Hodgkin-Lymphomen, Melanomen, malignen Hämopathien, Myelomen, Ovarialkrebs, nicht-kleinzelligem Lungenkrebs, Prostatakrebs und kleinzelligem Lungenkrebs.

28. Verbindung der Formel (I) nach einem der Ansprüche 1 bis 19 oder eines ihrer Additionssalze mit einer pharmazeutisch annehmbaren Säure oder Base zur Verwendung bei der Behandlung von Blasenkrebs, Gehirnkrebs, Brustkrebs, Uteruskrebs, chronischen Lymphoidleukämien chronisch-lymphatischen Leukämien, Darmkrebs, Speiseröhrenkrebs, Leberkrebs, lymphoblastischen Leukämien, Nicht-Hodgkin-Lymphomen, Melanomen, malignen Hämopathien, Myelomen, Ovarialkrebs nicht-kleinzelligem Lungenkrebs, Prostatakrebs und kleinzelligem Lungenkrebs.

29. Verwendung einer Verbindung der Formel (I) nach einem der Ansprüche 1 bis 19 oder eines ihrer Additionssalze mit einer pharmazeutisch annehmbaren Säure oder Base zur Herstellung eines Arzneimittels zur Behandlung von Blasenkrebs, Gehirnkrebs, Brustkrebs, Uteruskrebs, chronisch-lymphatischen Leukämien, Darmkrebs, Speiseröhrenkrebs, Leberkrebs, lymphoblastischen Leukämien, Nicht-Hodgkin-Lymphomen, Melanomen, malignen Hämopathien, Myelomen, Ovarialkrebs, nicht-kleinzelligem Lungenkrebs, Prostatakrebs und kleinzelligem Lungenkrebs.

30. Kombination einer Verbindung der Formel (I) nach einem der Ansprüche 1 bis 19 mit einem Antikrebsmittel ausgewählt aus genotoxischen Mitteln, mitotischen Giften, Anti-Metaboliten, Proteasom-Inhibitoren, Kinaseinhibitoren oder Antikörpern.

31. Pharmazeutische Zubereitung, enthaltend eine Kombination gemäß Anspruch 30 in Kombination mit einem oder mehreren pharmazeutisch annehmbaren Hilfsstoffen.

32. Kombination nach Anspruch 30 zur Verwendung bei der Behandlung von Krebs.

33. Verwendung einer Kombination nach Anspruch 30 zur Herstellung eines Arzneimittels zur Behandlung von Krebs.

34. Verbindung der Formel (I) nach einem der Ansprüche 1 bis 19 zur Verwendung in Kombination mit einer Bestrahlungstherapie bei der Behandlung von Krebs.

## Claims

1. Phosphate compound of formula (I): 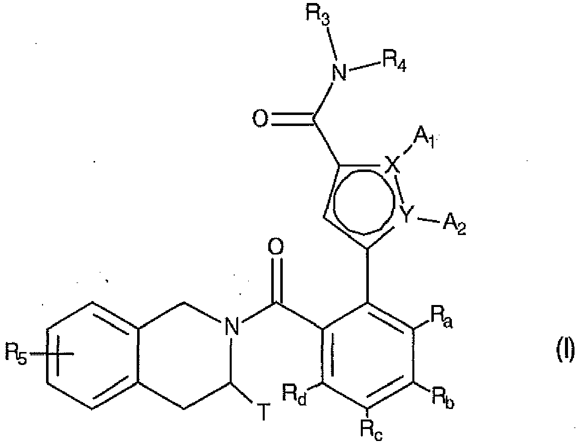 wherein:
◆ X and Y represent a carbon atom or a nitrogen atom, it being understood that they may not simultaneously represent two carbons atoms or two nitrogen atoms,
◆ A₁ and A₂, together with the atoms carrying them, form an optionally substituted, aromatic or non-aromatic heterocycle Het composed of 5, 6 or 7 ring members which may contain, in addition to the nitrogen represented by X or by Y, from one to 3 hetero atoms selected independently from oxygen, sulphur and nitrogen, it being understood that the nitrogen in question may be substituted by a group representing a hydrogen atom, a linear or branched (C₁-C₆)alkyl group or a group -C(O)-O-Alk wherein Alk is a linear or branched (C₁-C₆)alkyl group,
or A₁ and A₂ independently of one another represent a hydrogen atom, a linear or branched (C₁-C₆)polyhaloalkyl, a linear or branched (C₁-C₆)alkyl group or a cycloalkyl,
◆ T represents a hydrogen atom, a linear or branched (C₁-C₆)alkyl group optionally substituted by from one to three halogen atoms, a group (C₁-C₄)alkyl-NR₁R₂, or a group (C₁-C₄)alkyl-OR₆,
◆ R₁ and R₂ independently of one another represent a hydrogen atom or a linear or branched (C₁-C₆)alkyl group,
or R₁ and R₂ form with the nitrogen atom carrying them a heterocycloalkyl,
◆ R₃ represents a linear or branched (C₁-C₆)alkyl group, a linear or branched (C₂-C₆)alkenyl group, a linear or branched (C₂-C₆)alkynyl group, a cycloalkyl group, a (C₃-C₁₀)cycloalkyl-(C₁-C₆)alkyl group wherein the alkyl moiety is linear or branched, a heterocycloalkyl group, an aryl group or a heteroaryl group, it being understood that one or more of the carbon atoms of the preceding groups, or of their possible substituents, may be deuterated,
◆ R₄ represents phenyl substituted in the *para* position by a group of formula -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃), or -OPO(O⁻M₁⁺)(O[CH₂CH₂O]ₙCH₃), or R₄ represents a pyrimidin-5-yl group substituted in the *para* position by a group of formula -OPO(O⁻M₁⁺)(O⁻M₂⁺), wherein M and M' independently of one another represent a hydrogen atom, a linear or branched (C₁-C₆)alkyl group, a linear or branched (C₂-C₆)alkenyl group, a linear or branched (C₂-C₆)alkynyl group, a cycloalkyl or a heterocycloalkyl both composed of 5 or 6 ring members, while M₁⁺ and M₂⁺ independently of one another represent a pharmaceutically acceptable monovalent cation, and M₃²⁺ represents a pharmaceutically acceptable divalent cation and n is an integer from 1 to 5, it being understood that the phenyl group may optionally be substituted by one or more halogen atoms,
◆ R₅ represents a hydrogen or halogen atom, a linear or branched (C₁-C₆)alkyl group, or a linear or branched (C₁-C₆)alkoxy group,
◆ R₆ represents a hydrogen atom or a linear or branched (C₁-C₆)alkyl group,
◆ Rₐ, R_{b}, R_{c} and R_{d}, each independently of the others, represent R₇, a halogen atom, a linear or branched (C₁-C₆)alkoxy group, a hydroxy group, a linear or branched (C₁-C₆)polyhaloalkyl group, a trifluoromethoxy group, -NR₇R₇', nitro, R₇-CO-(C₀-C₆)alkyl-, R₇-CO-NH-(C₀-C₆)alkyl-, NR₇R₇'-CO-(C₀-C₆)alkyl-, NR₇R₇'-CO-(C₀-C₆)alkyl-O-, R₇-SO₂-NH-(C₀-C₆)alkyl-, R₇-NH-CO-NH-(C₀-C₆)alkyl-, R₇-O-CO-NH-(C₀-C₆)alkyl-, a heterocycloalkyl group, or the substituents of one of the pairs (Rₐ,R_{b}), (R_{b},R_{c}) or (R_{c},R_{d}) form together with the carbon atoms carrying them a ring composed of from 5 to 7 ring members, which may contain from one to 2 hetero atoms selected from oxygen and sulphur, it also being understood that one or more carbon atoms of the ring defined hereinbefore may be deuterated or substituted by from one to 3 groups selected from halogen and linear or branched (C₁-C₆)alkyl,
◆ R₇ and R₇' independently of one another represent a hydrogen, a linear or branched (C₁-C₆)alkyl, a linear or branched (C₂-C₆)alkenyl, a linear or branched (C₂-C₆)alkynyl, an aryl or a heteroaryl, or R₇ and R₇' together with nitrogen atom carrying them form a heterocycle composed of from 5 to 7 ring members,
it being understood that:
- "aryl" means a phenyl, naphthyl, biphenyl or indenyl group,
- "heteroaryl" means any mono- or bi-cyclic group composed of from 5 to 10 ring members, having at least one aromatic moiety and containing from 1 to 4 hetero atoms selected from oxygen, sulphur and nitrogen (including quaternary nitrogens),
- "cycloalkyl" means any mono- or bi-cyclic, non-aromatic, carbocyclic group containing from 3 to 10 ring members,
- "heterocycloalkyl" means any mono- or bi-cyclic, non-aromatic, condensed or spiro group composed of 3 to 10 ring members and containing from 1 to 3 hetero atoms selected from oxygen, sulphur, SO, SO₂ and nitrogen,
it being possible for the aryl, heteroaryl, cycloalkyl and heterocycloalkyl groups so defined and the groups alkyl, alkenyl, alkynyl and alkoxy to be substituted by from 1 to 3 groups selected from optionally substituted, linear or branched (C₁-C₆)alkyl, (C₃-C₆)spiro, linear or branched, optionally substituted (C₁-C₆)alkoxy, (C₁-C₆)alkyl-S-, hydroxy, oxo (or *N-*oxide where appropriate), nitro, cyano, -COOR', -OCOR', NR'R", linear or branched (C₁-C₆)polyhaloalkyl, trifluoromethoxy, (C₁-C₆)alkylsulphonyl, halogen, optionally substituted aryl, heteroaryl, aryloxy, arylthio, cycloalkyl, heterocycloalkyl optionally substituted by one or more halogen atoms or alkyl groups, it being understood that R' and R" independently of one another represent a hydrogen atom or an optionally substituted, linear or branched (C₁-C₆)alkyl group,
it being possible for the Het group defined in formula (I) to be substituted by from one to three groups selected from linear or branched (C₁-C₆)alkyl, hydroxy, linear or branched (C₁-C₆)alkoxy, NR₁'R₁" and halogen, it being understood that R₁' and R₁" are as defined for the groups R' and R" mentioned hereinbefore,
its enantiomers and diastereoisomers, and addition salts thereof with a pharmaceutically acceptable acid or base.

2. Compound of formula (I) according to claim 1, wherein R₄ represents phenyl substituted in the *para* position by a group of formula -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃), or -OPO(O⁻M₁⁺)(O[CH₂CH₂O]ₙCH₃), wherein M and M' independently of one another represent a hydrogen atom, a linear or branched (C₁-C₆)alkyl group, a linear or branched (C₂-C₆)alkenyl group, a linear or branched (C₂-C₆)alkynyl group, a cycloalkyl or a heterocycloalkyl both composed of 5 or 6 ring members, while M₁⁺ and M₂⁺ independently of one another represent a pharmaceutically acceptable monovalent cation, and M₃²⁺ represents a pharmaceutically acceptable divalent cation and n is an integer from 1 to 5, it being understood that the phenyl group may optionally be substituted by one or more halogen atoms.

3. Compound of formula (I) according to claim 1, wherein R₄ represents phenyl substituted in the *para* position by a group of formula -OPO(O⁻Na⁺)(O⁻Na⁺).

4. Compound of formula (I) according to one of claims 1 to 3, wherein X represents a carbon atom and Y represents a nitrogen atom.

5. Compound of formula (I) according to one of claims 1 to 3, wherein the group: 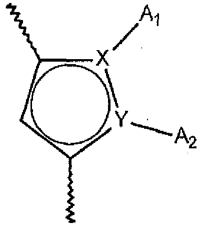 represents a 5,6,7,8-tetrahydroindolizine, an indolizine or a dimethylated pyrrole.

6. Compound of formula (I) according to one of claims 1 to 5, wherein T represents a methyl, (morpholin-4-yl)methyl or 3-(morpholin-4-yl)propyl group.

7. Compound of formula (I) according to one of claims 1 to 6, wherein Rₐ and R_{d} each represent a hydrogen atom and (R_{b},R_{c}), together with the carbon atoms carrying them, form a 1,3-dioxolane group or a 1,4-dioxane group; or Rₐ, R_{c} and R_{d} each represent a hydrogen atom and R_{b} represents a hydrogen or a halogen.

8. Compound of formula (I) according to one of claims 1 to 6, wherein Rₐ and R_{d} each represent a hydrogen atom, R_{b} represents a halogen atom and R_{c} a methoxy group.

9. Compound of formula (I) according to one of claims 1 to 6, wherein Rₐ, R_{b} and R_{d} each advantageously represent a hydrogen atom and R_{c} represents a group NR₇R₇'-CO-(C₀-C₆)alkyl-O-.

10. Compound of formula (I) according to one of claims 1 to 9, wherein R₃ advantageously represents a group selected from phenyl, 1*H*-indole, 1*H*-pyrrolo[2,3-*b*]-pyridine, pyridine, 1*H*-pyrazole, 1*H*-pyrrole and 2,3-dihydro-1*H*-pyrrolo[2,3-*b*]pyridine, those groups optionally having one or more substituents selected from linear or branched (C₁-C₆)alkyl, cyano and trideuteriomethyl.

11. Compound of formula (I) according to claim 1, selected from the following list:
- 4-[{[3-{6-{[(3*S*)-3-{morpholin-4-ylmethyl)-3,4-dihydroisoquinolin-2(1*H*)-yl]carbonyl}-1,3-benzodioxol-5-yl)-5,6,7,8-tetrahydroindolizin-1-yl]carbonyl}(phenyl)amino]phenyl disodium phosphate,
- 4-[{[5-(5-chloro-2-1[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}(pyridin-4-yl)amino]phenyl disodium phosphate,
- 4-({[5-(5-chloro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}[1-(trideuteriomethyl)-1*H*-pyrazol-4-yl]amino)phenyl disodium phosphate,
- 4-[{[5-(5-chloro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}(5-cyano-1,2-dimethyl-1*H*-pyrrol-3-yl)amino]phenyl disodium phosphate,
- 4-[{[5-(5-chloro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}(5-cyano-1-methyl-1*H-*pyrrol-3-yl)amino]phenyl disodium phosphate,
- 4-[{[5-(5-chloro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}(1-methyl-1*H*-pyrazol-4-yl)amino]phenyl disodium phosphate,
- 4-[(5-cyano-1,2-dimethyl-1*H*-pyrrol-3-yl){[5-(5-fluoro-2-{({3*S*)-3-(morpholin-4-ylmethyl)-3,4-dihydroisoquinolin-2(1*H*)-yl]carbonyl}phenyl)-1,2-dimethyl-1*H-*pyrrol-3-yl]carbonyl}amino]phenyl disodium phosphate,
- 4-[{[5-(5-fluoro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}(1-methyl-1*H*-pyrazol-4-yl)amino]phenyl disodium phosphate,
their enantiomers and diastereoisomers, and addition salts thereof with a pharmaceutically acceptable acid or base.

12. Compound of formula (I) according to claim 11, which is 4-[{[3-(6-{[(3*S*)-3-(morpholin-4-ylmethyl)-3,4-dihydroisoquinolin-2(1*H*)-yl]carbonyl}-1,3-benzodioxol-5-yl)-5,6,7,8-tetrahydroindolizin-1-yl]carbonyl}(phenyl)amino]phenyl disodium phosphate.

13. Compound of formula (I) according to claim 11, which is 4-[{[5-(5-chloro-2-{[(3*S*)-3-(morpholin-4-ylmethyl)-3,4-dihydroisoquinolin-2(1*H*)-yl]carbonyl}phenyl)-1,2-dimethyl-1*H*-pytrol-3-yl]carbonyl}(pyridin-4-yl)amino]phenyl disodium phosphate.

14. Compound of formula (I) according to claim 11, which is 4-({[5-(5-chloro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}[1-(trideuteriomethyl)-1*H*-pyrazol-4-yl]amino)phenyl disodium phosphate.

15. Compound of formula (I) according to claim 11, which is 4-[{[5-(5-chloro-2-{[(3*S*)-3-{morpholin-4-ylmethyl)-3,4-dihydroisoquinolin-2(1*H*)-yl]carbonyl}phenyl)-1,2-dimethyl-1*H*-pyrrol-3-yl]carbonyl}(5-cyano-1,2-dimethyl-1*H*-pyrrol-3-yl)amino]phenyl disodium phosphate.

16. Compound of formula (I) according to claim 11, which is 4-[{[5-(5-chloro-2-{[(3*S*)*-*3*-(*morpholin-4-ylmethyl)-3,4-dihydroisoquinolin-2(1*H*)-yl]carbonyl}phenyl)-1,2-dimethyl-1*H*-pyrrol-3-yl]carbonyl}(5-cyano-1-methyl-1*H*-pyrrol-3-yl)amino]phenyl disodium phosphate.

17. Compound of formula (I) according to claim 11, which is 4-[{[5-(5-chloro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}(1-methyl-1*H*-pyrazol-4-yl)amino]phenyl disodium phosphate.

18. Compound of formula (I) according to claim 11, which is 4-[(5-cyano-1,2-dimethyl-1*H*-pyrrol-3-yl){[5-(5-fluoro-2-{[(3*S*)-3-(morpholin-4-ylmethyl)-3,4-dihydro-isoquinolin-2(1*H*)-yl]carbonyl}phenyl)-1,2-dimethyl-1*H*-pyrrol-3-yl]carbonyl}amino]-phenyl disodium phosphate.

19. Compound of formula (I) according to claim 11, which is 4-[{[5-(5-fluoro-2-{[(3*S*)-3-(morpholin-4-ylmethyl)-3,4-dihydroisoquinolin-2(1*H*)-yl]carbonyl}phenyl)-1,2-dimethyl-1*H*-pyrrol-3-yl]carbonyl}(1-methyl-1*H*-pyrazol-4-yl)amino]phenyl disodium phosphate.

20. Process for the preparation of compounds of formula (I) according to claim 1, **characterised in that** there is used as starting material the compound of formula (II): 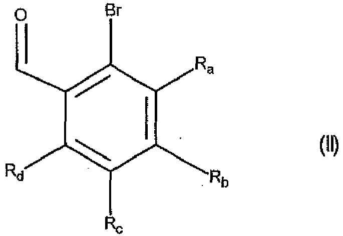 wherein Rₐ, R_{b}, R_{c} and R_{d} are as defined in claim 1,
which compound of formula (II) is subjected to a Heck reaction, in an aqueous or organic medium, in the presence of a palladium catalyst, of a base, of a phosphine and of the compound of formula (III): 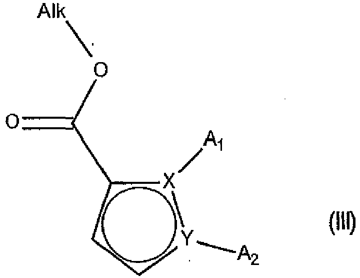 wherein A₁, A₂, X and Y are as defined in claim 1 and Alk represents a linear or branched (C₁-C₆)alkyl group,
to obtain the compound of formula (IV): 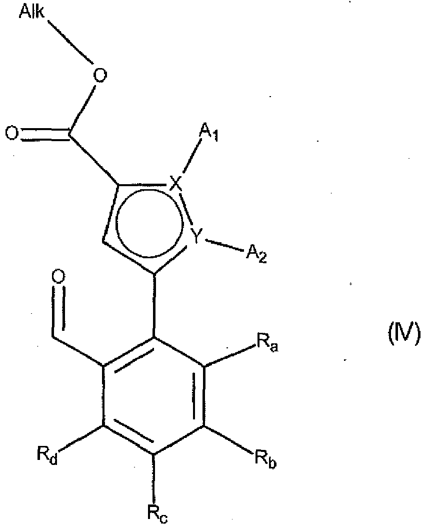 wherein A₁, A₂, X, Y, Rₐ, R_{b}, R_{c} and R_{d} are as as defined in claim 1 and Alk is as defined hereinbefore,
the aldehyde function of which compound of formula (IV) is oxidised to a carboxylic acid to form the compound of formula (V): 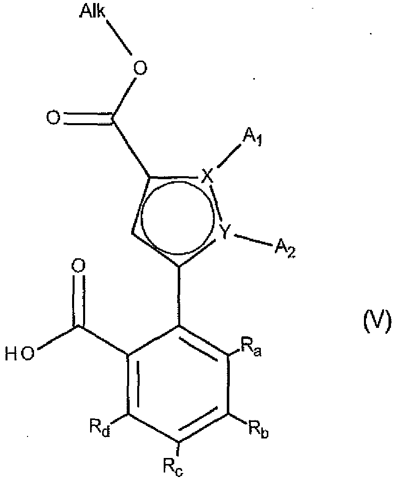 wherein A₁, A₂, X, Y, Rₐ, R_{b}, R_{c} and R_{d} are as as defined in claim 1 and Alk is as defined hereinbefore,
which compound of formula (V) is then subjected to peptide coupling with a compound of formula (VI): 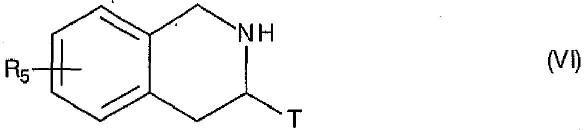 wherein T and R₅ are as as defined in claim 1,
to yield the compound of formula (VII): 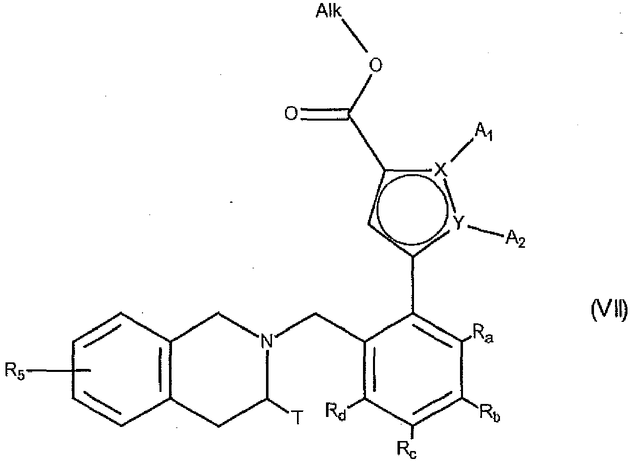 wherein A₁, A₂, X, Y, Rₐ, R_{b}, R_{c}, R_{d}, T and R₅ are as as defined in claim 1 and Alk is as defined hereinbefore,
the ester function of which compound of formula (VII) is hydrolysed to yield the corresponding carboxylic acid or carboxylate, which may be converted into an acid derivative such as the corresponding acyl chloride or anhydride before being coupled with an amine NHR₃R₄ wherein R₃ and R₄ have the same meanings as in claim 1, before being subjected to the action of a pyrophosphate, phosphonate or phosphoryl compound under basic conditions, it being possible for the compound thereby obtained to be optionally hydrolysed or hydrogenolysed to yield the compound of formula (I),
which compound of formula (I) may be purified according to a conventional separation technique, which is converted, if desired, into its addition salts with a pharmaceutically acceptable acid or base and which is optionally separated into its isomers according to a conventional separation technique,
it being understood that, at any time considered appropriate in the course of the above-described process, certain groups (hydroxy, amino...) of the reagents or intermediates of synthesis may be protected and then deprotected according to the requirements of synthesis.

21. Pharmaceutical composition comprising a compound of formula (I) according to any one of claims 1 to 19 or an addition salt thereof with a pharmaceutically acceptable acid or base in combination with one or more pharmaceutically acceptable excipients.

22. Pharmaceutical composition according to claim 21 for use as a prodrug of a pro-apoptotic agent.

23. Pharmaceutical composition according to claim 21 for use in the treatment of cancers, auto-immune diseases and diseases of the immune system.

24. Pharmaceutical composition according to claim 23 for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

25. Use of a pharmaceutical composition according to claim 21 in the manufacture of a medicament for use as a pro-apoptotic agent.

26. Use of a pharmaceutical composition according to claim 21 in the manufacture of a medicament intended for the treatment of cancers, auto-immune diseases and diseases of the immune system.

27. Use, according to claim 26, of a pharmaceutical composition in the manufacture of a medicament intended for the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

28. Compound of formula (I) according to one of claims 1 to 19, or an addition salt thereof with a pharmaceutically acceptable acid or base, for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

29. Use of a compound of formula (I) according to one of claims 1 to 19, or an addition salt thereof with a pharmaceutically acceptable acid or base, in the manufacture of a medicament intended for the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

30. Combination of a compound of formula (I) according to any one of claims 1 to 19 with an anti-cancer agent selected from genotoxic agents, mitotic poisons, anti-metabolites, proteasome inhibitors, kinase inhibitors and antibodies.

31. Pharmaceutical composition comprising a combination according to claim 30 in combination with one or more pharmaceutically acceptable excipients.

32. Combination according to claim 30 for use in the treatment of cancers.

33. Use of a combination according to claim 30 in the manufacture of a medicament for use in the treatment of cancers.

34. Compound of formula (I) according to any one of claims 1 to 19 for use in association with radiotherapy in the treatment of cancers.
